(19) Europäisches Patentamt
European Patent Office
Office européen des brevets

(11) Publication number: 0 337 714 A2

# (12) EUROPEAN PATENT APPLICATION

(21) Application number: 89303539.4

(22) Date of filing: 11.04.89

(51) Int. Cl.4: C07K 5/02 , C07C 125/065 , C07C 143/74 , C07C 153/057 , C07D 213/40 , C07H 13/04 , C07F 9/28 , C07F 9/09 , A61K 37/64 , A61K 31/16 , A61K 31/395

(30) Priority: 12.04.88 US 180507
24.08.88 US 236084
28.03.89 US 328643

(43) Date of publication of application:
18.10.89 Bulletin 89/42

(84) Designated Contracting States:
AT BE CH DE ES FR GB GR IT LI LU NL SE

(71) Applicant: MERCK & CO. INC.
126, East Lincoln Avenue P.O. Box 2000
Rahway New Jersey 07065-0900(US)

(72) Inventor: Sigal, Irving S.

deceased(US)
Inventor: Huff, Joel R.
738 Bergey Mill Road
Lederach, PA 19450(US)
Inventor: Darke, Paul L.
434 West Walnut Street Apt. 3
North Wales, PA 19454(US)
Inventor: Vacca, Joseph P.
766 Eisenhauer Drive
Telford, PA 18969(US)
Inventor: Young, Steven D.
1280 Mark Drive
Lansdale, PA 19446(US)
Inventor: Desolms, S. Jane
735 Port Indian Road
Norristown, PA 19403(US)
Inventor: Thompson, Wayne J.
Box 172 Whites Mill Road
Green Lane, PA 18054(US)
Inventor: Lyle, Terry A.
570 Camp Wa Wa Road
Lederach, PA 19440(US)
Inventor: Graham, Samuel L.
382 Chadwyck Circle
Harleysville, PA 19438(US)
Inventor: Ghosh, Arun K.
1141 Snyder Road Forge Gate Apt. D-37
Lansdale, PA 19446(US)

(74) Representative: Hesketh, Alan, Dr.
European Patent Department Merck & Co., Inc. Terlings Park Eastwick Road
Harlow Essex, CM20 2QR(GB)

## (54) HIV protease inhibitors useful for the treatment of aids.

(57) Compounds of the form
A-G-B-B-J
wherein A is an amine protecting group commonly employed in peptide synthesis, G a dipeptide isostere, B an amino acid or analog thereof, and J a small terminal group are described. These compounds are useful in the inhibition of HIV protease, the prevention or treatment of infection by HIV and the treatment of AIDS, either as compounds, pharmaceutically acceptable salts, pharmaceutical composition ingredients, whether or not in combination with other antivirals, immunomodulators, antibiotics or vaccines. Methods of treating AIDS and methods of preventing or treating infection by HIV are also described.

EP 0 337 714 A2

## HIV PROTEASE INHIBITORS USEFUL FOR THE TREATMENT OF AIDS

The present invention is concerned with compounds which inhibit the protease encoded by human immunodeficiency virus (HIV) or pharmaceutically acceptable salts thereof and are of value in the prevention of infection by HIV, the treatment of infection by HIV and the treatment of the resulting acquired immune deficiency syndrome (AIDS). It also relates to pharmaceutical compositions containing the compounds and to a method of use of the present compounds and other agents for the treatment of AIDS & viral infection by HIV.

### BACKGROUND OF THE INVENTION

A retrovirus designated human immunodeficiency virus (HIV) is the etiological agent of the complex disease that includes progressive destruction of the immune system (acquired immune deficiency syndrome: AIDS) and degeneration of the central and peripheral nervous system. This virus was previously known as LAV, HTLV-III, or ARV. A common feature of retrovirus replication is the extensive post-translational processing of precursor polyproteins by a virally encoded protease to generate mature viral proteins required for virus assembly and function. Interruption of this processing appears to prevent the production of normally infectious virus. For example, Crawford, S. et al., J. Virol., 53, 899, 1985, demonstrated that genetic deletion mutations of the protease in murine leukemia virus which prevent processing of precursor structural proteins results in non-infectious viral particles. Unprocessed structural proteins also have been observed in clones of non-infectious HIV strains isolated from human patients. These results suggest that inhibition of the HIV protease represents a viable method for the treatment of AIDS and the prevention or treatment of infection by HIV.

Nucleotide sequencing of HIV shows the presence of a pol gene in one open reading frame [Ratner, L. et al., Nature, 313, 277(1985)]. Amino acid sequence homology provides evidence that the pol sequence encodes reverse transcriptase, an endonuclease and an HIV protease [Toh, H. et al., EMBO J. 4, 1267 (1985); Power, M.D. et al., Science, 231, 1567 (1986); Pearl, L.H. et al., Nature 329, 351 (1987)]. Applicants demonstrate that the compounds of this invention are inhibitors of HIV protease.

### BRIEF DESCRIPTION OF THE INVENTION

Compounds of formula I, as herein defined, are disclosed. These compounds are useful in the inhibition of HIV protease, the prevention of infection by HIV, the treatment of infection by HIV and in the treatment of AIDS, either as compounds, pharmaceutically acceptable salts, pharmaceutical composition ingredients, whether or not in combination with other antivirals, immunomodulators, antibiotics or vaccines. Methods of treating AIDS, methods of preventing infection by HIV, and methods of treating infection by HIV are also disclosed.

## ABBREVIATIONS

| Designation | Amino Acid/Residue |
|---|---|
| Ala | D- or L-alanine |
| Allo-Ile | allo-isoleucine |
| Arg | D- or L-arginine |
| Cal (Cha) | β-cyclohexylalanine |
| Cys | D- or L-cysteine |
| Gly | glycine |
| His | D- or L-histidine |
| Ile | L-isoleucine |
| Leu | D- or L-leucine |
| Lys | D- or L-lysine |

| Designation | Amino Acid/Residue |
|---|---|
| Met | D- or L-methionine |
| Nle | L-norleucine |
| Nva | L-norvaline |
| Orn | D- or L-ornithine |
| Ph | phenyl |
| Phe | D- or L-phenylalanine |
| Pro | D- or L-proline |
| Sar | sarcosine (N-methylglycine) |
| Ser | D- or L-serine |
| Sta | statine, (3S,4S)-4-amino-3-hydroxy-6-methylheptanoic acid |
| Thr | D- or L-threonine |
| Trp | D- or L-tryptophan |
| Tyr | D- or L-tyrosine |
| Val | L-valine |
| | Protecting Group |
| BOC (Boc) | *t*-butyloxycarbonyl |
| BOM | benzyloxymethyl |
| CBZ (Cbz) | benzyloxycarbonyl(carbobenzoxy) |
| DNP | 2,4-dinitrophenyl |
| INOC | isonicotinoyloxycarbonyl |
| IPOC | isopropoxycarbonyl |
| OMe | methyl ether (methoxy), except when it immediately follows an amino acid residue abbreviation and it represents methyl ester. |

| | |
|---|---|
| OEt | ethoxy, except when it immediately follows an amino acid residue abbreviation and it represents ethyl ester |
| | |
| <u>Designation</u> | <u>Activating Group</u> |
| HBT(HOBT) | 1-hydroxybenzotriazole hydrate |
| OMs | methane sulfonyloxy |
| | <u>Condensing Agent</u> |
| DCCI (DCC) | dicyclohexylcarbodiimide |
| DPPA | diphenylphosphorylazide |
| | <u>Reagent</u> |
| $(BOC)_2O$ | di-*t*-butyl dicarbonate |
| DEAD | diethyl azodicarboxylate |
| TEA | triethylamine |
| TFA | trifluoroacetic acid |
| | <u>Coupling Reagents</u> |
| BOP reagent | benzotriazol-1-yloxytris-(dimethyl-amino)phosphonium hexafluorophosphate |
| BOP-Cl | bis(2-oxo-3-oxazolidinyl) phosphinic chloride |
| DSO | N,N'-disuccinimidyl oxalate |
| EDC | 1-ethyl-3-(3-dimethylaminopropyl) carbodiimide hydrochloride |
| MCPBA | m-Chloroperbenzoic acid |
| TBDMS | t-Butyl-dimethylsilyl |

## <u>DETAILED DESCRIPTION OF THE INVENTION AND PREFERRED EMBODIMENTS</u>

This invention is concerned with the use of compounds of formula I, combinations thereof, or pharmaceutically acceptable salts thereof, in the inhibition of HIV protease, the prevention or treatment of

infection by HIV and in the treatment of the resulting acquired immune deficiency syndrome (AIDS). Compounds of formula I are defined as follows:

A-G-B-B-J I,

wherein A is:

1) trityl,

2) hydrogen;

3)

$R^1-\overset{O}{\overset{\|}{C}}-$ wherein $R^1$ is

a) hydrogen,

b) $C_{1-4}$ alkyl, substituted with one or more halogens adjacent to the carbonyl carbon where halogen is F, Cl, Br, and I;

4) phthaloyl wherein the aromatic ring is unsubstituted or substituted with one or more of

a) $C_{1-4}$ alkyl,

b) halo,

c) hydroxy,

d) nitro,

e) $C_{1-3}$ alkoxy,

f) $C_{1-3}$ alkoxycarbonyl,

g) cyano,

h)

$-\overset{O}{\overset{\|}{C}}-NR_2$ wherein R is H or $C_{1-4}$ alkyl;

5)

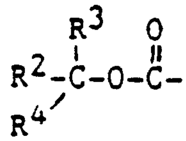

wherein $R^2$,$R^3$, and $R^4$ are independently

a) H,

b) $C_{1-6}$ alkyl unsubstituted or substituted with one or more of

i) halo,

ii) alkyl $SO_2$-,

iii) aryl $SO_2$-,

c) Aryl unsubstituted or substituted with one or more of

i) $C_{1-4}$ alkyl,

ii) $C_{1-3}$ alkoxy,

iii) halo,

iv) nitro,

v) acetoxy,

vi) dimethylaminocarbonyl,

vii) phenyl,

viii) $C_{1-3}$ alkoxycarbonyl

d) fluorenyl,

e) $R^2$, $R^3$, and $R^4$ may be independently joined to form a monocyclic, bicyclic, or tricyclic ring system which is $C_{3-10}$ cycloalkyl and may be substituted with $C_{1-4}$ alkyl,

f) a 5-7 membered heterocycle such as pyridyl, furyl, or benzisoxazolyl;

6)

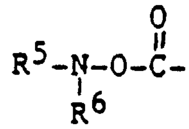

wherein $R^5$ and $R^6$ are

a) $C_{1-4}$ alkyl,
b) aryl,
c) $R^5$ and $R^6$ are joined to form a 5-7 membered heterocycle;

7)

$R^7-SO_2NH-\overset{O}{\overset{\|}{C}}-$ wherein $R^7$ is aryl unsubstituted or substituted with one or more of

a) $C_{1-4}$ alkyl,
b) halo,
c) nitro,
d) $C_{1-3}$ alkoxy;

8)

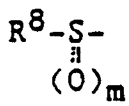

wherein m is 0-2 and $R^8$ is

a) $R^7$ as defined above,
b) trityl;

9)

$(R^7)_2\ \overset{X}{\overset{\|}{P}}-$ wherein X is O, S or NH, and $R^7$ is defined above;

G is

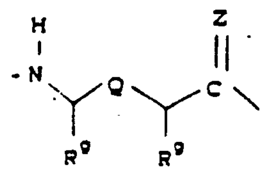

or

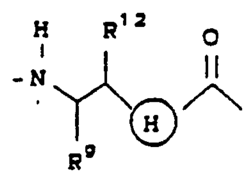

wherein Z is O, S, or HH and
$R^9$ is independently

1) hydrogen,
2)

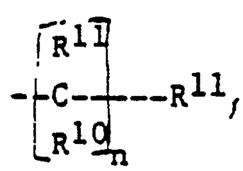

3) -OR, wherein R is H, or $C_{1-4}$ alkyl
4) $-NR_2$,

5) $C_{1-4}$alkylene $-R^{11}$;

wherein n is 0-5 and $R^{10}$ is independently

a) hydrogen,

b) hydroxy, or

c) $C_{1-4}$alkyl;

$R^{11}$ is

a) hydrogen,

b) aryl, unsubstituted or substituted with one or more of i) halo,

ii) hydroxy,

iii) $-NH_2$, $-NO_2$, -NHR, or $-NR_2$, wherein R is H, or $C_{1-4}$ alkyl,

iv) $C_{1-4}$ alkyl,

v) $C_{1-3}$ alkoxy,

vi) -COOR

vii) $-\overset{O}{\overset{\parallel}{C}}NR_2$,

viii) $-CH_2NR_2$,

ix) $-CH_2NH\overset{O}{\overset{\parallel}{C}}R$,

x) -CN,

xi) $-CF_3$,

xii) $-NH\overset{O}{\overset{\parallel}{C}}R$,

xiii) aryl $C_{1-3}$alkoxy or aryl $C_{1-4}$ alkyl,

xiv) aryl,

xv) $-NRSO_2R$,

xvi) $-OP(O)(OR_x)_2$ wherein $R_x$ is H or aryl, or

xvii) $-O-\overset{O}{\overset{\parallel}{C}}-C_{1-4}$alkyl substituted with one or more of amine or quaternary amine;

c) 5 or 6 membered heterocycle including up to 3 heteroatoms selected from N, O, and S, such as imidazolyl, thiazolyl, piperidinyl, furanyl, oxazolyl, thiadiazolyl, piperazinyl, pyridyl, or pyrazinyl, any of which heterocycle may be unsubstituted or substituted with one or more of

i) halo,

ii) hydroxy,

iii) $-NH_2$, -NHR, $-NR_2$;

iv) $C_{1-4}$ alkyl,

v) $C_{1-3}$ alkoxy,

vi) -COOR,

vii) $-\overset{O}{\overset{\parallel}{C}}NR_2$,

viii) $-CH_2NR_2$,

ix) $-NH\overset{O}{\overset{\parallel}{C}}R$,

x) -CN,

xi) $CF_3$,

xii) $-NHSO_2R$,

xiii) $-OP(O)(OR_x)_2$ wherein $R_x$ is H or aryl, or

xiv) $-O-\overset{O}{\overset{\parallel}{C}}-C_{1-4}$alkyl substituted with one or more of amine or quaternary amine;

d) $C_{1-6}$ alkyl or $C_{1-6}$ alkenyl unsubstituted or substituted with one or more of

i) hydroxy,

ii) $C_{1-4}$ alkyl,

iii) $-NH_2$, -NHR, $-NR_2$,
iv)
NH
||
-NH-CH ,
v)
NH
||
-NH- C - $NH_2$,
vi) -COOH,
vii)
O
||
- C OR,
viii) -SR, or aryl thio,
ix) $-SO_2NHR$,
x) $C_{1-4}$ alkyl sulfonyl amino or aryl sulfonyl amino,
xi) -CONHR,
xii)
O
||
-NH C R,
xiii) -OR,
xiv) aryl $C_{1-3}$ alkoxy, or,
xv) aryl;

e) $C_{3-7}$ cycloalkyl unsubstituted or substituted with one or more of i) hydroxy,
ii) $C_{1-4}$ alkyl,
iii) $-NH_2$, -NHR, $-NHR_2$,
iv)
NH
||
-NH- CH ,
v)
NH
||
-NH- C - $NH_2$,
vi) -COOH,
vii)
O
||
- C -OR,
viii) -SR,
ix) $-SO_2NH_2$,
x) alkyl sulfonylamino or aryl sulfonylamino,
xi) -CONHR, or
xii) -NH $\overset{O}{C}$ R;

f) a 5- to 7-membered carbocyclic or 7- to 10-membered bicyclic carbocyclic ring which is either saturated or unsaturated, such as cyclopentane, cyclohexane, indan, norbornane, or naphthalene, the carbocyclic ring being unsubstituted or substituted with one or more of
i) halo,
ii) -OR, wherein R is H or $C_{1-4}$ alkyl,
iii)
O
||
- COR,
iv)
O
||
- C $NR_2$,
v) $-CH_2NR_2$,
vi) $-SO_2NR_2$; $-S(O)_yR$ and y = 0, 1 or 2;
vii) $-NR_2$,
viii)
O
||
-NH CR,
ix) $C_{1-4}$ alkyl,
x) phenyl,

xi) $-CF_3$, or

xii)

R
|
- N-$SO_2R$;

g) benzofuryl, indolyl; azabicyclo $C_{7-11}$ cycloalkyl; or benzopiperidinyl;

$R^{12}$ is -OH or $-NHR^{13}$, wherein $R^{13}$ is -H,

O
||
- CH, $-C_{1-4}$-alkyl, or -COOR; and

Ⓗ is

1) $C_{3-7}$ cycloalkyl either unsubstituted or substituted with one or more of

a) $C_{1-4}$ alkyl,
b) hydroxy,
c) $-NR_2$,
d) -COOR,
e) -CONHR,
f) $-NHSO_2R$,
g)

O
||
-NH C R,

h) aryl,
i) aryl substituted with $C_{1-4}$alkyl,
j) heterocycle, or
k) heterocycle substituted with $C_{1-4}$alkyl;

2) phenyl either unsubstituted or substituted with one or more of

a) hydroxy,
b) -OR,
c) $-NHR^{13}$,
d) -COOR,
e)

O
||
- C $NR_2$, or,

O
||
f) -NH C R;

3) 5 to 7-membered heterocycle such as imidazolyl, thiazolyl, furyl, oxazolyl, piperidyl, piperazinyl, pyridyl, or pyrazinyl, any of which heterocycle may be unsubstituted or substituted with one or more of

i) halo,
ii) hydroxy,
iii) $NR_2$, or,
iv) $C_{1-4}$ alkyl;

Q is

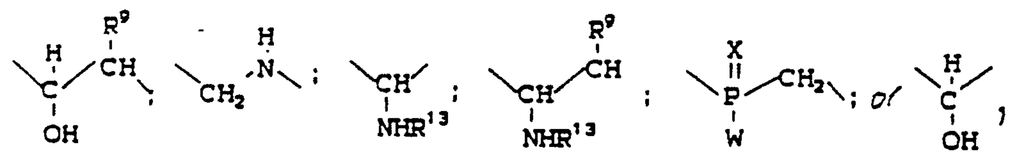

wherein $R^9$ and $R^{13}$ are defined above;

X is O, S or NH; and

W is

1) OH,
2) $NH_2$,
3) OR, or

4) NHR;

B is, independently, absent, or

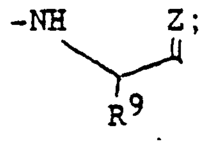

J is

1) $YR^{14}$ wherein:

Y is O or NH, and

$R^{14}$ is

a) H;

b) $C_{1-6}$ alkyl, unsubstituted or substituted with one or more of

i) $-NR_2$,

ii) -OR,

iii) $-NHSO_2C_{1-4}$ alkyl,

iv) $-NHSO_2$ aryl, or $-NHSO_2$(dialkylaminoaryl),

v) $-CH_2OR$,

vi) $-C_{1-4}$alkyl,

vii)

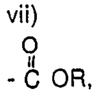

viii)

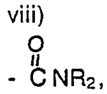

ix)

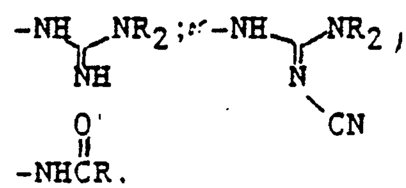

x)

xi)

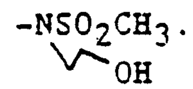

xii)

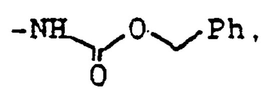

xiii) $-NR_3^{\oplus} A^{\ominus}$ wherein $A^{\ominus}$ is a counterion,

xiv) $-NR^{15}R^{16}$ wherein $R^{15}$ and $R^{16}$ are the same or different and are $C_{1-5}$ alkyl joined together directly to form a 5-7 membered heterocycle,

xv) aryl,

xvi) -CHO,

xvii) $-OP(O)(OR_x)_2$ wherein $R_x$ is H or aryl, or

xvii)

-O- 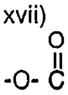 $-C_{1-4}$alkyl substituted with one or more of amine or quaternary amine;

c) $-(CH_2CH_2O)_nCH_3$ or $-(CH_2CH_2O)_n$ H;

2) $N(R^{14})_2$; or

3) $-NR^{15}R^{16}$ wherein $R^{15}$ and $R^{16}$ are defined above;

4)

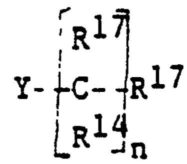

wherein:
Y, $R^{14}$ and n are defined above, and
$R^{17}$ is
a) hydrogen;
b) aryl unsubstituted or substituted with one or more of
i) halo,
ii) -OR, wherein R is H or $C_{1-4}$ alkyl,
iii) $-\overset{O}{\overset{\|}{C}}OR$,
iv) $-\overset{O}{\overset{\|}{C}}NR_2$,
v) $-CH_2NR_2$,
vi) $-SO_2NR_2$,
vii) $-NR^2_2$,
viii) $-NH\overset{O}{\overset{\|}{C}}R$,
xi) $C_{1-4}$ alkyl,
x) phenyl
xi) $-CF_3$,
xii) $-\overset{R}{\overset{|}{N}}-SO_2R$,
xiii) $-C_{1-4}$ alkyl $-NR_2$,
xiv) $-OP(O)(OR_x)_2$ wherein $R_x$ is H or aryl, or
xv) $-O-\overset{O}{\overset{\|}{C}}-C_{1-4}$alkyl substituted with one or more of amine or quaternary amine;
c) Heterocycle as defined below, unsubstituted or substituted with one or more of
i) halo,
ii) -OR, wherein R is H, $C_{1-4}$alkyl, or $C_{1-4}$alkenyl,
iii) $-\overset{O}{\overset{\|}{C}}OR$,
iv) $-\overset{O}{\overset{\|}{C}}NR_2$,
v) $-CH_2NR_2$,
vi) $-SO_2NR_2$,
vii) $-NR_2$,
viii) $-NH\overset{O}{\overset{\|}{C}}R$,
xi) $C_{1-4}$ alkyl,
x) phenyl
xi) $-CF_3$,
xii) $-\overset{R}{\overset{|}{N}}-SO_2R$,

xiii) phenyl $C_{1-4}$ alkyl,
xiv) $-OP(O)(OR_x)_2$ wherein $R_x$ is H or aryl, or
xv)
$-O-\overset{O}{\overset{\|}{C}}-C_{1-4}$alkyl substituted with one or more of amine or quaternary amine;

d) A 5 to 7 membered carbocyclic or 7-10 membered bicyclic carbocyclic ring which is either saturated or unsaturated, such as cyclopentane, cyclohexane, indan, norbornane, or naphthalane, the carbocyclic ring being unsubstituted or substituted with one or more of
i) halo,
ii) -OR, wherein R is H or $C_{1-4}$ alkyl,
iii)
$-\overset{O}{\overset{\|}{C}}OR$,
iv) $-\underset{O}{\underset{\|}{C}}NR_2$,
v) $-CH_2NR_2$
vi) $-SO_2NR_2$,
vii) $-NR_2$,
viii)
$-NH\overset{O}{\overset{\|}{C}}R$,
xi) $C_{1-4}$ alkyl,
x) phenyl
xi) $-CF_3$,
xii)
$-\overset{R}{\overset{|}{N}}-SO_2R$,
xiii) $-OP(O)(OR_x)_2$ wherein $R_x$ is H or aryl, or
xiv)
$-O-\overset{O}{\overset{\|}{C}}-C_{1-4}$alkyl substituted with one or more of amine or quaternary amine;
or pharmaceutically acceptable salts thereof.

In the compounds of the present invention, the A, G, B and J components and the like may have asymmetric centers and occur as racemates, racemic mixtures and as individual diastereomers, with all isomeric forms being included in the present invention.

When any variable (e.g., aryl, heterocycle, R, $R^1$, $R^2$, $R^3$, $R^4$, $R^7$, $R^{10}$, $R^{11}$, $R^{12}$, $R^{14}$, $R^{15}$, $R^{16}$, $R^{17}$, $A^{\ominus}$, n, Z, etc.) occurs more than one time in any constituent or in formula I, its definition on each occurrence is independent of its definition at every other occurrence. Also, combinations of substituents and/or variables are permissible only if such combinations result in stable compounds.

As used herein except where noted, "alkyl" is intended to include both branched- and straight-chain saturated aliphatic hydrocarbon groups having the specified number of carbon atoms (Me is methyl, Et is ethyl, Pr is propyl, Bu is butyl); "alkoxy" represents an alkyl group of indicated number of carbon atoms attached through an oxygen bridge; and "cycloalkyl" is intended to include saturated ring groups, such as cyclopropyl, cyclobutyl, cyclopentyl, cyclohexyl (Cyh) and cycloheptyl. "Alkenyl" is intended to include hydrocarbon claims of either a straight or branched configuration and one or more unsaturated carbon-carbon bonds which may occur in any stable point along the chain, such as ethenyl, propenyl, butenyl, pentenyl, and the like. "Halo", as used herein, means fluoro, chloro, bromo and iodo; and "counterion" is used to represent a small, single negatively-charged species, such as chloride, bromide, hydroxide, acetate, trifluoroacetate, perchlorate, nitrate, benzoate, maleate, tartrate, hemitartrate, benzene sulfonate, and the like.

As used herein, with exceptions as noted, "aryl" is intended to mean phenyl (Ph) or naphthyl. "Carbocyclic" is intended to mean any stable 5- to 7-membered carbon ring or 7- to 10-membered bicyclic carbon ring, any of which may be saturated or partially unsaturated.

The term heterocycle, as used herein except where noted, represents a stable 5- to 7-membered mono- or bicyclic or stable 7- to 10-membered bicyclic heterocyclic ring which is either saturated or unsaturated, and which consists of carbon atoms and from one to three heteroatoms selected from the group consisting

of N, O and S, and wherein the nitrogen and sulfur heteroatoms may optionally be oxidized, and the nitrogen heteroatom may optionally be quaternized, and including any bicyclic group in which any of the above-defined heterocyclic rings is fused to a benzene ring. The heterocyclic ring may be attached at any heteroatom or carbon atom which results in the creation of a stable structure. Example of such heterocyclic elements include piperidinyl, piperazinyl, 2-oxopiperazinyl, 2-oxopiperidinyl, 2-oxopyrrolodinyl, 2-oxoazepinyl, azepinyl, pyrrolyl, 4-piperidonyl, pyrrolidinyl, pyrazolyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, thiadiazolyl, benzopyranyl, benzothiazolyl, benzoxazolyl, furyl, tetrahydrofuryl, tetrahydropyranyl, thienyl, benzothienyl, thiamorpholinyl, thiamorpholinyl sulfoxide, thiamorpholinyl sulfone, and oxadiazolyl.

One embodiment of the compounds of the present invention encompasses those compounds of Formula I in which B is independently present twice and Z is O. In this embodiment, it is preferred that J is $NH_2$ and Q is

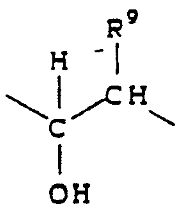 ; 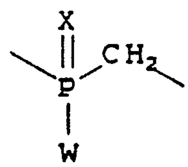 ; or 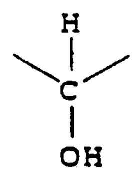

A second embodiment of the compounds of the present invention consists of those compounds of Formula I in which B is present once and Z is O. In this embodiment, it is preferred that Q is

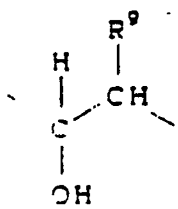 ; 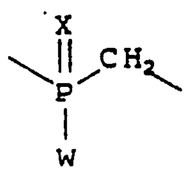 ; or 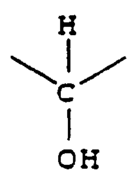 .

A third embodiment of the compounds of the present invention encompasses those compounds of Formula I in which B is absent. In this embodiment, it is preferred that Q is

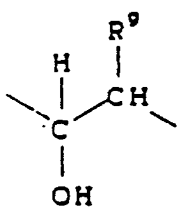 ; 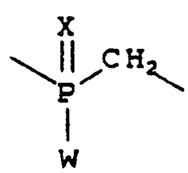 ; or 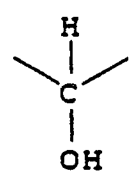 .

A fourth embodiment of the compounds of the present invention encompasses those compounds of Formula I in which G is

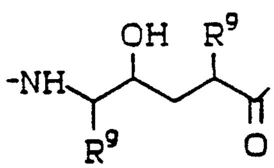 or 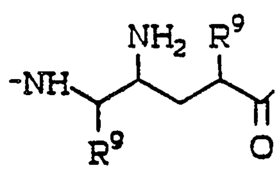 .

A fifth embodiment of the compounds fo the present invention encompasses those compounds of Formula I in which G is

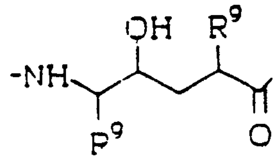 or 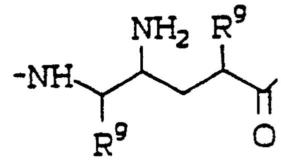 .

and B is absent or present once.

A sixth embodiment of the compounds of the present invention encompasses those compounds of Formula I in which G is

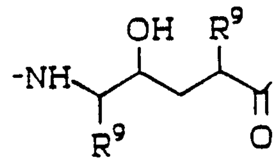 or 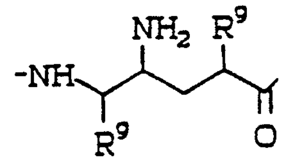 .

B is absent or present once, and

J is 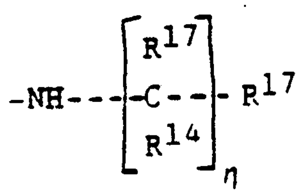 .

A seventh embodiment of the compounds of the present invention encompasses those compounds of Formula I in which

A is 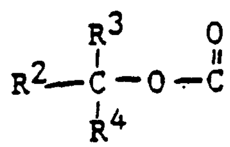 ;

G is 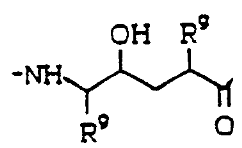 or 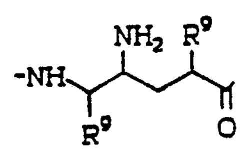 ;

B is absent or present once, and

J is 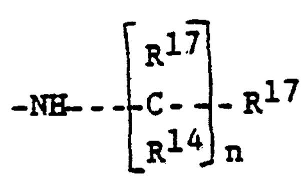 .

Preferred compounds of the present invention are compounds I, II, III, IV, V, VI, and VII as follows:

Compound I:

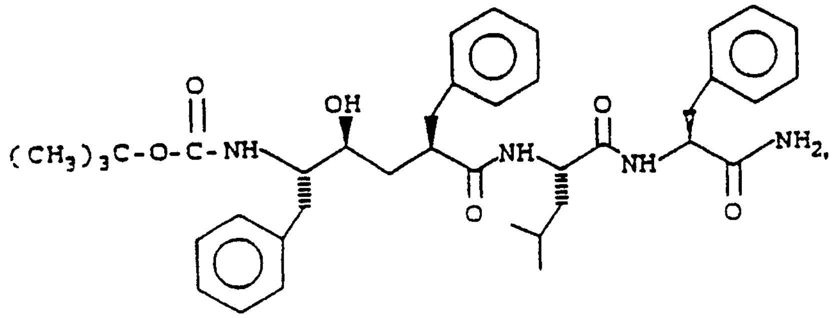

N'-(1,1-dimethylethoxycarbonyl)-5(S)-amino-4(S)-hyrdroxy-6-phenyl-2(R)-(phenylmethyl)hexanoyl-leucyl-phenylalanylamide;

Compound II:

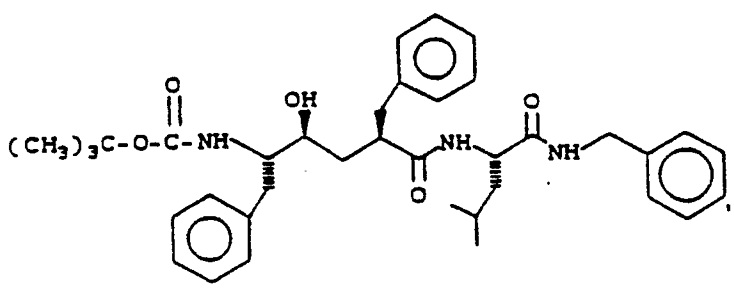

N-Benzyl-N'-(1,1-dimethylethoxycarbonyl)-5(S)-amino-4(S)-hydroxy-6-phenyl-2(R)-(phenylmethyl)hexanoyl-leucyl-amide,

Compound III:

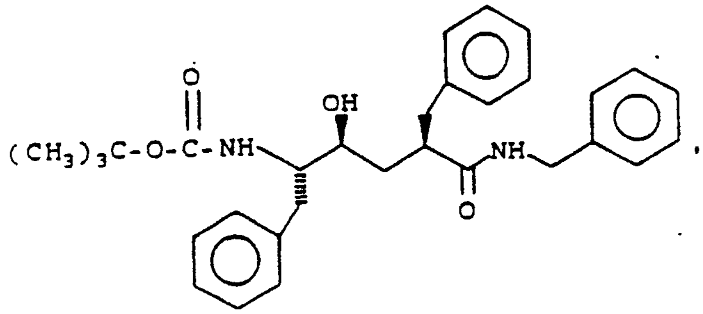

N-benzvl-N'-(1,1-dimethylethoxycarbonyl)-5(S)-amino-4(S)-hvdroxy-6-phenyl-2(R)-(phenylmethyl)-hexanoic carboxamide.

Compound IV:

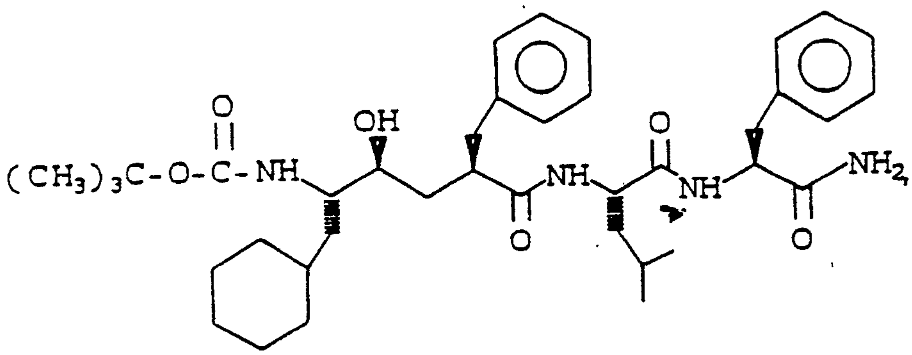

N'-(1,1-dimethylethoxycarbonyl)-5(S)-amino-4(S)-hydroxy-6-cyclohexyl-2(R)-(phenylmethyl)hexanoyl-leucyl-phenylalanylamide:

Compound V:

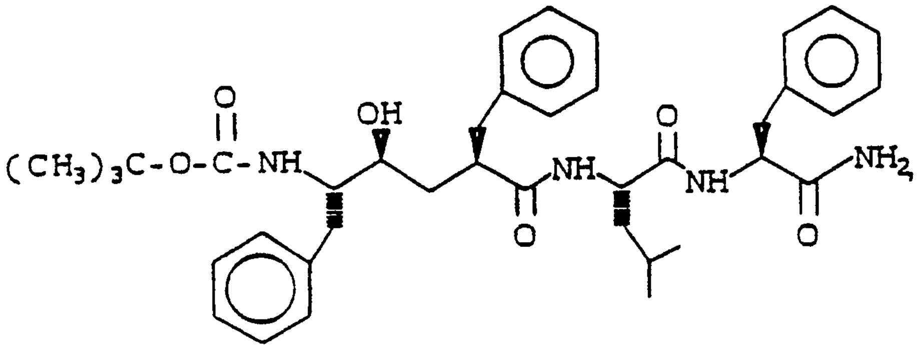

N'-(1,1-dimethylethoxycarbonyl)-5(S)-amino-4(S)-hydroxy-6-phenyl-2(R)-(phenylmethyl)hexanoyl-leucyl-phenylalanine methyl ester;

Compound VI:

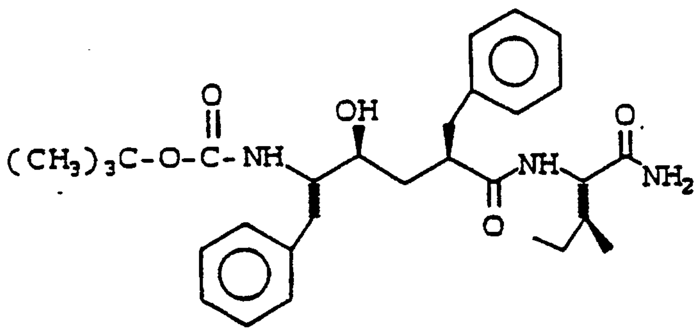

N'-(1,1-dimethylethoxycarbonyl)-5(S)-amino-4(S)-hydroxy-6-phenyl-2(R)-(phenylmethyl)hexanoyl-isoleucylamide: and

Compound VII:

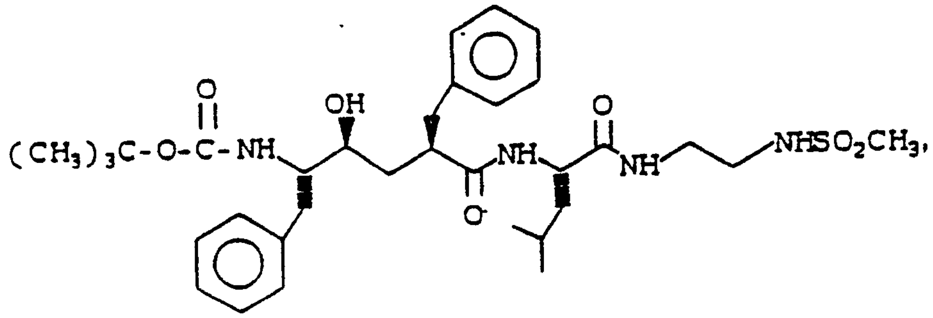

N-(2-(methanesulfonylamino)ethyl)-N'-(1,1-dimethylethoxycarbonyl)-5(S)-amino-4(S)-hydroxy-6-phenyl-2(R)-(phenylmethyl) hexanoyl-leucylamide.

Other compounds of the invention include, but are not limited to: N′-(1,1-Dimethylethoxycarbonyl)-5(S)-amino-4(S)-hydroxy-6-phenyl-2(R)-(phenylmethyl)hexanoyl-leucyl-phenylalanylamide,
N-Benzyl-N′(1,1-Dimethylethoxycarbonyl)-5(S)-amino-4(S)-hydroxy-6-phenyl-2(R)-(phenylmethyl)hexanoyl-leucyl-amide,
5(S)-[(1,1-Dimethylethoxycarbonyl)-amino]-4(S)-hydroxy-6-phenyl-2(R)-(phenylmethyl)hexanoyl-Leu-amide,
N′-(1,1-Dimethylethoxycarbonyl)-5(S)-amino-4(S)-hydroxy-6-cyclohexyl-2(R)-(phenylmethyl)hexanoyl-leucyl-phenylalanylamide,
5(S)-[(1,1-Dimethylethoxycarbonyl)amino]-4(S)-hydroxy-6-phenyl-2(R)(phenylmethyl)hexanoyl Leu-(4-I-Phe)-amide,
5(S)-[(Phenylmethyloxycarbonyl)amino]-4(S)-hydroxy-6-phenyl-2(R)(phenylmethyl)hexanoyl Leu-Phe-amide,
5(S)-[(1,1-dimethylethoxycarbonyl)amino]-4(S)-hydroxy-2(R)-n-butyl-6-phenylhexanoyl Leu-Phe-amide,
3-(1(S)-Benzyloxycarbonylamino-2-phenylethyl phosphinyl)-2(S,R)-(phenylmethyl)propanoyl-L-Leu-Phe-amide,
N-[4-[(Benzyloxycarbonyl)amino]butyl]-5(S)-[(1,1-dimethylethoxy)carbonylamino]-4(S)-hydroxy-6-phenyl-2-(R)-(phenylmethyl)hexanoyl-Leu-amide,
N′-(1,1-Dimethylethoxycarbonyl)-5(S)-amino-4(S)-hydroxy-6-phenyl-2(R)-(phenylmethyl)-hexanoyl-leucyl-phenylalanine methyl ester,
5(S)-[(1,1-Dimethylethoxycarbonyl)-amino]-4(S)-hydroxy-6-phenyl-2(R)-(2-naphthylmethyl)hexanoyl Leucine-Phenylalanine amide,
5(S)-[(1,1-Dimethylethoxycarbonyl)-amino]-4(S)-hydroxy-2(R)-(phenylmethyl)nonanoyl-Leucyl-Phenylalanyl amide,
5(S)-[(1,1-Dimethylethoxycarbonyl)-amino]-4(S)-hydroxy-6-phenyl-2(R)-(phenylmethyl)-hexanoyl-N-(2-hydroxy-1(S)-(phenylmethyl)ethyl-Leu amide,
5(S)-[(1,1-dimethylethoxycarbonyl)-amino]-4(S)-hydroxy-6-phenyl-2(R)-(phenylmethyl)-hexanoyl-Ala amide,
N-(2-(Methanesulfonylamino)ethyl)-N′-(1,1-dimethylethoxycarbonyl)-5(S)-amino-4(S)-hydroxy-6-phenyl-2(R)-(phenylmethyl)hexanoyl-leucylamide,
5(S(-[(1,1-Dimethylethoxycarbonyl)-amino]-4(S)-hydroxy-6-phenyl-2(R)-(phenylmethyl)hexanoyl-Ile amide,
5(S)-[(1,1-Dimethylethoxycarbonyl)-amino]-4(S)-hydroxy-6-phenyl-2(R)-[(4-benzyloxyphenyl)-methyl]-hexanoyl-Leu-Phe amide,
5(S)-[(1,1-Dimethylethoxycarbonyl)-amino]-4(S)-hydroxy-6-phenyl-2(R)-(phenylmethyl)hexanoyl-Ile-Val-amide,
5(S)-[(1,1-Dimethylethoxycarbonyl)-amino]-4(S)-hydroxy-6-phenyl-2(R)-[(4-hydroxyphenyl)methyl]-hexanoyl-Leu-Phe amide,
5(S)-[(1,1-Dimethylethoxycarbonyl)-amino]-4(S)-hydroxy-6-phenyl-2(R)-(methyl)hexanoyl-Leu-Phe-amide,
5(S)-[(1,1-Dimethylethoxycarbonyl)-amino]-4(S)-hydroxy-6-phenyl-2(R)-(phenylmethyl)-hexanoyl-N-(2-hydroxy-1(S)-phenylethyl)Leu amide,
5(S)-[(1,1-Dimethylethoxycarbonyl)-amino]-4(S)-hydroxy-6-phenyl-2(R)-(phenylmethyl)-hexanoyl-N-(2-acetamidoethyl)Leu amide,
5(S)-[(1,1-Dimethylethoxycarbonyl)-amino]-4(S)-hydroxy-6-phenyl-2(R)-(3′phenyl-prop-2′-en-1′-yl)-leucine-phenylalanine-amide,
5(S)-[(1,1-Dimethylethoxycarbonyl)-amino]-4(S)-hydroxy-6-phenyl-2(R)-(phenylmethyl)-hexanoyl-N-(2-hydroxyethyl)Leu amide,
5(S)-[(1,1-Dimethylethoxycarbonyl)-amino]-4(S)-hydroxy-6-phenyl-2(R)-(phenylmethyl)hexanoyl-Ile-benzylamide,
5(S)-[(1,1-Dimethylethoxycarbonyl)amino]-4(S)-hydroxy-6-phenyl-2(R)-(phenylmethyl)hexanoyl-phenylglycine amide,
4(S)-[(1,1-Dimethylethoxycarbonyl)amino]-3(S)-hydroxy-6-methylheptanoyl-glutamyl-phenylalanine amide,
5(S)-[(1,1-Dimethylethoxycarbonyl)amino]-4(S)-hydroxy-6-phenyl-2(R)-(phenylmethyl)hexanoyl-N-(4-dimethylaminopropyl)-Ile-amide,
5(S)-[(1,1-Dimethylethoxycarbonyl)amino]-4(S)-hydroxy-6-phenyl-2(R)-(4′-phenyl-phenylmethyl)-leucine-phenylalanine amide,
5(S)-[(1,1-Dimethylethoxycarbonyl)amino]-4(S)-hydroxy-6-phenyl-2(R)-(phenylmethyl)hexanoyl valine amide,
5(S)-[(1,1-Dimethylethoxycarbonyl)amino]-4(S)-hydroxy-6-phenyl-2(R)-(phenylmethyl)hexanoyl-Leu-Phe-benzyl ester,
5(S)-[(1,1-Dimethylethoxycarbonyl)amino]-4(S)-hydroxy-6-phenyl-2(R)-(4′-phenyl-phenylmethyl)-isoleucine-N-benzyl amide,
5(S)-[(1,1-Dimethylethoxycarbonyl)amino]-4(S)-hydroxy-6-(2-naphthyl)-2(R)-(phenylmethyl)hexanoyl-Leu-Phe amide,
5(S)-[(1,1-Dimethylethoxycarbonyl)amino]-4(S)-hydroxy-2(R),6-diphenylhexanoyl-Leu-Phe amide,
5(S)-[(1,1-Dimethylethoxycarbonyl)amino]-4(S)-hydroxy-6-phenyl-2(R)-(phenylmethyl)hexanoyl-N-(2-dimethylamino ethyl)-Ile amide,
5(S)-[(1,1-Dimethylethoxycarbonyl)amino]-4(S)-hydroxy-6-phenyl-2(R)-(phenylmethyl)hexanoyl-N-(2-pyridylmethyl)Ile amide,
5(S)-[(1,1-Dimethylethoxycarbonyl)amino]-4(S)-hydroxy-6-(2-naphthyl)-2(R)-(phenylmethyl)hexanoyl-Ile-benzylamide,
5(S)-[(1,1-Dimethylethoxycarbonyl)amino]4(S)-hydroxy-6-phenyl-2(R)-(phenylmethyl)-hexanoyl-Ile-Phe amide,
N-(Methyl-5-amino-5-deoxy-2,3,0-isopropylidene-β-D-ribosyl)-5(S)-(1,1-dimethylethoxycarbonylamino)-4(S)-hydroxy-6-phenyl-2(R)(phenylmethyl)-hexanoyl Ile amide,
5(S)-[(1,1-Dimethylethoxycarbonyl)amino]-4(S)-hydroxy-6-phenyl-2(R)-(phenylmethyl)hexanoyl-S-neopentylglycine amide,
N-(2(S,R)-Hydroxy-1(R,S)-indanyl)-5(S)-(1,1-dimethylethoxycarbonylamino)-4(S)-hydroxy-6-phenyl-2(R)-phenylmethylhexanamide,
5(S)-(1,1-Dimethylethoxycarbonylamino)-4(S)-hydroxy-6-phenyl-2(R)-(3′-phenylpropyl)hexanoyl-leucine-phenylalanine amide,
5(S)-(1,1-Dimethylethoxycarbonylamino)-4(S)-hydroxy-6-phenyl-2(R)-(phenylmethyl)hexanoyl-N-(4-dimethylamino propyl)-Val amide,
N-(Methyl-5-amino-5-deoxy-β-D-ribosyl)-5(S)-(1,1-dimethylethoxycarbonylamino)-4(S)-hydroxy-6-phenyl-2-(R)-(phenylmethyl)hexanoyl-Ile amide,
5(S)-[(1,1-Dimethylethoxycarbonyl)amino]-4(S)-hydroxy-6-phenyl-2(R)-(phenylmethyl)hexanoyl-S-tert-butylglycine-benzylamide,
5(S)-[(1,1-Dimethylethoxycarbonyl)amino]-4(S)-hydroxy-6-phenyl-2(R)-(phenylmethyl)hexanoyl-N-(2,3-dihydroxy-propyl)-Ile amide,
5(S)-[(1,1-Dimethylethoxycarbonyl)amino]-4(S)-hydroxy-6-phenyl-2(R)-(phenylmethyl)hexanoyl-cyclohexylglycine-amide,
5(S)-[(1,1-Dimethylethoxycarbonyl)amino]-4(S)-hydroxy-6-phenyl-2(R)-(3′phenylprop-2′-en-1′yl)hexanoyl-N-(3-dimethyl amino propyl) valine amide,
N′-(1,1-Dimethylethoxycarbonyl)-5(S)-amino-4(S)-hydroxy-6-phenyl-2(R)-(3′-phenylprop-2′-en-1′-yl) hexanoyl-(S)-phenylglycyl-(2-hydroxyethyl)amide,
5(S)-[1,1-Dimethylethoxycarbonylamino]-4(S)-hydroxy-6-phenyl-2(R)-(phenylmethyl)hexanoyl-N-(phenylmethyl)-valine amide,
N-(cis-2(S,R)Hydroxy-1(R,S)-indanyl)-5(S)-(1,1-dimethylethoxycarbonylamino)-4(S)-hydroxy-6-(4-benzyloxyphenylmethyl)-2(R)-(phenylmethyl)hexanamide,

5(S)-(1,1-Dimethylethoxycarbonylamino)-4(S)-hydroxy-6-phenyl-2(R)-(phenylmethyl)-hexanoyl-N-[2-(imidazol-4-yl)-1-(hydroxymethyl)-propyl]-Ile-amide,
4(S)-[(1,1-Dimethylethoxycarbonyl)amino]-3(S)-hydroxy-5-cyclohexylpentanoyl-glutamyl-phenylalanine amide,
5(S)-[(1,1-Dimethylethoxycarbonyl)amino]-4(S)-hydroxy-6-phenyl-2(R)-(3′phenylprop-2′-en-1′-yl) hexanoyl --(S)-phenylglycine amide,
5(S)-[(1,1-Dimethylethoxycarbonyl)amino]-4(S)-hydroxy-6-phenyl-2(R)-(4-nitrophenylmethyl)phenylglycine amide,
5(S)-[1,1-Dimethylethoxycarbonylamino]-4(S)-hydroxy-6-phenyl-2(R)-(phenylmethyl)hexanoyl-phenylglycine amide,
N-(2(S or R)-hydroxy-1,2,3,4-tetrahydro-1(R or S)-naphthyl)-5(S)-(1,1-dimethylethoxycarbonylamino)-4(S)-hydroxy-6-phenyl-2(R)-phenylmethyl hexanamide,
N-(2(R or S)-hydroxy-1,2,3,4-tetrahydro-1(S or R)-naphthyl)-5(S)-(1,1-dimethylethoxycarbonylamino)-4(S)-hydroxy-6-phenyl-2(R)-phenylmethyl hexanamide,
N-(cis-2(S,R)-Hydroxy-1(R,S)-indanyl)-5(S)-(1,1-dimethylethoxycarbonylamino)-4(S)-hydroxy-6-(4-hydroxyphenyl)-2(R)-phenylmethylhexanamide,
5(S)-(1,1-Dimethylethoxycarbonyl)amino-4(S)-hydroxy-6-phenyl-2(R)-(phenylmethyl)hexanoyl-N-(2,3-hydroxypropyl)-Val-amide.
N-(cis-2(R)-Hydroxy-1(S)-indanyl)-5(S)-(1,1-dimethylethoxycarbonylamino)-4(S)-hydroxy-6-phenyl-2(R)-phenylmethyl-hexanamide,
5(S)-(1,1-Dimethylethoxycarbonylamino)-4(S)-hydroxy-3(S or R)-hydroxy-6-phenyl-2(R)-phenylmethyl-hexanoyl-Leu-Phe-amide,
5(S)-(1,1-Dimethylethoxycarbonylamino)-4(S)-hydroxy-6-phenyl-2(R)-(3′-phenylprop-2-en-1′-yl)-hexanoyl-N-(2(R,S),3-dihydroxypropyl)phenylglycine amide,
5(S)-[(1,1-Dimethylethoxycarbonyl)amino]-4(S)-hydroxy-6-phenyl-2(R)-(phenylmethyl)hexanoyl-N-(2-benzimidazolyl methyl)Ile amide
5(S)-(1,1-Dimethylethoxycarbonylamino)-4(S)-hydroxy-6-phenyl-2(R)-(phenylmethyl)hexanoyl-N-(benzyl)-allo-Ile amide,
N-(Methyl-5-amino-5-deoxy-β-D-ribosyl)-5(S)-(1,1-dimethylethoxycarbonylamino)-4(S)-hydroxy-6-phenyl-2-(R)-phenylmethylhexanoyl-Val amide,
N-(Methyl-5-amino-5-deoxy-2,3,0-isoproplidene-β-D-ribosyl)-5(S)-(1,1-dimethylethoxycarbonylamino)-4(S)-hydroxy-6-phenyl-2(R)-(phenylmethyl)hexanoyl-Val amide,
5(S)-(1,1-Dimethylethoxycarbonylamino)-4(S)-hydroxy-6-phenyl-2(R)(phenylmethyl)hexanoyl-N-[2-(2-methoxyethoxy)ethyl]isoleucine amide,
5(S)-(1,1-Dimethylethoxycarbonylamino)-4(S)-hydroxy-6-phenyl-2(R)-(3′phenylpropyl)hexanoyl-N-(2″(R,S),3″-dihydroxypropyl)-phenylglycine amide,
[4(S)-(1,1-Dimethylethoxycarbonylamino)-3(S)-hydroxy-5-phenylpentanoyl]-S-glutamyl-S-phenylalanine amide,
5(S)-[(1,1-Dimethylethoxycarbonyl)amino]-4(S)-hydroxy-6-phenyl-2(R)-(phenylmethyl)hexanoyl-N-[2-(4-imidazolyl)ethyl] valine amide,
N-(cis-2(R)-Hydroxy-1(S)-indanyl)-5-(S)-(1,1-dimethylethoxycarbonylamino)-4(S)-hydroxy-6-phenyl-2(R)-(3′-phenylprop-2′-en-1′-yl)hexanamide,
N-(1(R or S), 2(S or R)-Dihydroxy-3-(S or R)-indanyl)-5(S)-(1,1-dimethylethoxycarbonylamino)-4(S)-hydroxy-6-phenyl-2(R)-(phenylmethyl)hexanamide,
N-[2(R)-Hydroxy-1(S)-indanyl]-5(S)-[(1,1-dimethylethoxycarbonyl)amino]-4(S)-hydroxy-6-(4-hydroxyphenyl)-2(R)-(phenylmethyl) hexanamide,
5(S)-[(1,1-Dimethylethoxycarbonyl)amino]-4(S)-hydroxy-6-phenyl-2(R)-((4-iodophenyl)methyl)hexanoyl valine amide,
N-(cis-2(R)-Hydroxy-1(S)-indanyl)-5(S)-((1,1-dimethylethoxycarbonyl)amino)-4(S)-hydroxy-6-phenyl-2(R)-((4-iodophenyl)methyl)hexanamide,
5(S)-[(1,1-Dimethylethoxycarbonyl)amino]-4(S)-hydroxy-6-phenyl-2(R)-(phenylmethyl)hexanoyl-N-(2-(1-piperidinyl)ethyl)valine amide,
5(S)-[(1,1-Dimethylethoxycarbonyl)amino]-4(S)-hydroxy-6-phenyl-2(R)-(phenylmethyl)hexanoyl-N-(2-hydroxyethyl)valine amide,
5(S)-[(1,1-Dimethylethoxycarbonyl)amino]-2(R)-phenylmethyl-4(S)-hydroxy-6-phenyl-hexanoyl-N-(2-[(2-methoxyethoxy)-ethoxy]ethyl)isoleucine amide,
5(S)-(1,1-Dimethylethoxycarbonylamino)-2(R)-phenylmethyl-4(S)-hydroxy-6-phenylhexanoyl-N-[2-(5-dimethylaminonaphthylsulfonamido)ethyl]isoleucine amide,

5(S)-(1,1-Dimethylethoxycarbonylamino)-4(S)-hydroxy-6-phenyl-2(R)-(phenylmethyl)hexanoyl-N-(3-diethylamino-2(R,S)-hydroxypropyl)valine amide,
N-(1(R or S), 2(S or R)-dihydroxy-3-(S or R)-indanyl)-5(S)-(1,1-dimethylethoxycarbonylamino)-4(S)-hydroxy-6-(4′-hydroxyphenyl)-2(R)-(phenylmethyl)hexanamide,
N-(cis-2(R)-Hydroxy-1(S)-indanyl)-5(S)-(1,1-dimethylethoxycarbonylamino)-4(S)-hydroxy-6-(3-dimethylaminomethyl-4-hydroxyphenyl)-2(R)-(phenylmethyl)hexanamide,
5(S)-[(1,1-Dimethylethoxycarbonyl)amino]-4(S)-hydroxy-6-phenyl-2(R)-(phenylmethyl)-hexanoyl-N-(2-benzimidazoylmethyl)valine amide,
5(S)-[(1,1-Dimethylethoxycarbonyl)amino]-4(S)-hydroxy-6-phenyl-2(R)-(phenylmethyl)-hexanoyl-N-[2-(3-(1H-imidazol-4-yl))-1-hydroxymethylethyl]valine amide,
N-(cis-2(R)-Hydroxy-1(S)-indanyl)-5(S)-[(1,1-dimethylethoxycarbonyl)amino]-4(S)-hydroxy-6-(2-naphthyl)-2-(R)-(phenylmethyl)hexanamide,
5(S)-[(1,1-Dimethylethoxycarbonyl)amino]-4(S)-hydroxy-6-phenyl-2(R)-(phenylmethyl)-hexanoyl-N-(4-dimethylaminobenzyl)valine amide,
5(S)-[(1,1-Dimethylethoxycarbonyl)amino]-4(S)-hydroxy-6-phenyl-2(R)-(phenylmethyl)-hexanoyl-N-(2-benzimidazolylmethyl)phenylglycineamide,
3[1-(1R)-(1,1-Dimethylethoxycarbonylamino)-2-(phenylethyl)phosphinyl]-2(S or R)-phenylmethyl-N-(2(R)-hydroxy-1-indanyl)propanamide,
5(S)-[(1,1-Dimethylethoxycarbonyl)amino]-4(S)-hydroxy-6-phenyl-2(R)-(phenylmethyl)hexanoyl-N-(2-benzimidazolyl)valine amide,
N-(2(R)-hydroxy-1(S)-indanyl)-5(S)-[(4-pyridinylmethoxycarbonyl)amino]-4(S)-hydroxy-6-phenyl-2(R)-(phenylmethyl)hexanamide,
N-(2(R)-hydroxy-1(S)-indanyl)-N′-[(1-methylethoxycarbonyl)amino]-4(S)-hydroxy-6-phenyl-2(R)-(phenylmethyl)hexanamide,
5(S)-(1,1-Dimethylethoxycarbonylamino)-4(S)-hydroxy-6-phenyl-2(R)-(4′-iodophenylmethyl)hexanoyl-N-(3′-dimethylaminopropyl)-valine amide,
N-(benzyl)-5(S)-(1,1-dimethylethoxycarbonylamino)-4(S)-hydroxy-6-phenyl-2(R)-(phenylmethyl)hexanoyl valine thiomide,
N-(1(R),2(S)-dihydroxy-3(S)-indanyl)-N′-(1,1-dimethylethoxycarbonylamino)-4(S)-hydroxy-6-phenyl-2(R)-(3′phenylprop-2′en-yl)hexanamide,
N-[4(S)-benzopyranyl]-5(S)-(1,1-dimethylethoxycarbonylamino)-4(S)-hydroxy-6-phenyl-2(R)-(phenylmethyl)-hexanamide,
N-[4(R,S)-benzopyranyl]-5(S)-(1,1-dimethylethoxycarbonylamino)-4(S)-hydroxy-6-phenyl-2(R)-(phenylmethyl)hexanoyl-valine amide,
N-(allyl-5-amino-5-deoxy-2,3-0-isopropylidene-β-D-ribosyl)-5(S)-(1,1-dimethylethoxycarbonylamino)-4(S)-hydroxy-6-phenyl-2(R)-(phenylmethyl)hexanoyl-valine amide,
5(S)-[(1,1-dimethylethoxycarbonyl)amino]-4(S)-hydroxy-6-phenyl-2(R)-(phenylmethyl)hexanoyl-N-(2-methylaminoethyl)-valine amide,
N-(cis-1-hydroxy-cyclopent-2-en-3-yl)-5(S)-(1,1-dimethylethoxycarbonylamino)-4(S)-hydroxy-6-phenyl-2(R)-(phenylmethyl)hexanamide,
5(S)-[(1,1-dimethylethoxycarbonyl)amino]-4(S)-hydroxy-6-phenyl-2(R)-(phenylmethyl)hexanoyl-N-(3-hydroxy-5-isoxazoloyl-methyl)valine amide,
N-(cis-2(R)-hydroxy-1(S)-indanyl)-5(S)-benzyloxycarbonylamino -4(S)-hydroxy-6-phenyl-2(R)-(phenylmethyl)-hexanamide,
N-(2-hydroxyethyl)-5(S)-(1,1-dimethylethoxycarbonylamino)-4(S)-hydroxy-2(R)-(phenylmethyl)hexanoyl-isoleucyl-amide,
5(S)-(1,1-dimethylethoxycarbonylamino)-4(S)-hydroxy-6-phenyl-2(R)-(phenylthiomethyl)-N-(2-hydroxyethyl)-phenyl glycine amide,
N-(cis-2(R or S)-aminocarbonyl-1(S or R)-indanyl)-5(S)-(1,1-dimethylethoxycarbonylamino)-4(S)-hydroxy-6-phenyl-2(R)-(phenylmethyl)hexanamide,
N-(2(R)-hydroxy-1(S)-indanyl)-5(S)-[(1,1-dimethylethoxycarbonyl)amino]-4(S)-hydroxy-6-(4-hydroxyphenyl)-2-(R)-(4-hydroxyphenylmethyl)hexanamide,
N-(cis-2(R or S)-carboxy-1(S or R)-indanyl)-5(S)-(1,1-dimethylethoxycarbonylamino)-4(S)-hydroxy-6-phenyl-2(R)-(phenylmethyl)hexanamide,
N-(cis-2(R)-hydroxy-1(S)-indanyl)-5-(1,1-dimethylethoxycarbonylamino)-4(S)-hydroxy-6-phenyl-2(R)-(4-nitrophenylmethyl)hexanamide,
5(S)-(1,1-dimethylethoxycarbonylamino)-4(S)-hydroxy-6-phenyl-2(R)-(4-nitrophenylmethyl)-hexanoyl-N-(2-hydroxyethyl)-phenylglycine amide,

N-(cis-2(R)-hydroxy-1(S)-indanyl)-5(S)-(1,1-dimethylethoxycarbonylamino)-4(S)-hydroxy-6-phenyl-2(R)-(4-aminophenylmethyl)-hexanamide,
N′-(1,1-dimethylethoxycarbonyl)-5(S)-amino-4(S)-hydroxy-6-phenyl-2(R)-(phenylmethyl)hexanoyl-N-[3-((4′-morpholino)propyl)]-valine amide,
N-[5-amino-5-deoxy-D-ribosyl]-5(S)-(1,1-dimethylethoxycarbonylamino)-4(S)-hydroxy-6-phenyl-2(R)-phenylmethyl-hexanoyl-valine amide,
N-(5-amino-5-deoxy-2,3,0-isopropylidene-D-ribosyl)-5(S)-(1,1-dimethylethoxycarbonylamino)-4(S)-hydroxy-6-phenyl-2(R)-(phenylmethyl)hexanoyl-valine amide,
5(S)-[(1,1-dimethylethoxycarbonyl)amino]-4(S)-hydroxy-6-phenyl-2(R)-(phenylmethyl)hexanoyl-N-(4-pyridylmethyl)valine amide,
5(S)-[(1,1-dimethylethoxycarbonyl)amino]-4(S)-hydroxy-6-phenyl-2(R)-(phenylmethyl)hexanoyl-N-(2-pyridylmethyl)valine amide,
5(S)-[(1,1-dimethylethoxycarbonyl)amino]-4(R)-benzyl-4(S)-hydroxy-6-phenylhexanoyl-N-(2-[2-(2-methoxyethoxy)ethoxy]ethyl)valine amide,
5(S)-[(1,1-dimethylethoxycarbonyl)amino]-2(S)-hydroxy-6-phenyl-2(R)-(phenylmethyl)hexanoyl-N-(3-pyridylmethyl)valine amide,
N-[cis-3(S)-hydroxy-4(S)-benzopyranyl]-5(S)-(1,1-dimethylethoxycarbonylamino)-4(S)-hydroxy-6-phenyl-2(R)-phenylmethyl-hexanamide,
N-(1R,2S-dihydroxy-3S-indanyl)-5(S)-(1,1-dimethylethoxycarbonylamino)-4(S)-hydroxy-6-(4′-hydroxyphenyl)-2(R)-(4′-hydroxyphenyl)methylhexanamide,
N-(cis-2(R)-hydroxy-1(S)-indanyl)-5(S)-(1,1-dimethylethoxycarbonylamino)-4(S)-hydroxy-6-phenyl-2(R)-(phenylthiomethyl)hexanamide,
Dilithium N-(2-phosphoryloxyethyl)-5(S)-(1,1-dimethylethoxycarbonylamino)-4(S)-hydroxy-2(R)-(phenylmethyl)hexanoyl-isoleucylamide,
N-(Methyl-5-amino-5-deoxy-(αβ)-D-xylosyl)-5(S)-(1,1-dimethylethoxycarbonylamino)-4(S)-hydroxy-6-phenyl-2(R)-(phenylmethyl)hexanoyl-Ile amide,
5(S)-[(4-pyridylmethoxycarbonyl)amino]-4(S)-hydroxy-6-phenyl-2(R)-(phenylmethyl)hexanoyl-N-(2-benzimidazolylmethyl)-Ile amide,
N-(cis-2(R)-hydroxy-1(S)-indanyl)-5(S)-(1,1-dimethylethoxycarbonylamino)-4(S)-hydroxy-6-phenyl-2(R)-(4-(1,1-dimethylethyl)phenylmethyl)hexanamide,
or pharmaceutically acceptable salt thereof.

The pharmaceutically-acceptable salts of the compounds of Formula I (in the form of water- or oil-soluble or dispersible products) include the conventional non-toxic salts or the quaternary ammonium salts of these peptides, which are formed, e.g., from inorganic or organic acids or bases. Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others.

HIV protease inhibitors of Formula I may be prepared in accordance with well-known procedures for preparing peptide analogs from their constituent amino acids or analogs thereof. In general, once the G substituent is made, the rest of the synthesis follows the principle of amide bond formation by the coupling methods of either solution-phase or solid-phase peptide synthesis. The addition and removal of one or more protecting groups is also typical practice.

Structures and abbreviations for the G components of the inhibitors of the present invention include:

1. 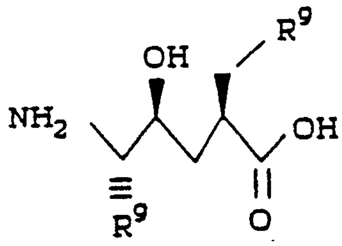

,

a hydroxyethylene dipeptide isostere prepared, for example, by an intermediate lactone according to Evans, B.E. et al J. Org. Chem. 50, 4615(1985) or Kempf, D.J. J. Org. Chem. 51, 3921(1986). Synthetic routes to similar peptide bond isosteres, such as

2. 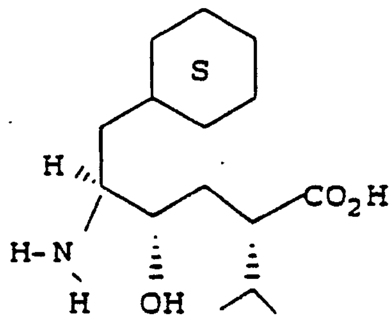

Cal[CH(OH)$CH_2$]Val,

are readily available. The intermediate BOC-Cal[CH(OH)$CH_2$]Val-OH lactone is obtained from intermediates prepared using methods described by P. Buhlmayer et al, in Published European Patent Application 184,550-A2. Other synthetic routes to peptide bond isosteres of like character are described in the following:

a. Szelke et al, in Peptides, Structure and Function. Proceedings of the Eighth American Peptide Symp(ed. V.J. Hruby and D.H. Rich) pp. 579-82, Pierce Chemical Co., Rockford, IL;

b. D.T. Pals et al in European Patent Appln. 173,481-A2;

c. A.H. Fray et al, J. Org. Chem., 51, 4828-4833 (1986); and

d. M. Szelke et al, PCT Int. Appl. WO 84 03.044;

Structures and abbreviations for other G components of the inhibitors of the present invention include:

3. 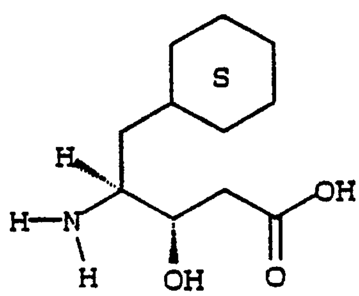

ACHPA

with BOC-ACHPA-OEt being prepared by the method described by Boger et al., J. Med. Chem., 1985, 28, 1779-1790;

4. 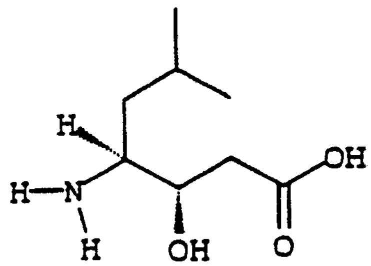

Statine (Sta)

with BOC-Sta-OEt being prepared in accordance with the procedure described by Rich et al., J. Org. Chem., 43, 3624 (1978);

5. 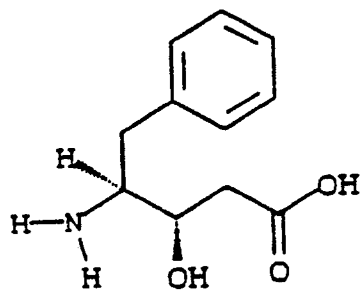

AHPPA

with BOC-AHPPA-OEt being prepared as described by Rich et al., J. Org. Chem., 23, 27-33 (1980);

6. 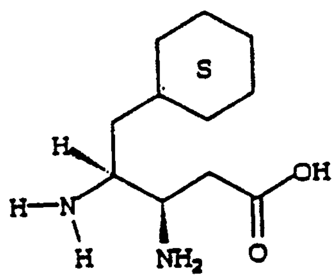

AmACHPA

7. 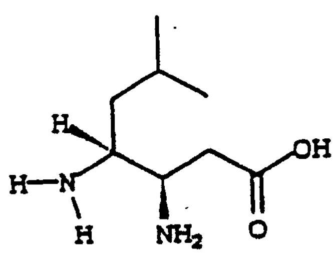

AmSta

with BOC-AmSta(CBZ)-OH being prepared as shown in the following Scheme, and BOC-AmACHPA(CBZ)-OH being prepared as illustrated in the Scheme by substituting BOC-ACHPA-OEt for BOC-Sta-OEt:

Synthesis of protected 3-amino-3-deoxy-(3S, 4S)-statine:

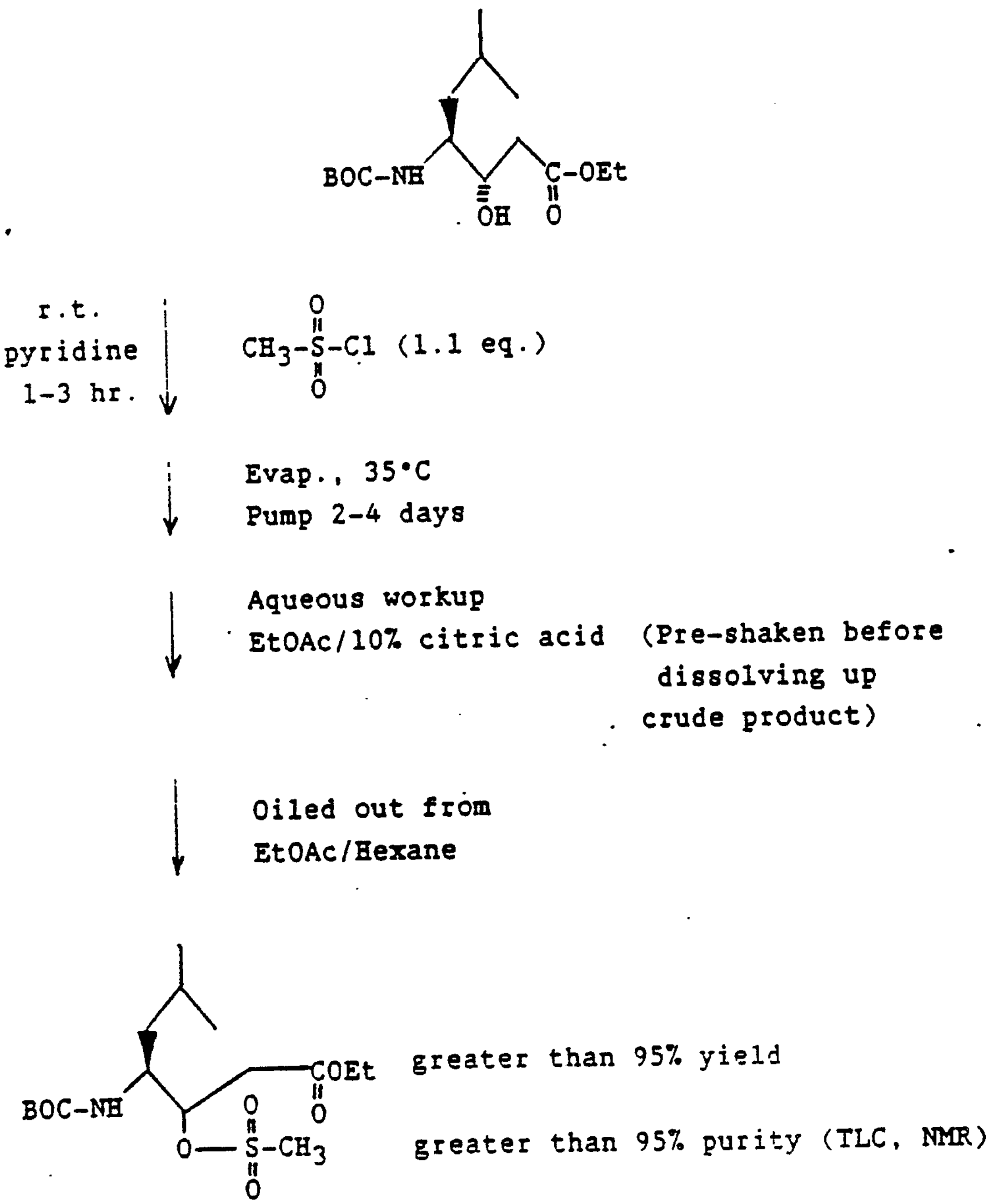
r.t.
pyridine
1-3 hr.
(1.1 eq.)
Evap., 35°C
Pump 2-4 days
Aqueous workup
EtOAc/10% citric acid
(Pre-shaken before
dissolving up
crude product)
Oiled out from
EtOAc/Hexane
greater than 95% yield
greater than 95% purity (TLC, NMR)

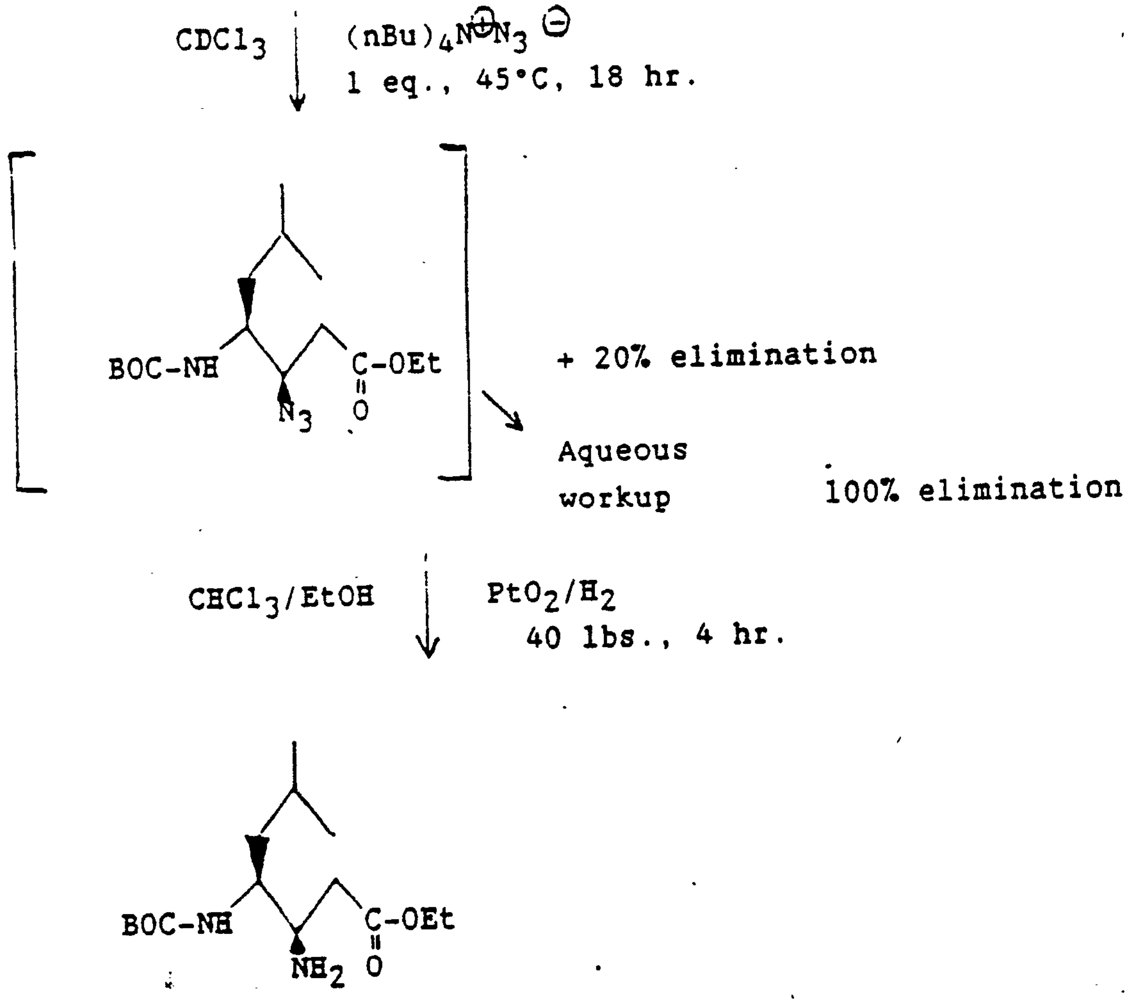


The amine is isolated by extraction into weak acid. Protection of the amine function is performed as follows:

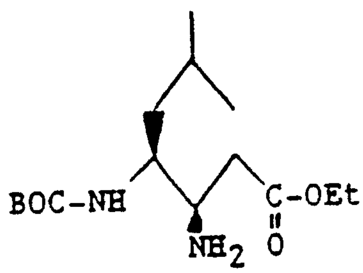

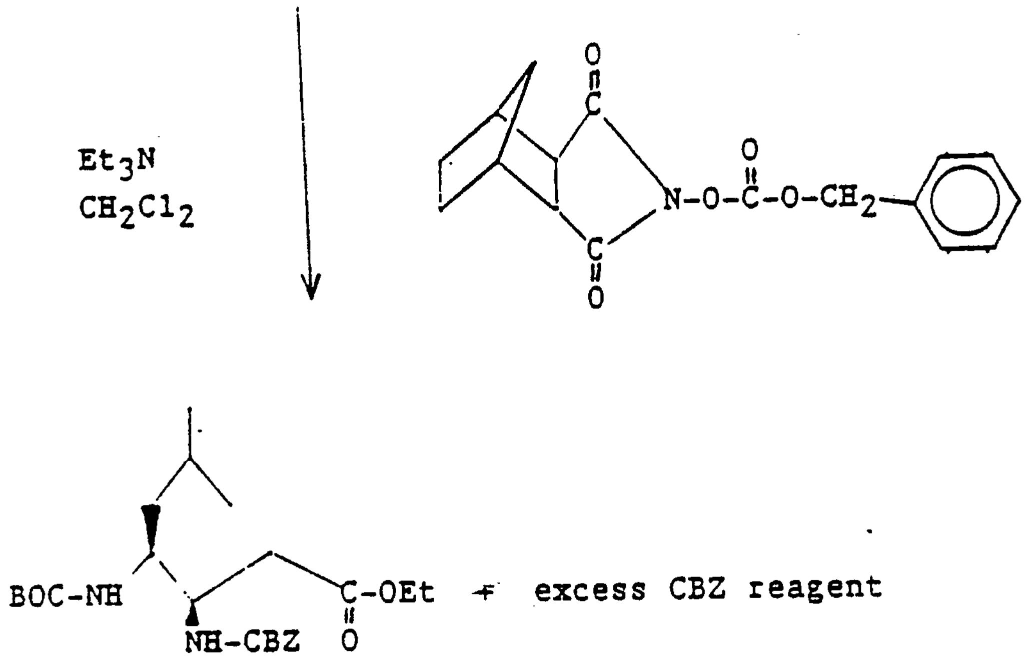


(predominantly R at position 3 as shown).

Base hydrolysis gives the free acid for incorporation into the synthesis of the compounds of Formula I; or alternatively, this material may be prepared as described by Jones et al, in Peptides, Structure and Function. Proceedings of the Ninth American Peptide Symposium (eds. C. M. Deber, V.J. Hruby and K. D. Kopple) pp. 759-62, 1985, Pierce Chemical Co., Rockford, IL.; Arrowsmith et al, J. Chem. Soc. Chem. Commun. 755-7 (1986); and Raddatz et al, Published Eur. Pat. Appl. 161,588;
with efficient methods for preparing the 2-substituted statine component G (such as

8. 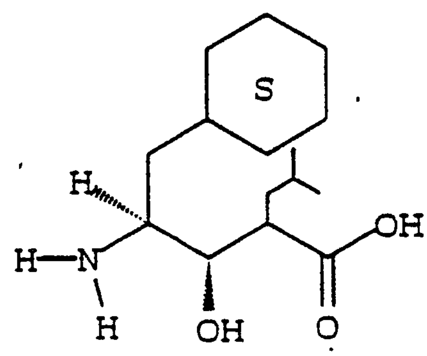

(2-isobutyl)ACHPA

in a suitably protected form being described in Pub. Eur. Pat Appln. 157,409 (with other pertinent references including D. Veber et al, Biochem. Soc. Trans., 12, 956-959 (1984) and H. Stein et al, Fed. Proc. 45, 869, Abstract No 4151 (1986)).

Amide couplings used to form the compounds of this invention are typically performed by the carbodiimide method with reagents such as dicyclohexylcarbodiimide, or N-ethyl, N′-(3-dimethylaminopropyl) carbodiimide. Other methods of forming the amide or peptide bond include, but are not limited to synthetic routes via an acid chloride, azide, mixed anhydride or activated ester. Typically, solution phase amide couplings are performed, but solid-phase synthesis by classical Merrifield techniques may be employed instead.

The selection of protecting groups is, in part, dictated by particular coupling conditions, in part by the amino acid and peptide components involved in the reaction. Such amino-protecting groups ordinarily employed include those which are well known in the art, for example, urethane protecting substituents such as benzyloxycarbonyl (carbobenzoxy), p-methoxycarbobenzoxy, p-nitrocarbobenzoxy, t-butyloxycarbonyl, and the like. It is preferred to utilize t-butyloxycarbonyl (BOC) for protecting the α-amino group in the amino acids undergoing reaction at the carboxyl end of said amino acid, in part because the BOC protecting group is readily removed by relatively mild acids such as trifluoroacteic acid (TFA), or hydrogen chloride in ethyl acetate.

The OH group of Thr, Tyr or Ser and analogs thereof may be protected by the Bzl (benzyl) group and the epsilon-amino group of Lys may be protected by the IPOC group or the 2-chlorobenzyloxycarbonyl (2-Cl-CBZ) group. Treatment with HF or catalytic hydrogenation are typically employed for removal of IPOC or 2-Cl-CBZ.

One scheme for preparing compounds of Formula I is presented below. Example I specifically illustrates the application of the following Scheme (I) to specific compounds.

## SCHEME I

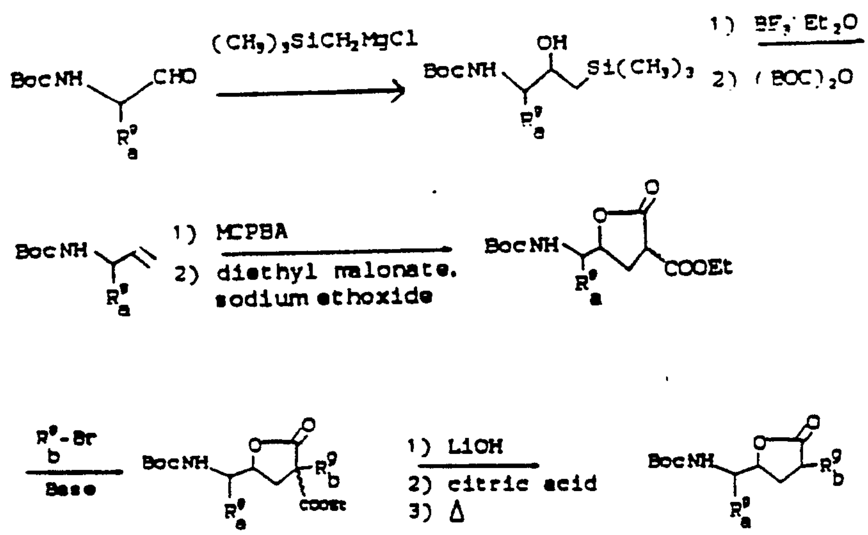


Illustrations of the product of Scheme I includes the following compounds of Table I

TABLE I

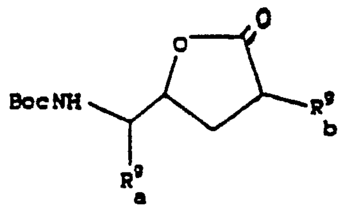

| $R^9_a$ | $R^9_b$ |
|---|---|
| $PhCH_2-$ | $-H$ |
| $PhCH_2-$ | $-(CH_2)_3CH_3$ |
| 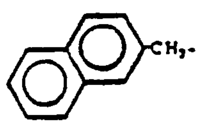 | $-CH_2Ph$ |

| $R^9_a$ | $R^9_b$ |
|---|---|
| $PhCH_2-$ | 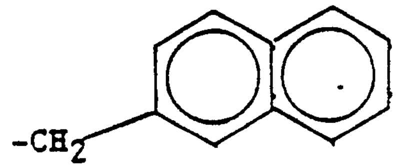 |
| $PhCH_2-$ | 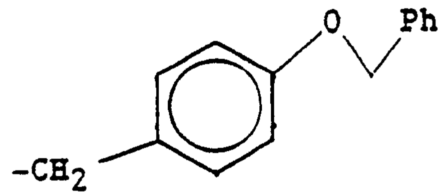 |
| $PhCH_2-$ | 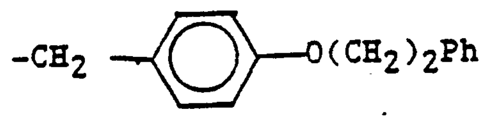 |
| $PhCH_2-$ | $-CH_3$ |
| $PhCH_2-$ | 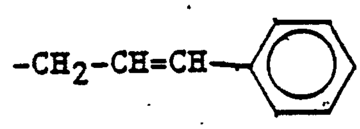 |
| $PhCH_2-$ | 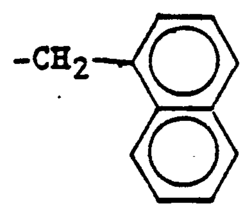 |
| $PhCH_2-$ | $-(CH_2)_2OCH_3$ |

| $R^9{}_a$ | $R^9{}_b$ |
|---|---|
| $PhCH_2-$ | $-CH_2-$ 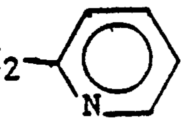 |
| 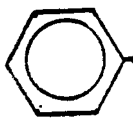 $-CH_2-$ | $-CH_2Ph$ |
| $CH_3(CH_2)_3-$ | $-CH_2PH$ |
| $PhCH_2O-$ 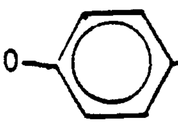 $-CH_2-$ | $-CH_2Ph$ |
| $PhCH_2O-$ 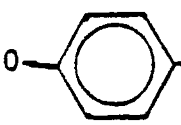 $-CH_2-$ | $-CH_2CH{=}CH-$ 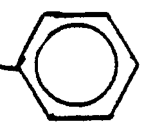 |
| $PhCH_2OCH_2-$ | $-CH_2Ph$ |
| $PhCH_2O\overset{CH_3}{C}H-$ | $-CH_2-$ 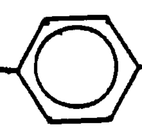 $-OCH_2PH$ |
| $(CH_3)_2CHCH_2-$ | $-CH_2CH(CH_3)_2$ |
| $PhCH_2-$ | $-\overset{CH_3}{C}HCH_2CH_3$ |
| $(CH_3)_2CH-$ | $-CH_2Ph$ |

| $R^9_a$ | $R^9_b$ |
|---|---|
| 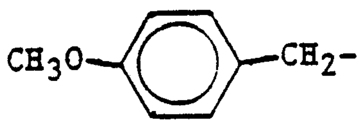 | $-CH_2Ph$ |
| 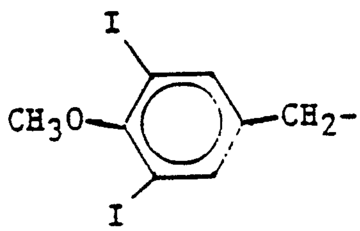 | $-CH_2Ph$ |
| $PhCH_2-$ | 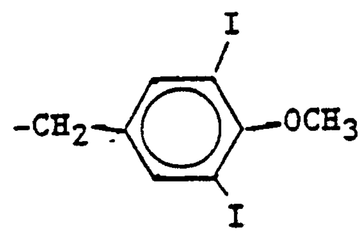 |
| $PhCH_2-$ | $-(CH_2)_3Ph$ |
| $PhCH_2-$ | 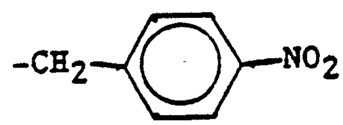 |
| $PhCH_2-$ | 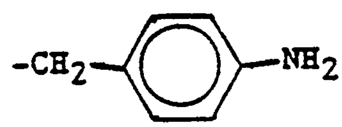 |
| $PhCH_2-$ | 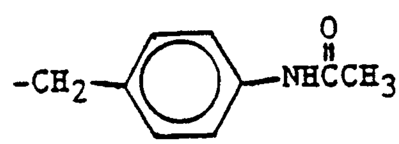 |

| $R^9_a$ | $R^9_b$ |
|---|---|
| 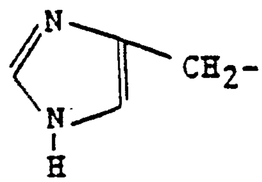 | $-CH_2Ph$ |
| $PhCH_2-$ | 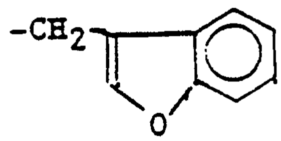 |
| $PhCH_2-$ | 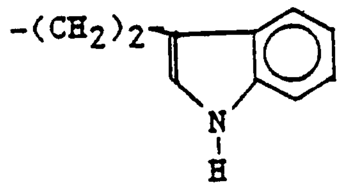 |
| 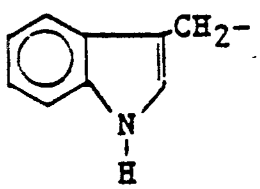 | 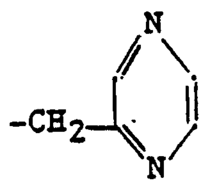 |
| $PhCH_2-$ | $-CH_2SPh$ |
| $PhCH_2-$ | 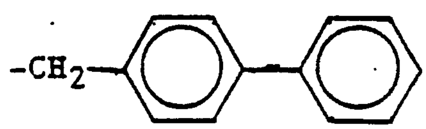 |

| $R^9_a$ | $R^9_b$ |
|---|---|
| $PhCH_2-$ | $CH_3$ |
| $PhCH_2-$ | $-CH_2CH_3$. |

B groups are added onto the carboxyl terminal end by, for example, amide coupling in Scheme II. See Example 1 for specific illustration.

SCHEME II

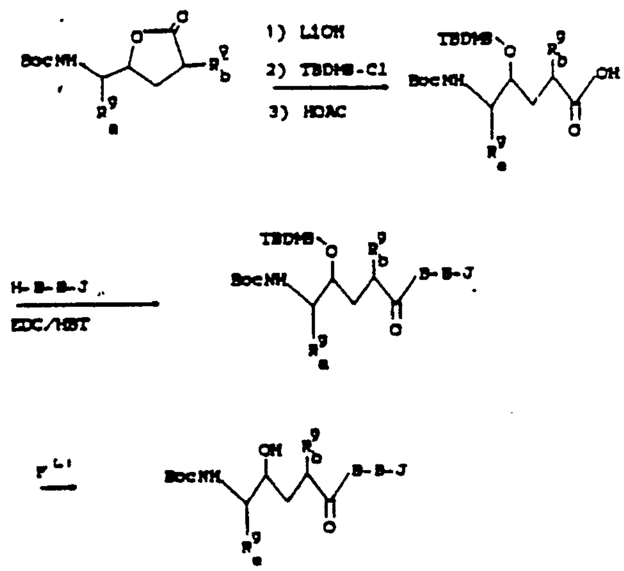


Products of Scheme II include compounds with substituents listed in Table II.

## TABLE II

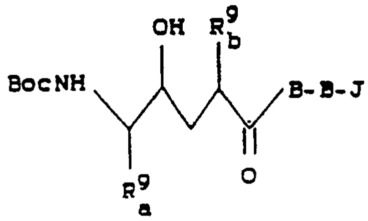

| $R^9_a$ | $R^9_b$ | B-B-J |
|---|---|---|
| $PhCH_2-$ | $-CH_2-CH=CH-Ph$ | -LeuPhe[$NH_2$] |
| 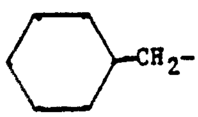 | $-CH_2Ph$ | -LeuPhe[$NH_2$] |
| $PhCH_2-$ | $-CH_2Ph$ | -Ile[$NH_2$] |
| $PhCH_2-$ | $-CH_2Ph$ | 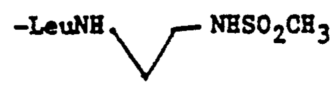 |
| $PhCH_2-$ | $-CH_2Ph$ | -LeuPhe[$OCH_3$] |
| $PhCH_2-$ | $-(CH_2)_3CH_3$ | -LeuPhe[$NH_2$] |
| $PhCH_2-$ | $-CH_2Ph$ | 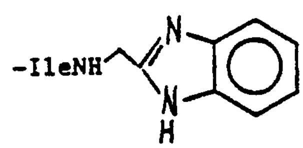 |

| $R^9{}_a$ | $R^9{}_b$ | B-B-J |
|---|---|---|
| 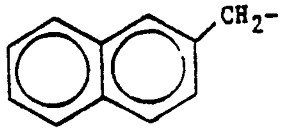 | $-CH_2Ph$ | 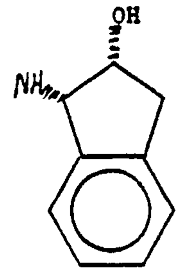 |
| $PhCH_2-$ | 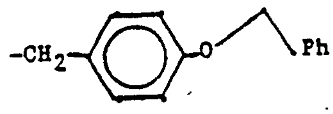 | $-LeuPhe[NH_2]$ |
| $CH_3(CH_2)_3-$ | $-CH_2Ph$ | $-LeuPhe[NH_2]$ |
| $PhCH_2-$ | $-CH_2Ph$ | 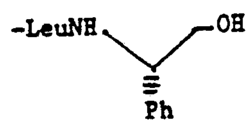 |
| $PhCH_2$ | $-CH_2Ph$ | 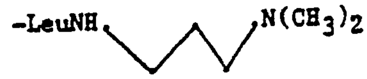 |
| $PhCH_2-$ | 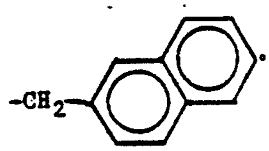 | $-LeuPhe[NH_2]$ |
| $PhCh_2-$ | $-CH_2Ph$ | 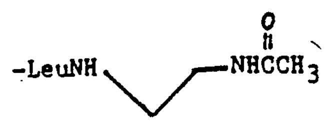 |
| $PhCH_2-$ | $-CH_2Ph$ | 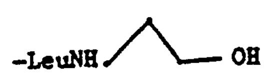 |

| $R^9_a$ | $R^9_b$ | B-B-J |
|---|---|---|
| $PhCH_2-$ | $-CH_2Ph$ | -LeuPhe |
| $PhCH_2-$ | $-CH_2Ph$ | 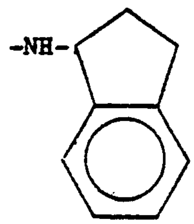 |
| 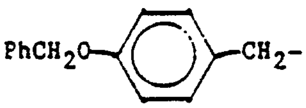 | $-CH_2Ph$ | 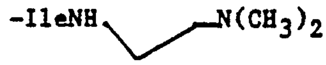 |
| $PhCH_2O-CH_2-$ | $-CH_2-Ch_2-CH_2-Ph$ | -IlePhe[$NH_2$] |
| 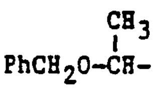 | $-CH_2Ph$ | 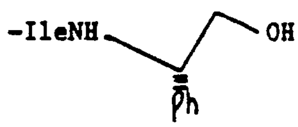 |
| $PhCH_2-$ | $-CH_2Ph$ | $-(CH_2)_2NHSO_2CH_3$ |
| $PhCH_2-$ | $-CH_2-CH=CH-Ph$ | 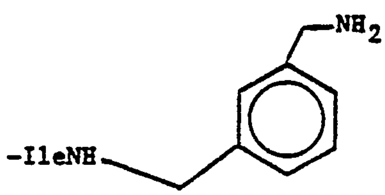 |
| $PhCH_2-$ | $-CH_2Ph$ | 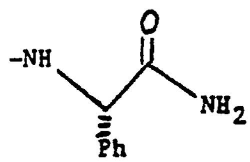 |

Additional J groups are added by Schemes IIIa and IIIb.

IIIa:

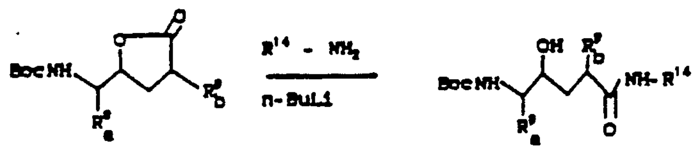


IIIb:

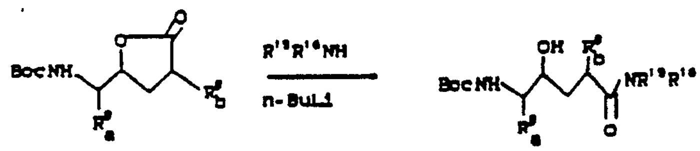


See also Example 4. Product compounds include those of the following Tables IIIa & IIIb.

## TABLE IIIa

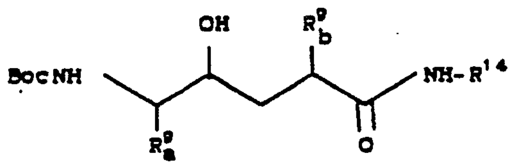

| $R_a^9$ | $R_b^9$ | $-NH-R^{14}$ and other J groups |
|---|---|---|
| $PhCH_2$ | $-CH_2Ph$ | $-NH-(CH_2)_2CH(CH_3)_2$ |
| $PhCH_2$ | $-CH_2Ph$ | 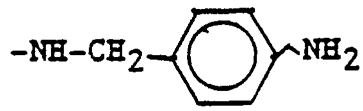 |
| $PhCH_2$ | $-CH_2Ph$ | 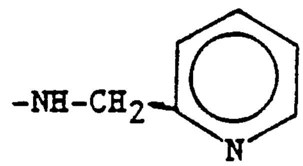 |
| $PhCH_2$ | $-CH_2Ph$ | 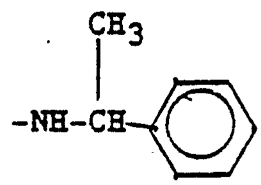 |
| $PhCH_2$ | $-CH_2Ph$ | 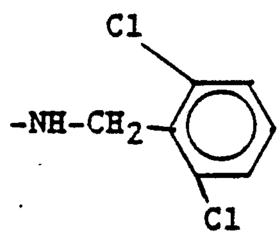 |

## TABLE IIIb

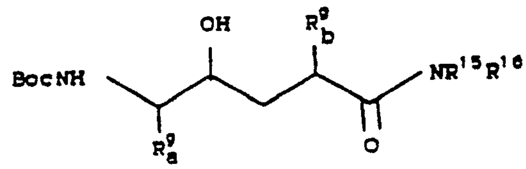

| $R_a^9$ | $R_b^9$ | $NR^{15}R^{16}$ |
|---|---|---|
| $PhCH_2$ | $-CH_2Ph$ | 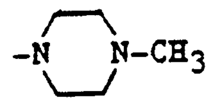 |
| $PhCH_2$ | $-CH_2Ph$ | 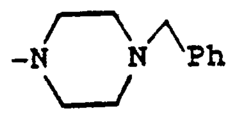 |
| $PhCH_2$ | $-CH_2Ph$ | 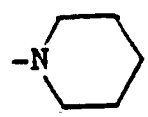 |
| $PhCH_2$ | $-CH_2Ph$ | 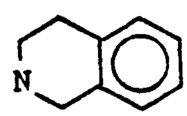, |

a benzopiperidinyl J group.

| $R_a^9$ | $R_b^9$ | $NR^{15}R^{16}$ |
|---|---|---|
| $PhCH_2$ | $-CH_2Ph$ | 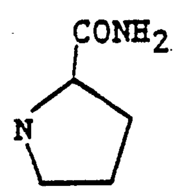 |

The compounds of the present invention are useful in the inhibition of HIV protease, the prevention or treatment of infection by the human immunodeficiency virus (HIV) and the treatment of consequent pathological conditions such as AIDS. Treating AIDS or preventing or treating infection by HIV is defined as including, but not limited to, treating a wide range of states of HIV infection: AIDS, ARC (AIDS related complex), both symptomatic and asymtomatic, and actual or potential exposure to HIV. For example, the compounds of this invention are useful in treating infection by HIV after suspected past exposure to HIV by e.g., blood transfusion, accidental needle stick, or exposure to patient blood during surgery.

In the present invention, compounds with asymmetric centers may occur as racemates, racemic mixtures and as individual diastereomers, with all isomeric forms of the compounds being included in the present invention.

For these purposes, the compounds of the present invention may be administered orally, parenterally (including subcutaneous injections, intravenous, intramuscular, intrasternal injection or infusion techniques), by inhalation spray, or rectally, in dosage unit formulations containing conventional non-toxic pharmaceutically-acceptable carriers, adjuvants and vehicles.

Thus, in accordance with the present invention there is further provided a method of treating and a pharmaceutical composition for treating HIV infection and AIDS. The treatment involves administering to a patient in need of such treatment a pharmaceutical composition comprising a pharmaceutical carrier and a therapeutically-effective amount of a compound of the present invention, or a pharmaceutically-acceptable salt thereof.

These pharmaceutical compositions may be in the form of orally-administrable suspensions or tablets; nasal sprays; sterile injectable preparations, for example, as sterile injectable aqueous or oleagenous suspensions or suppositories.

When administered orally as a suspension, these compositions are prepared according to techniques well-known in the art of pharmaceutical formulation and may contain microcrystalline cellulose for imparting bulk, alginic acid or sodium alginate as a suspending agent, methylcellulose as a viscosity enhancer, and sweetners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions are prepared according to techniques well-known in the art of pharmaceutical formulation and may be prepared as solutions in saline, employing benzyl alcohol or other suitable preservatives, absorption promoters to enhance bioavailability, flourocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquidify and/or dissolve in the rectal cavity to release the drug.

Dosage levels of the order of 0.02 to 5.0 or 10.0 grams-per-day are useful in the treatment or prevention of the above-indicated conditions, with oral doses two-to-five times higher. For example, infection by HIV is effectively treated by the administration of from 10 to 50 milligrams of the compound per kilogram of body weight from one to three times per day. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The present invention is also directed to combinations of the HIV protease-inhibitory compounds with one or more agents useful in the treatment of AIDS.

For example, the compounds of this invention can be given in combination with the antivirals, immunomodulaters, antibiotics or vaccines or other derivative forms thereof as listed in the Table IV [source: Marketletter, Nov. 30. 1987, pp. 26-27; Genetic Engineering News, Jan. 1988, Vol. 8, 23]:

TABLE IV[1]

A. Antivirals

| Drug Name | Manufacturer | Indication |
|---|---|---|
| AL-721 | Ethigen | ARC, PGL |
| BETASERON (interferon beta) | Triton Biosciences | AIDS, ARC, KS |
| CARRISYN (polymannoacetate) | Carrington Labs | ARC |
| CYTOVENE (ganciclovir) | Syntex | CMV |
| DDC (dideoxycytidine) | Hoffmann-La Roche | AIDS, ARC |

[1]Abbreviations: AIDS (Acquired Immune Deficiency Syndrome); ARC (AIDS related complex); CMV (Cytomegalovirus, which causes an opportunistic infection resulting in blindness or death in AIDS patients); HIV (Human Immunodeficiency Virus, previously known as LAV, HTLV-III or ARV); KS (Kaposi's sarcoma); PCP (Pneumonocystis carinii pneumonia, an opportunistic infection); PGL (persistent generalized lymphadenopathy).

| Drug Name | Manufacturer | Indication |
|---|---|---|
| FOSCARNET (trisodium phosphonoformate) | Astra AB | HIV inf, CMV retinitis |
| HPA-23 | Rhone-Poulenc Sante | HIV infection |
| ORNIDYL (eflornithine) | Merrell Dow | PCP |
| PEPTIDE T (octapeptide sequence) | Peninsula Labs | AIDS |
| RETICULOSE (nucleophospho-protein) | Advanced Viral Research | AIDS, ARC |
| RETROVIR advanced (zidovudine; AZT) | Burroughs Wellcome | AIDS, ARC pediatric AIDS, KS, asympt HIV, less severe HIV, neurological in-volvement. |

| Drug Name | Manufacturer | Indication |
|---|---|---|
| RIFABUTIN (ansamycin LM 427) | Adria Labs | ARC |
| (trimetrexate) | Warner-Lambert | PCP |
| UA001 | Ueno Fine Chem Industry | AIDS, ARC |
| VIRAZOLE (ribavirin) | Viratek/ICN | AIDS, ARC, KS |
| WELLFERON (alfa interferon) | Burroughs Wellcome | KS, HIV, in comb with RETROVIR |
| ZOVIRAX (acyclovir) | Burroughs Wellcome | AIDS, ARC, in comb with RETROVIR |

B. Immunomodulators

| Drug Name | Manufacturer | Indication |
|---|---|---|
| ABPP KS (bropirimine) | Upjohn | Advanced AIDS, |
| AMPLIGEN (mismatched RNA) | DuPont HEM Research | ARC, PGL |
| (Anti-human alpha interferon antibody) | Advanced Biotherapy Concepts | AIDS, ARC, KS |

| | | |
|---|---|---|
| Colony Stimulating Factor (GM-CSF) | Sandoz Genetics Institute | AIDS, ARC, HIV, KS |
| CL246,738 (CL246,738) | American Cynamid | AIDS |
| IMREG-1 | Imreg | AIDS, ARC, PGL, KS |
| IMREG-2 | Imreg | AIDS, ARC, PGL, KS |
| IMUTHIOL (diethyl dithio carbamate) | Merieux Institute | AIDS, ARC |

| Drug Name | Manufacturer | Indication |
|---|---|---|
| IL-2 (interleukin-2) | Cetus | AIDS, KS |
| IL-2 (interleukin-2) | Hoffmann-La Roche Immunex | AIDS, KS |
| INTRON-A (interferon alfa) | Schering-Plough | KS |
| ISOPRINOSINE (inosine pranobex) | Newport Pharmaceuticals | ARC, PGL, HIV seropositive patients |
| (methionine enkephalin) | TNI Pharmaceuticals | AIDS, ARC |

| | | |
|---|---|---|
| MTP-PE (muramyl-tripeptide) | Ciba-Geigy | KS |
| THYMOPENTIN (TP-5) (thymic compound) | Ortho Pharmaceuticals | HIV infection |
| ROFERON (interferon alfa) | Hoffmann-La Roche | KS |
| (recombinant erythropoietin) | Ortho Pharmaceuticals | severe anemia assoc with AIDS & RETROVIR therapy |
| TREXAN (naltrexone) | DuPont | AIDS, ARC |
| TNF (tumor necrosis factor) | Genentech | ARC, in combination interferon gamma |

C. Antibiotics

| Drug Name | Manufacturer | Indication |
|---|---|---|
| PENTAM 300 (pentamidine isethionate) | LyphoMed | PCP |

D. Vaccines

Any one of a variety of AIDS or HIV vaccines presently under study and development can be used in combination with the compounds of this invention or salt or derivative forms thereof, in the treatment or prevention of AIDS and diseases of similar character caused by HIV.

It will be understood that the scope of combinations of the compounds of this invention with AIDS antivirals, immunomodulators, antibiotics or vaccines is not limited to the list in the above Table, but includes in principle any combination with any pharmaceutical composition useful for the treatment of AIDS.

SYNTHESIS

The preparation and synthesis follows, in general, U.S. Patent 4,661,473; Evans, B.E. et al, J. Org. Chem., 50, 4615, (1985) and Evans, B.E. et al., "A Stereocontrolled Synthesis of Hydroxyethylene Dipeptide Isosteres," Proc. Am. Pept. Symp., 9, 743-6(198), and Luly, J.R. et al, J. Org. Chem. 52, 1487 (1987), all herein incorporated by reference.

## EXAMPLE 1

### Preparation of N′-(1,1-Dimethylethoxycarbonyl)-5(S)-amino-4(S)-hydroxy-6-phenyl-2(R)-(phenylmethyl)-hexanoyl-Leucyl-phenylalanyl amide, Compound I

#### Step A: Preparation of N-3(S)-[(1,1-Dimethylethoxycarbonyl)amino]-2(RS)-hydroxy-4-phenyl-1-trimethylsilyl butane:

To a stirred suspension of magnesium turnings (9.79 g, 403 mmol) in dry diethyl ether (200 mL) under nitrogen was added chloromethyltrimethylsilane (50 mL, 358 mmol). The reaction was initiated by gentle warming and then was cooled in an ice bath to maintain gentle reflux. After exotherm was complete the reaction was stirred at room temperature for 1 hour then cooled to -78° C in a dry ice/acetone bath. To the solution of the Grignard was added dropwise with stirring a solution of N-2(S)-[(1,1-dimethylethoxycarbonyl)-amino]-3-phenyl propionaldehyde (19.3 g, 77.4 mmol) in dry diethyl ether (250 mL) dropwise such that the temperature of the reaction remained below -55° C. The resultant gray suspension was allowed to warm to room temperature where it was stirred for 30 minutes then was quenched by pouring into a mixture of ice (500 g) and 10% citric acid (500 mL). The organic phase was collected and the aqueous phase was extracted with diethyl ether (3 X 300 mL). The combined organics were washed with 10% citric acid (1 X 300 mL) and brine (1 X 200 mL), dried over anhydrous magnesium sulfate, filtered, and concentrated to give crude N-3(S)-[(1,1-dimethylethoxycarbonyl)amino]-2(RS)-hydroxy-4-phenyl-1-trimethylsilyl butane (26.6 g, quantitative crude yield) as a yellow oil. An analytical sample was obtained by low pressure chromatography (silica gel, 230-400 mesh; diethyl ether: hexanes, 30%:70%) followed by recrystallization from heptane. mp = 91-95° C:

| elemental analysis. Calcd. for $C_{18}H_{31}NO_3Si$ (337.53): | | | | | | |
|---|---|---|---|---|---|---|
| | C = | 64.05, | H = | 9.26, | N = | 415; |
| Found: | C = | 64.05, | H = | 9.13, | N = | 4.22; $[\alpha]_D20$ = -40.0° |

#### Step B: Preparation of 3(S)-Amino-4-phenyl-1-butene.

To a stirred solution of the product of Step A (22.8 g, 67.5 mmoL) in dry methylene chloride (400 mL) cooled in an ice bath and under nitrogen was added in a fine stream boron trifluoride etherate (43 mL, 345 mmol). The solution was allowed to warm to room temperature where it was stirred for 4 days. Reaction was cooled in an ice bath and quenched by the dropwise addition of 10% sodium hydroxide (400 mL). The organic phase was collected and the aqueous phase was extracted with methylene chloride (2 X 250 mL). The combined organics were washed with brine (1 X 200 mL), dried over anhydrous magnesium sulfate, filtered, and concentrated to give crude 3(S)-amino-4-phenyl-1-butene (14.2 g) as a yellow oil.

#### Step C: Preparation of N-3(S)-[(1,1-Dimethylethoxycarbonyl)amino]-4-phenyl-1-butene:

A solution of the product of Step B (14.2 g) and di-tert-butyl dicarbonate (31.0 g, 142 mmoL) in dry methylene chloride (200 mL) was stirred at room temperature for 18 hours, washed with 10% citric acid (3 X 100 mL), water (1 X 100 mL), sat'd. sodium bicarbonate (3 X 125 mL), and brine (1 X 250 mL), dried over

anhydrous magnesium sulfate, filtered and concentrated to yield crude N-3(S)-1[(1,1-dimethylethoxycarbonyl)amino]-4-phenylbutene (34.6 g) as a yellow oil. Crude product was purified by low pressure chromatography (silica gel, 230-400 mesh, 10 X 20 cm column; diethylether: hexanes, 20%: 80%) to yield N-3(S)-[(1,1-dimethylethoxylcarbonyl)amino]-4-phenyl-1-butene (16.3 g, 97.6% yield) as a white solid. An analytical sample was obtained by recrystallization from heptane. mp = 67.5-68.5° C;

| elemental analysis. Calcd. for $C_{15}H_{21}NO_2$ (247.34): | | | | | | |
|---|---|---|---|---|---|---|
| | C = | 72.84, | H = | 8.56, | N = | 5.66. |
| Found: | C = | 72.78, | H = | 8.76, | N = | 5.64. |

Step D: Preparation of 1(R)-[1′(S)-(1,1-Dimethylethoxycarbonyl)amino-2-phenylethyl]oxirane:

To a solution of the product of Step C (9.4 g, 38 mmol) in dry methylene chloride (100 mL) cooled in an ice bath and under nitrogen was added 3-chloroperoxybenzoic acid (technical grade, 80-85%; 41 g, 200 mmol). The mixture was stirred at 0° C for 18 hours and 25° C for 23 hours, then diluted with diethyl ether (300 mL), and poured in ice cold aqeous 10% sodium sulfite (1 L). The organic layer was collected and the aqueous layer was extracted with diethyl ether (3 X 100 mL). The combined organics were washed with 10% sodium sulfite (3 X 100 mL), satd. sodium bicarbonate (3 X 100 mL), and brine (1 X 100 mL), dried over anhydrous sodium sulfate, filtered and concentrated to give a white solid. Crude product was purified by low pressure chromatography (silica gel 230 - 400 mesh, 8 X 15 cm column; ethyl acetate: hexanes, 25%:75%) to yield 1(R)-[1′(S)-(1,1-dimethylethoxycarbonyl)amino-2-phenylethyl]oxirane (7.0 g, 70% yield) as a clear oil which crystallized upon standing. An analytical sample was obtained by recrystallization from heptane. mp = 51.5-52° C;

| elemental analysis. Calcd. for $C_{15}H_{21}NO_2$ (263.34): | | | | | | |
|---|---|---|---|---|---|---|
| | C = | 68.42, | H = | 8.04, | N = | 5.32. |
| Found: | C = | 68.22, | H = | 8.26, | N = | 5.29; $[\alpha]_D^{20}$ = 1.34° |

Step E: Preparation of(5S,1′S)-3-carboethoxy-5-(1-((1′,1′-dimethylethoxycarbonyl)amino)-2-phenylethyl)-dihydrofuran-2-(3H)-one.

The product from Step D, 9.93 g, was dissolved in 100 mL of absolute ethanol and added to a solution of 2.6 g of sodium and 20.1 mL of diethyl malonate in 170 mL of absolute ethanol. After stirring overnite, the reaction was acidified to pH 4 with 10% citric acid and extracted with 2 X 500 mL of ether. The combined organic extracts were washed 1 X 500 mL $H_2O$, 1 X 500 mL sat'd $NaHCO_3$, 1 x 500 mL sat'd brine and dried over $MgSO_4$. The solvents were removed and the crude product purified by low pressure chromatography on silica gel eluting with 50% ether/hexanes (or EtOAc/hexanes). The yield of semi-solid product was 10.6 g. The later fractions contained 2.5 g of the undesired 5 R isomer as a white solid.

Step F: Preparation of (5S,1′S-)3-carboethoxy-3-phenylmethyl-5-(1-((1,1-dimethylethoxycarbonyl)-amino)-2-phenylethyl)dihydrofuran-2-(3H)-one.

The product of Step E, 10.6 g, was dissolved in 100 mL of abs. ethanol containing 3.7 mL of benzyl bromide and added to a solution of 0.71 g of sodium in 100 mL of absolute ethanol. The solution was heated to 50° C for 1.5 hours, then cooled in an ice bath and acidified with 500 ml of 10% citric acid. The mixture was extracted 3 X 500 mL of ether and the combined ether extracts washed with 400 mL of $H_2O$, 400 mL of brine, dried ($MgSO_4$) and the solvent removed under reduced pressure to give 13.6 g of a clear colorless oil which was essentially homogeneous by TLC (25% ethyl acetate/hexanes).

Step G: Preparation of (3R,5S,1′S)-3-benzyl-5-(1-((1,1-dimethylethoxycarbonyl)amino)-2-phenylethyl)-dihydrofuran-2-(3H)-one.

The product of Step F, 13.6 g, was dissolved in 250 mL of 1,2-dimethoxyethane, and to it was added 117 mL of 1 M lithium hydroxide at room temperature. After stirring for 12 hours, the solvents were removed under reduced pressure, the residue suspended in 200 mL of 10% citric acid and extracted 3 X 500 mL of diethyl ether. The combined ether extracts were washed with 500 mL of brine, dried ($MgSO_4$) and the concentrated to dryness. The residue was dissolved in 250 mL of toluene, heated to reflux for 12 hours, then concentrated to dryness under reduced pressure. Purification by medium pressure chromatography over silica gel eluting with 15% ethyl acetate/hexanes gave 3.2 g of the 3R-lactone as a clear foam. Further elution with the same solvents gave 6.15 g of the 3S-lactone as a white solid.

Step H: Preparation of N′-(1,1-dimethylethoxycarbonyl)-5(S)-amino-4(S)-(1′,1′-dimethylethyl-1,1-dimethylsilyloxy)-6-phenyl-2(R)-(phenylmethyl)-hexanoic acid.

(3R, 5S, 1′S)-3-Benzyl-5-(1-((1,1-dimethylethoxycarbonyl)amino)-2-phenylethyl)dihydrofuran-2-(3H)-one, 0.5 g, was dissolved in 30 mL of a 2:1 mixture of ethylene glycol dimethyl ether/water, and to it was added 5 mL of 1 M lithium hydroxide at room temperature. After stirring for 1 hour, the solvent was removed in vacuo and the residue partitioned between 20 mL chloroform and 20 mL 10% citric acid. The layers were separated and the aqueous phase extracted with 3 X 20 mL chloroform. The combined organic layers were dried ($Na_2SO_4$) and the solvent removed to yield 0.46 g of the crude hydroxy acid. This residue was dissolved in 5 mL of dry DMF and 0.845 g tert-butyl dimethylsilyl chloride and 0.725 g of imidazole were added. After stirring for 18 hours, the reaction was poured into 50 mL of water and extracted with 3 X 20 mL of ethyl acetate. The combined organic extracts were washed with 3 x 20 mL of 10% citric acid, 1 x 20 mL of water, 3 X 10 mL of saturated aqueous solution of $Na_2CO_3$, and 20 mL of brine. After drying ($Na_2SO_4$), the solvent was removed and the resulting residue dissolved in a mixture of 5 mL of THF, 5 mL of glacial acetic acid, and 2 mL of water. The mixture was stirred for 4 hours, then poured into 50 mL of water and extracted with 3 X 20 mL of ether. The combined ether extracts were washed with 2 X 20 mL of water, brine, dried ($Na_2SO_4$), and the solvent removed. Purification by medium pressure chromatography over silica gel, eluting with $MeOH/CHCl_3$ gave 0.53 g of the product as a white solid.

Step I: Preparation of N′-(1,1-dimethylethoxycarbonyl)-5(S)-amino-4(S)-(1′,1′-dimethylethyl-1,1-dimethylsilyloxy)-6-phenyl-2(R)-(phenylmethyl)-hexanoyleucyl-phenylalanyl amide.

The product from Step H, 0.183 g, was dissolved in 10 mL of dry DMF, and to it was added 0.124 g of leucinyl-phenylalanyl amide hydrochloride hemihydrate, 0.051 g of 1-hydroxybenzotriazole hydrate and 0.069 g of dimethyl-3-(3-dimethylaminopropyl) carbodiimide hydrochloride. Triethylamine was added to the stirring solution until the pH was 8.5. After stirring for 2 hours, the reaction was poured into 80 mL of water and extracted with 5 X 10 mL of ethyl acetate. The combined organic extracts were washed with 3 X 20 mL of 10% citric acid, 1 X 20 mL of water, 3 X 20 mL of a saturated aqueous solution of $Na_2CO_3$, 30 mL of brine, dried ($Na_2SO_4$), and the solvent removed to give 0.2 g of the product after purification by preparative thin layer chromatograpy (5% methanol/chloroform).

Step J: Preparation of N′-(1,1-dimethylethoxycarbonyl)-5(S)-amino-4(S)-hydroxy-6-phenyl-2(R)-(phenylmethyl)hexanoyl-leucyl-phenylalanyl amide.

The product from Step I, 0.2 g, was placed in a flask and to it was added 2 mL of a 1M solution of tetrabutylammonium fluoride in THF. After stirring for 2 hours, the solvent was removed in vacuo, and the residue was taken up in 50 mL of 10% methanol/chloroform and passed through a pad of silica gel. The solvent was removed in vacuo, and the remaining solid triturated with ethyl acetate. The solid was filtered through a silica pad and washed with ethyl acetate. The silica gel pad was then washed with chloroform (200 mL). The chloroform solution of the product was evaporated to yield 0.074 g of pure product. The ethyl acetate solution was concentrated, and the residue was chromatographed over silica gel, eluting with methanol/chloroform to give an additional 0.054 g of product. The combined yield of the product was 0.128 g: mp 218-210°C.

## EXAMPLE 2

Preparation of N-benzyl-N′-(1,1-dimethylethoxycarbonyl)-5(S)-amino-4(S)-hydroxy-6-phenyl-2(R)-(phenylmethyl)hexanoyl-Leucyl-amide, Compound II

Step A: Preparation of N-benzyl N′-(1,1-dimethylethoxycarbonyl)-5(S)-amino-4(S)-(1′,1′-dimethylethyl-1,1-dimethylsilyloxy)-6-phenyl-2(R)-(phenylmethyl)hexanoyl-leucyl amide.

N′-(1,1-Dimethylethoxycarbonyl)-5(S)-amino-4(S)-(1′,1′-dimethylethyl-1,1-dimethylsilyloxy)-6-phenyl-2-(R)-(phenylmethyl)hexanoic acid from step A in Example 1, 0.183 g, is dissolved in 10 mL of dry DMF, and to it is added 0.058 g of N-benzyl leucine amide hydrochloride, 0.051 g of 1-hydroxybenztriazole hydrate and 0.069 of 1-ethyl-3-(3-dimethylaminopropyl) carbodiimide hydrochloride. Triethylamine is added to the stirring solution until the pH is 8.5. After stirring for 2 hours, the reaction is poured into 80 mL of water and extracted with 5 X 10 mL of ethyl acetate. The combined organic extracts are washed with 3 X 20 mL of 10% citric acid, 1 X 20 mL of water, 3 X 20 mL of a saturated aqueous solution of $Na_2CO_3$, 30 mL of brine, dried ($Na_2SO_4$), and the solvent removed to give the product after purification by preparative thin layer chromatography (5% methanol/chloroform).

Step B: Preparation of N-Benzyl N′-(1,1-Dimethylethoxycarbonyl)-5(S)-amino-4(S)-hydroxy-6-phenyl-2(R)-(phenylmethyl)hexanoyl-Leucyl amide.

The product from Step A, Example 2, 0.1 g, is placed in a flask and to it is added 2 mL of a 1 M solution of tetrabutylammonium fluoride in THF. After stirring for 2 hours, the solvent is removed in vacuo, and the residue is taken up in 50 mL of 10% methanol/chloroform and passed through a pad of silica gel. The solvent is removed in vacuo, and the remaining solid triturated with ethyl acetate. The solid is filtered through a silica pad and washed with ethyl acetate. The silica gel pad is then washed with chloroform (200 mL). The chloroform solution is evaporated to yield product.

## EXAMPLE 3

Preparation of N′-(1,1-dimethylethoxycarbonyl)-5(S)-amino-4(S)-hydroxy-6-phenyl-2(R)-(4-hydroxyphenylmethyl)hexanoyl-leucyl-phenylalanyl amide

To a stirred solution of N′-(1,1-dimethylethoxycarbonyl)-5(S)-amino-4(S)-hydroxy-6-phenyl-2(R)-(4-benzyloxyphenylmethyl)hexanoyl-leucylphenylalanyl amide, 25 mg, in 10 mL of tetrahydrofuran was added a suspension of 10% palladium on carbon, 25 mg, in 10 mL of absolute methanol. The mixture was stirred under an atmosphere of hydrogen for 4 hours at room temperature, then filtered and concentrated to dryness. The residue was dissolved in 1 mL of tetrahydrofuran and 1 mL of water was added. A white solid precipitated which was collected and dried under vacuum over $P_2O_5$. The yield was 20 mg of pure product. mp 222-223 °C (C,H,N).

## EXAMPLE 4

Preparation of N-benzyl-N′-(1,1-dimethylethoxycarbonyl)-5(S)-amino-4(S)-hydroxy-6-phenyl-2(R)-(phenylmethyl)-hexanamide. Compound III

The lithium salt of benzyl amine was prepared by adding 0.5 mL of a 2.5 M solution of n-butyl lithium to

0.134 g of benzyl amine in 10 mL of dry THF at -78° C. To this was added 0.1 g of (3R, 5S, 1'S)-3-benzyl-5-(1-((1,1-dimethylethoxycarbonyl)amino)-2-phenylethyl)-dihydrofuran-2-(3H)-one (Step G of Example 1) in 5 mL of THF. After stirring for 15 minutes, the reaction was quenched by the addition of 15 mL of 10% citric acid, and the mixture was allowed to warm to room temperature. The volume was tripled by the addition of ethyl acetate. The organic layer was separated, and the aqueous phase was washed with ethyl acetate. The combined organic solutions were washed with 3 x 10 mL 10% citric acid, 1 x 20 mL of saturated $Na_2CO_3$ solution, brine, and dried ($Na_2SO_4$). The solvent was removed in vacuo to yield a white solid. The solid was recrystallized from hexane/ethyl acetate to yield 0.065 g of the product as a crystalline solid; mp 191-192° C.

## EXAMPLE 5

Preparation of N'-(1,1-dimethylethoxycarbonyl)-4(S)-amino-3(S)-hydroxy-5-cyclohexylpentanoyl-leucyl-phenylalanylamide.

N-(1,1-dimethyethoxycarbonyl)-4(S)-amino-3 (S)-hydroxy-5-cyclohexylpentanoic acid (Boc-ACHPA), (0.575 g, 1.82 mmol) was dissolved in 5.5 mL of dry DMF, under argon. To this well stirred mixture was added L-leucyl-phenylalanine amide hydrochloride semihydrate (0.74 g, 2.18 mmol), hydroxybenztriazole hydrate (0.258 g, 1.91 mmol), ethyl 3-(dimethylamino)-propylcarbodiimide hydrochloride (0.374 g, 1.91 mmol), and triethylamine (0.57 mL, 4.10 mmol). This mixture was stirred at room temperature for 1 hour. The reaction mixture was diluted with ethyl acetate (30 mL). This mixture was washed with water (3 X 15 mL), 10% aqueous citric acid (15 mL), and brine (15 mL). Drying ($Na_2SO_4$), filtration, and removal of the solvent in vacuo gave the crude coupling product. This material was chromatographed on silica gel using 6% methanol in chloroform as eluant. There was obtained 0.582 g of the title compound as a white crystalline solid. mp: 171-175° C.

## EXAMPLE 6

Preparation of N'-[3-[(1(R)-Benzyloxycarbonylamino-2-phenylethyl)phosphinyl]-2(R)-phenylmethyl propanoyl]-leucyl-phenylalanylamide, and N'-[3-[(1(R)-Benzyloxycarbonylamino-2-phenylethyl)phosphinyl]-2-(S)-phenylmethylpropanoyl]-leucyl-phenylalanylamide.

Step A: Preparation of Methyl-1(R)-benzyloxycarbonylamino-2-phenylethylphosphinate.

A 10% solution of trimethylsilyldiazomethane in pentane was added dropwise to a solution of 0.790 g of 1(R)-benzyloxycarbonylamino-2-phenylethyl phosphonous acid in a mixture of benzene (35 mL)/methanol (5 mL) until a pale yellow color persisted in the stirred reaction solution. After one hour at 0° the solution was concentrated and the residue chromatographed over silica gel, eluting with methylene chloride/acetone/methanol (18:1:1), to afford 0.400 g of pure methyl ester.

Step B: Preparation of Methyl-1(R)-benzyloxycarbonylamino-2-phenylethyl-(2(R,S)-carbomethoxy-3-phenyl-1-propyl)phosphinate.

The product of Step A, 0.397 g, was dissolved in 5 mL of absolute methanol and, at 0° was treated with 0.67 mL of a 2.0 M solution of sodium methoxide in methanol under a nitrogen atmosphere. After 10 minutes, methyl 2-benzyl acrylate (0.240 g) was added in one portion, the cooling bath removed, and the mixture was stirred at ambient termperature of 18 hours. The mixture was concentrated to an oil which was partitioned between 15 mL of 1 N hydrochloric acid and 10 mL ethyl of ethyl acetate. The layers were separated and the aqueous phase was extracted with 2 X 10 mL acetate. The combined organic layers were washed (brine) and dried ($Na_2SO_4$) and the solvent removed to give an oil which was chromatographed on silica gel (flash). Elution with ethyl acetate afforded 0.264 g of the product.

Step C: Preparation of Methyl-1(R)-benzyloxycarbonylamino-2-phenylethyl-(2(R,S)-carboxy-3-phenyl-1-propyl)phosphinate.

A solution of the product of Step B. 0.264 g, in 1.5 mL of 1,2-dimethoxyethane was treated with 0.56 mL of a 1.0 N aqueous lithium hydroxide solution. After stirring under nitrogen for 4 hours at room temperature, the solvent was removed and 30 mL ice water was added to the residue. The resulting mixture was extracted with 2 X 10 mL ether/ethyl acetate mixtures, following which the aqueous phase was acidified to pH 4-5 using several drops of 50% acetic acid. The acidic mixture was extracted with 3 X 15 mL ethyl acetate and then the combined acidic organic layers were washed with water, brine, and dried ($Na_2SO_4$). Removal of the solvent in vacuo left the product as an oil: 0.153 g.

Step D: Preparation of N-[3-[Methoxy-1(R)-benzyloxycarbonylamino-2-phenylethyl)phosphinyl]-2(R,S)-phenylmethyl propanoyl]-leucylphenyl-alanylamide.

The product from Step C, 0.153 g, was dissolved in 4 mL of dry acetonitrile and stirred under nitrogen in an ice bath. To the solution were added 0.119 g of 1-leucyl L-phenylalanylamide hydrochloride hemihydrate, 0.155 g of benzotriazol-1-yloxytris (dimethylamino)phosphonium hexafluorophosphate, and triethylamine to pH 8.5. After stirring at 0° for 2 hours the mixture was warmed to room termperature and stirred 3 hours further. 10 mL of dilute aqueous sodium chloride was added and the mixture was extracted with 4 X 10 mL of ethyl acetate. The combined organic extracts were washed with water, 2 N hydrochloric acid, water, saturated aqueous sodium bicarbonate, brine, and dried ($MgSO_4$). Removal of the solvent left 0.164 g of a sticky solid which was a mixture of 4 major components as evidenced by thin layer chromatograpy (chloroform/methanol/ammonium hydroxide, 90:10:1). The mixture was flash chromatographed, eluting with the same solvent mixture used for TLC. Early fractions containing an intimate mixture of 3 components were combined and concentrated to give 66 mg of a diastereomeric mixture of the product. Continued elution provided an additional 52 mg of product mixture which was predominantly a single TLC spot.

Step E: Preparation of N-[3-[(1(R)-Benzyloxycarbonyl-amino-2-phenylethyl)phosphinyl]-2 (S)-phenyl-methyl propanoyl]-leucylphenlalanylamide, Compound A, and N-[3-[(1(R)-Benzyloxycarbonylamino-2-phenylethyl)-phosphinyl]-2(S)-phenylmethyl propanoyl]-leucyl-phenlalanylamide, Compound B.

A solution containing 0.066 g of the diastrereomeric mixture of Step D and 0.014 g of anhydrous lithium iodide in 2 mL dry tetrahydrofuran was stirred at room temperature for 7 days, during which time an additional 0.030 g of lithium iodide was added. At the end of 1 week the solvent was removed and the residue suspended in 5 mL of saturated sodium bicarbonate. The mixture was extracted with 4 x 5 mL ethyl acetate and the combined organic layers were concentrated. Separation of the two diastereomers was accomplished by preparative high pressure liquid chromatograpy using a Waters Delta Pak C-18 column, eluting with a gradient of acetonitrile, 0-95% in water (0.1% trifluoroacetic acid) over one hour. The fractions of retention time 4.22 minutes and that of 7.00 minutes were lyophilized separately to afford 0.013 g of Compound A and 0.012 g of Compound B, respectively.

## EXAMPLE 7

Preparation of N′-(Benzyloxycarbonyl)-5(S)-amino-4(S)-hydroxy-6-phenyl-2(R)-(phenylmethyl)-hexanoyl-leucyl-phenylalanyl amide.

Step A: Preparation of 5(S)-amino-4(S)-(t-butyldimethylsilyloxy)-6-phenyl-2(R)-(phenyl) methyl)-hexanoyl-Leucyl-PhenyalanylAmide.

To a cold (0° C) solution of 350 mgs. (0.468 mmol) of N′-(1,1-dimethylethoxycarbonyl)-5(S)-amino-4(S)-(t-butyl-dimethylsilyloxy)-6-phenyl-2(R) (phenylmethyl)-hexanoyl-leucyl-Phenylalanyl Amide in 10 mLs of methylene chloride was added 5 mLs of trifluoroacetic acid. After 1 hour., the reaction was poured into a separatory funnel containing sat'd. $NaHCO_3$ solution and washed with methylene chloride (2 X 30 mLs). The organics were dried, filtered and concentrated to give an oily residue which was flashed chromatographed ($SiO_2$, 5% $MeOH/NH_3$ sat'd. chloroform) to give 240 mgs. of product along with 75 mgs of 5(S)-amino-4(S)-hydroxy-6-phenyl-2(R)-(phenylmethyl)-hexanoyl-Leucyl-Phenylalanyl Amide.

Step B: Preparation of 5(S)-(Benzyloxycarbonyl)-amino-4(S)-hydroxy-6-phenyl-2(R)-(phenylmethyl)-hexanoyl-Leucyl-Phenyalanyl Amide.

To a solution of 25 mgs. (0.038 mmol) of 5(S)-amino-4(S)-(t-Butyl-dimethylsilyloxy)-6-phenyl-2(R)-(phenylmethyl)-hexanoyl-Leucyl-Phenylalanyl Amide in 10 mLs of methylene chloride which contained 10 ul (0.072 mmol of triethylamine was added 10 ul (0.070 mmol) of benzyl chloroformate. After 3 hours the reaction was complete and was poured into a separatory funnel and washed with 2 X 10 mLs of 10% citric acid solution, 10 mLs of said $NaHCO_3$ soltuion, and the organics were dried filtered and concentrated to dryness. The residue was diluted with 2 mLs of THF and 1 mL of a 1 M solution of tetrabutylammonium fluoride (TBAF) was added. After 1 hour an additional 0.5 mL of TBAF was added. After 2 hours the reaction was concentrated and passed through a pad of $SiO_2$ which was eluted with 5% $MeOH/CHCl_3$ to give the crude product. This was further purified using reverse phase HPLC on a Waters C-18 column to give 12 mgs. of the desired product mp = 207-210° C.

## EXAMPLE 8

Assay for Inhibition of Synthetic Viral Protease

Inhibition studies of the reaction of the synthetic protease [amino acid residues 69-167 of the pol open reading frame in Ratner, L. et al, Nature, 313, 277 (1985) and synthesized by Merrifield solid-phase synthesis] with a peptide substrate [Val-Ser-Gln-Asn-Tyr-Pro-Ile-Val, 2 mg/mL when the reaction is initiated) were in 50 mM Na acetate, pH 5.5, at 30° C for 1 hour. Various concentrations of inhibitor in 1.0 µl DMSO were added to 36 µl of assay solution and the reaction initiated by the addition of 4 ul (1.6 µg) of synthetic protease. The reaction was quenched with 160 ul of 12% acetic acid. Products of the reaction were separated by HPLC (VYDAC wide pore 5 cm C-18 reverse phase, acetonitrile gradient, 0.1% trifluoroacetic acid). The extent of inhibition of the reaction was determined from the peak heights of the products. HPLC of the products, independently synthesized, provided quantitation standards and confirmation of the product composition. Compounds I, III, and IV showed an $IC_{50}$ of about 2 nM, about 50 nM and about 27 nM, respectively.

## EXAMPLE 9

Assay for Inhibition of Microbial Expressed Viral Protease

Inhibition studies of the reaction of the protease expressed in Eschericia coli with a peptide substrate [Val-Ser-Gln-Asn-(betanapthyl)Ala-Pro-Ile-Val, 0.5 mg/mL at the time the reaction is initiated] were in 50 mM Na acetate, pH 5.5, at 30° for 1 hour. Various concentrations of inhibitor in 1.0 ul DMSO were added to 25 ul of the peptide solution in water. The reaction is initiated by the addition of 15 ul of 0.33 nM protease (0.11 ng) in a solution of 0.133 M Na acetate pH 5.5 and 0.267% bovine serum albumin. The reaction was quenched with 160 ul of 5% phosphoric acid. Products of the reaction were separated by HPLC (VYDAC wide pore 5 cm C-18 reverse phase, acetonitrile gradient, 0.1% phosphoric acid). The extent of inhibition of the reaction was determined from the peak heights of the products. HPLC of the products, independently

synthesized, proved quantitation standards and confirmation of the product composition. Compounds I, II and III showed $IC_{50}$ values of about 0.6 nM, about 1.4 nM and about 120 nM, respectively.

## EXAMPLE 10

Preparation of N-((1R,2S,3S)-1,2-Dihydroxy-3-indanyl)-N′-(1,1-dimethylethoxycarbonyl)-5(S)-amino-4(S)-hydroxy-6-phenyl-2(R)-(phenylmethyl)hexanamide

Step A: (1RS,2RS,3RS)3-Amino-1-bromo-2-hydroxyindan cyclic carbamate

A mixture of 2.5 g (14.3 mmol) of the known 1-amino-2-hydroxyindan cyclic carbamate, 2.9 g (16.3 mmol) of N-bromosuccinimide, and 100 mL of $CCl_4$ was heated at reflux for 3 hours. After cooling, the mixture was diluted with 100 mL of $CHCl_3$ and washed with 10% $Na_2SO_3$. The aqueous layer was extracted with $CHCl_3$, and the combined organic layers were washed with 10% $Na_2SO_3$, dried over $Na_2SO_4$, and the solvents removed at reduced pressure to give an oil which was chromatographed on 250 g of fine $SiO_2$ using 1:1 EtOAc-hexanes to afford 950 mg (26%) of the Step A product as a colorless solid.

Step B: (1RS,2SR,3SR)1-Acetoxy-3-amino-2-hydroxyindan cyclic carbamate

A solution of 950 mg (3.7 mmol) of 1-Bromo-2-hydroxy-3-aminoindan cyclic carbamate (Step A product) and 1.9 g (6.3 mmol) of tetra-n-butylammonium acetate in 50 mL of DMF was heated at 80 °C under $N_2$ for 6 hours. The solvents were removed at reduced pressure and the residue was partitioned between $CHCl_3$ and water. The aqueous layer was extracted twice with $CHCl_3$ and the combined organic layers were washed with water, dried over $Na_2SO_4$ and the solvents removed to give an oil which was chromatographed on 125 g fine $SiO_2$ using EtOAc-hexanes to afford 372 mg (43%) of Step B product as a colorless solid: mp 131-133 °C,

| Anal; Calc'd for: $C_{12}H_{11}NO_4$ | | | | | | |
|---|---|---|---|---|---|---|
| | C, | 61.80; | H, | 4.75; | N, | 6.01. |
| Found: | C, | 62.12; | H, | 4.73; | N, | 5.96. |

Step C: (1RS,2SR,3SR)3-Amino-1,2-dihydroxyindan

To a stirred solution of 180 mg (0.77 mmol) of 1-acetoxy-2-hydroxy-3-aminoindan cyclic carbamate (Step B product) in 12 mL of EtOH was added a solution of 705 mg (17.6 mmol) of NaOH in 5 mL of $H_2O$. This mixture was heated at reflux for 3 hours, the solvents removed at reduced pressure, and the residue partitioned between brine and $CHCl_3$, and the combined organic layers were dried over $Na_2SO_4$. The solvents were removed to give 60 mg (47%) of Step C product as a solid.

Step D: (1R,2S,3S)-N-1,2-dihydroxy-3-indanyl)-L-phenylalanine amide

To a stirred solution of 422 mg (2.55 mmol) of 1,2-dihydroxy-3-aminoindan (Step C product), 746 mg (2.81 mmol) of Boc-L-Phenylalanine, 539 mg (2.81 mmol) of EDC, and 380 mg (2.81 mmol) of 1-hydroxybenzotriazole hydrate in 10 mL of DMF under $N_2$ was added 783 µL (5.62 mmol) of $Et_3N$. After stirring overnight at room temperature, the solvents were removed at reduced pressure, and the residue was partitioned between $CHCl_3$ and 1.0 M citric acid. The aqueous layer was extracted with $CHCl_3$ and the combined organic layers were washed with two portions of $Na_2CO_3$ and dried over $Na_2SO_4$. The solvents were removed at reduced pressure and the residue was dissolved in 14 mL of $CH_2Cl_2$ and treated with 7 mL of TFA under $N_2$ for 0.5 hours. The solvents were removed at reduced pressure, and the residue was

partitioned between 2% HCl and ether. The aqueous layer was basified with 40% NaOH, extracted with three portions of $CHCl_3$, dried over $Na_2SO_4$ and chromatographed on 23 g fine $SiO_2$ using 223 mg (56%) of Step D product as a colorless solid.

Step E: (1R,2S,3S)-3-Amino-1,2-dihydroxyindan

To a solution of 215 mg (0.69 mmol) of N-(1,2-dihydroxy-3-indanyl) L-phenylalanine amide (Step D product) in 10 mL of EtOH was added a solution of 84 mg (2.1 mmol)of NaOH in 4 mL of $H_2O$. The mixture was heated at reflux for 6 hours, the solvents removed at reduced pressure, and the residue triturated with 8 mL of $CHCl_3$. The triturate was applied to a column of 4 g of fine $SiO_2$ using 88:12:1.2 $CHCl_3$-$CH_3OH$-$NH_4OH$ at the eluent to give 104 mg (91%) of Step E product as a colorless solid.

Step F: N-((1R,2S,3S)-1,2-Dihydroxy-3-indanyl)-5(S)-(1,1-dimethylethoxycarbonylamino)-4(S)-hydroxy-6-phenyl-2(R)-(phenylmethyl)-hexanamide

To a stirred solution of 58 mg (0.11 mmol) of 5-(1,1-dimethylethoxycarbonylamino)-4-t-butyl-dimethylsilyloxy-2-(phenylmethyl)-6-phenyl hexanoic acid of Example 1, 21 mg (0.13 mmol) of (-)1,2-dihydroxy-3-aminoindan from Step E, 24 mg (0.13 mmol) of EDC, and 17 mg (0.13 mmol) of HOBT in 2 mL of DMF under $N_2$ was added 36 mL (0.25 mmol) of $Et_3N$. After stirring for 17 hours at room temperature, the solution was poured into cold 1.0 M citric acid and extracted with two portions of EtOAc. The two portions of 10% $Na_2CO_3$, brine, dried over $Na_2SO_4$ and the solvents removed to give a residue which was dissolved in 2.0 mL of a 1.0 M solution of tetra-n-butylammonium fluoride in THF. After stirring for 18 hours at room temperature, the solvents were removed at reduced pressure, and the residue was partitioned between $CHCl_3$ and $H_2O$. The aqueous layer was extracted with 1:9 $CH_3OH$-$CHCl_3$ and the combined organic layers were washed with $H_2O$, dried over $Na_2SO_4$, and the solvents removed to give a solid which was triturated with 1:4 $Et_2O$-hexanes to afford 32 mg (52%) of the title compound as a colorless solid: mp 190-192° C (dec)

| Anal. Calc'd for $C_{33}H_{40}N_2O_6$ | | | | | | |
|---|---|---|---|---|---|---|
| | C, | 70.69; | H, | 7.19; | N, | 5.00 |
| Found: | C, | 70.50; | H, | 7.18; | N, | 4.76 |

EXAMPLE 11

Preparation of N-(2(R)-hydroxy-1(S)-indanyl)-N′S-(1,1-dimethylethoxycarbonyl)-5(S)-amino)-4(S)-hydroxy-6-phenyl-2(R)-(4-hydroxyphenylmethyl) hexanamide

Step A: Preparation of (5S,1′S)-3-carboethoxy-3-(4-benzyloxyphenylmethyl)-5-[1-(1,1-dimethylethoxycarbonyl)amino)-2-phenylethyl]dihydrofuran-2-(3H)-one

To a stirred solution of (5S,1′S)-3-carboethoxy-5-[1-((1′,1′-dimethylethoxycarbonyl)amino)-7-phenylethyl)-dihydrofuran-2-(3H)-one (product of Example 1, Step E), 2 g (5.3 mmol) in 25 mL of absolute ethanol was added a solution of 0.13 g of sodium in 2.2 mL of absolute ethanol followed by 1.30 g (5.5 mmol) of 4-benzyloxybenzyl chloride. The solution was heated to 50° C under nitrogen for 1 hour, then cooled in an ice bath and acidified with 20 mL of 10% citric acid and diluted with 200 mL of water. The mixture was extracted with 3 X 100 mL of ether and the combined ether extracts washed with 50 mL of water, 200 mL of sat'd $NaHCO_3$ and dried over $MgSO_4$. Removal of solvents under reduced pressure and purification by low pressure chromatograpy on silica gel, eluting with 40% ether in hexanes gave 1.56 g (51% yield) of a clear colorless gloss essentially homogeneous by TLC (50% ether/hexanes).

Step B thru Step C: Preparation of N′-(1,1-dimethylethoxycarbonyl)-5(S)-amino-4(S)-(1′,1′-dimethylethyl 1,1-dimethylsilyloxy)-6-phenyl-2(R)-(4-benzyloxyphenylmethyl)-hexanoic acid

Employing the procedure substantially as described in Example 1, Steps G and H, but substituting for the (5S,1′S)-3-carboethoxy-3-phenyl-methyl-5-(1-(1,1-dimethylethoxycarbonyl)amino)-2-phenylethyl)-dihydrofuran-2-(3H)-one the product of Step A, 1.56 g, there was produced in sequence the following:

(Step B)

(3R,5S,1′S)-3-(4-benzyloxyphenylmethyl)-5-(1-((1,1-dimethylethoxycarbonyl)amino)-2-phenyl-ethyl-dihydrofuran-2-(3H)-one, 0.52 g (38% yield) of 3R-lactone as a colorless foam. Further elution gave 0.61 g of a mixture of 3S and 3R lactones.

(Step C)

N′-(1,1-dimethylethoxycarbonyl)-5(S)-amino-4(S)-(1′,1′-dimethylethyl-1,1-dimethylsilyloxy)-6-phenyl-2-(R)-(4-benzyloxyphenylmethyl) hexanoic acid, 0.60 g (94% yield) as a colorless foam essentially homogeneous by TLC (3% methanol/chloroform).

Step D: Preparation of N-(2(R)-hydroxy-1(S)-indanyl)-5(S)-(1,1-dimethylethoxycarbonylamino)-4(S)-hydroxy-6-phenyl-2(R)-(4-benzyloxyphenylmethyl) hexanamide

The product from Step C, 0.12 g, was dissolved in 2 ml dry DMF and to it was added 40 mg of 1(S)-amino-2(R)-hydroxyindane, 25 mg of 1-hydroxybenzotriazole hydrate and 70 mg of dimethyl-3-(3-dimethyl aminopropyl)carbodiimide hydrochloride. Triethylamine was added to the stirred solution until the pH was 8.5 (32 mL). After stirring for concentrated to dryness under reduced pressure, the residue was dissolved in 100 mL of chloroform and worked with 1 X 50 mL of 10% citric acid, 1 X 50 mL $H_2O$, 1 X 50 mL sat'd $NaHCO_3$, dried over $MgSO_4$ and concentrated to dryness. The residue was dissolved in 1 mL of tetrahydrofuran and added to 2 mL of 1 M tetrabutylammonium fluoride in THF. After stirring overnight at room temperature the reaction mixture was diluted with 10 mL of 10% citric acid and the white precipitate collected by filtration. The product was purified by low pressure chromatograpy on silica gel eluting with 2% methanol/$CH_2Cl_2$ to give 85 mg of product which was essentially homogeneous by TLC (3% methanol/$CH_2Cl_2$).

Step E: Preparation of N-(2(R)-hydroxy-1(S)-indanyl)-5(S)-(1,1-dimethylethoxycarbonylamino)-4(S)-hydroxy-6-phenyl-2(R)-(4-hydroxyphenylmethyl-hexanamide

The product of Step D, 85 mg was dissolved in 10 mL of methanol and 10 mL of THF, and to it was added 0.10 g of 10% palladium on carbon. The mixture was stirred under an atmosphere of hydrogen for 48 hours at room temperature, then filtered and concentrated to dryness. The residue was dissolved in 10 mL of hot ethanol and 20 mL water was added. On cooling the white solid precipitate was collected and dried under vacuum over $P_2O_5$. The yield was 72 mg (98% yield) of pure product: mp 218-219 °C (effervesces, sinters at 215)

| elemental analysis, Calc'd for $C_{33}H_{40}N_2O_6$: (560.696): | | | | | | |
|---|---|---|---|---|---|---|
| | C, | 70.69; | H, | 7.19; | N, | 5.00; |
| Found: | C, | 70.62; | H, | 7.39; | N, | 4.79. |

Step F: Resolution of 1-Amino-2-hydroxyindan

From the known racemic 1-amino-2-hydroxyindan, the resolution was carried out as described for the 3-amino-1,2-dihydroxyindan above. The (1S,2R)-1-amino-2-hydroxyindan resulting from saponification of the higher $R_f$ diastereomer was shown to have an $a_D$ of -58° (c = 1.0, $CHCl_3$). The (1R,2S)-1-amino-2-hydroxyindan resulting from saponification of the lower $R_f$ diastereomer was found to have an $a_D$ of + 62° (c = 1.0, $CHCl_3$).

## EXAMPLE 12

### Preparation of N-(2(R)-hydroxy-1(S)-indanyl)-5(S)-[(1,1-dimethylethoxycarbonyl)amino]-4(S)-hydroxy-6-(4-hydroxyphenyl)-2(R)-phenylmethylhexanamide

### Step A: Preparation of N-methoxy-N-methyl-N′-2(S)-[(1,1-dimethylethoxycarbonyl)amino]-3-(4-benzyloxyphenyl)propionamide

To a stirred solution of N,O-dimethylhydroxylamine hydrochloride (15.8 g, 162 mol) in 120 mL dry methylene chloride cooled to 0° C in an ice/water bath and under nitrogen was added N-methylpiperidine (19.7 mL, 162 mmol) such that the temperature of the reaction remained below 2° C. The clear solution was stored at 0° C until needed.

To a stirred solution of N-Boc-O-benzyl-L-tyrosine (50.0 g, 135 mmol) in 800 mL dry methylene chloride and 200 mL dry tetrahydrofuran cooled to -20° C in a dry ice/isopropanol/water bath and under nitrogen was added rapidly N-methylpiperidine (19.7 mL, 162 mmol). The solution was allowed to warm to -12° C and isobutyl chloroformate (21.1 mL, 162 mmol) was added rapidly such that the temperature of the reaction remained between -12° C and -8° C. After addition was complete the reaction was stirred at 0° C for 30 minutes then the previously prepared solution of N,O-dimethylhydroxylamine hydrochloride and N-methylpiperidine in methylene chloride was added in one portion. The reaction was allowed to warm to 25° C where it was stirred for 15 hours. After cooling to 0° C in an ice/water bath the reaction was washed with 10% citric acid (2 X 200 mL) and brine (1 X 200 mL), dried over anhydrous magnesium sulfate, filtered, and concentrated to give 60.2 g tan solid. Crude product was dissolved in ethyl acetate and filtered through a pad of silica gel (230-400 mesh, 400 g in a 2 l sintered glass funnel). The pad was rinsed with 3 l ethyl acetate and the filtrate was concentrated to give Step A product,N-methoxy-N-methyl-N′-2(S)-[(1,1-dimethylethoxycarbonyl)amino]-3-(4-benzyloxyphenyl)propionamide (51.8 g, 92.8% yield), as a white fluffy solid, mp = 107-108° C;

| elemental analysis, Calcd for $C_{23}H_{30}N_2O_5$ (414.50): | | | | | | |
|---|---|---|---|---|---|---|
| | C, | 66.65; | H, | 7.30; | N, | 6.76; |
| Found: | C, | 66.68; | H, | 7.13; | N, | 6.64; $[a]_D^{25}$ = 5.6° (c = 1.7, methanol). |

### Step B: Preparation of N-2(S)-[(1,1-dimethylethoxycarbonyl)amino]-3-(4-benzyloxyphenyl)propionaldehyde

To a suspension of lithium aluminum hydride (2.22 g, 58.0 mmol) in 200 mL dry diethyl ether cooled to -40° C in a dry ice/isopropanol bath and under nitrogen was added dropwise a solution of Step A product, N-methoxy-N-methyl-N′-2(S)-[(1,1-dimethylethoxycarbonyl)amino]-3-(4-benzyloxyphenyl)pro pionamide (20.5 g, 49.5 mmol), such that the temperature of the reaction remained between -36° C and -38° C. After addition was complete the reaction was warmed to 7° C, then cooled to -35° C and quenched by the addition of 40 mL 2.75 M potassium bisulfate. After warming to 25° C and stirring for 1 hour the mixture was filtered through a pad of Celite* with diethyl ether rinse. The filtrate was washed with 10% citric acid (3 X 100 mL), water (1 X 100 mL), diluted $NaHCO_3$ (2 X 100 mL), and brine (1 X 100 mL), dried over anhydrous magnesium sulfate, filtered, and concentrated to give Step B product,N-2(S)-[(1,1-dimethylethoxycarbonyl)-amino]-3-(4-benzyloxyphenyl)pro pionaldehyde (17.76 g, quantitative yield), as a white solid, mp = 98-99° C, $[a]_D^{25}$ = 27.4° (c = 1.6, methanol).

Step C Thru Step J: Preparation of N′-(1,1-dimethylethoxycarbonyl)-5(S)-amino-4(S)-(1′,1′-dimethylethyl-1,1-dimethylsilyloxy)-6-(4-benzyloxyphenyl)-2(R)-(phenylmethyl)-hexanoic acid

Employing the procedure substantially as described in Example 1, Steps A through J but substituting for the N-2(S)-[(1,1-dimethylethoxycarbonyl)amino]-3-phenyl propionaldehyde the product of Step B, 13.4 g, there was produced in sequence, the following:

(Step C Thru Step E)

N-3(S)-[(1,1-Dimethylethoxycarbonyl)amino]-4-(4-benzyloxypenyl)-1-butene (9.9 g, 74% yield) mp 87-8° C,

| elemental analysis, Calcd. for $C_{22}H_{27}NO_3$ (353467): | | | | | | |
|---|---|---|---|---|---|---|
| | C, | 74.76; | H, | 7.70; | N, | 3.96. |
| Found: | C, | 74.85 | H, | 7.69; | N, | 3.93. $[a]_D^{25}$ = t6.23° (c = 1, methanol). |

(Step F)

1(R)-[1′(S)-(1′,1′-dimethylethoxycarbonylamino-2-(4-benzyloxyphenyl)ethyl]oxirane as an oil (77% yield) homogeneous by thin layer chromatography (silica gel, 25% ethyl acetate/hexanes).

(Step G)

(5S,1′S)-3-carboethoxy-5-(1-((1′,1′-dimethylethoxycarbonyl)amino)-2-(4-benzyloxyphenyl)-ethyl)-dihydrofuran-2-(3H)-one (79% yield) as a white solid, mp 117-119° C

| analysis, Calc'd for $C_{27}H_{33}NO_7$ (483.567): | | | | | | |
|---|---|---|---|---|---|---|
| | C, | 67.06; | H, | 6.88; | N, | 2.90. |
| Found: | C, | 66.94; | H, | 6.96; | N, | 2.91. $[a]_D^{25}$ = -11.27°, (c = 1.81, methanol). |

(Step H)

(5S,1′S)-3-carboethoxy-3-phenylmethyl-5-[1-((1,1-dimethylethoxycarbonyl)amino)-2-(4-benzyloxymethyl)ethyl]dihydrofuran-(3H)-one, (97% yield) as a clear glass which was essentially homogeneous by TLC (50% diethylether/hexanes).

Step I

(3R,5S,1′S)-3-benzyl-5-[1-((1,1-dimethylethoxycarbonyl)amino)-2-(4-benzyloxyphenyl)ethyl]-dihydrofuran-2-(3H)-one, (38% yield) as a clear glass which was homogeneous by TLC (50% diethyl ether/hexane). The latter fractions of the chromatograpy gave 40% of a mixture of the 3S and 3R lactones.

(Step J)

N′-(1,1-dimethylethoxycarbonyl)-5(S)-amino-4(S)-(1′,1′-dimethylethyl-1,1-dimethylsilyloxy)-6-(4-benzyloxyphenyl)-2(R)-(phenylmethyl)-hexanoic acid, 94% yield, as a colorless foam.

Step K Thru Step L: Preparation of N-(2(R)-hydroxy-1(S)-indanyl)-5(S)-[(1,1-dimethylethoxycarbonyl)amino]-4(S)-hydroxy-6-(4-hydroxyphenyl)-2(R)-(phenylmethyl) hexanamide

Employing the procedure substantially as described in Examples I, Steps D through E, but substituting for the N′-(1,1-dimethylethoxycarbonyl)-5(S)-amino-4(S)-(1′,1′-dimethylethyl-1,1-dimethylsilyloxy)-6-phenyl-2(R)-(4-benzyloxyphenylmethyl)hexanoic acid the product of Step J, 0.350 g, there was produced in sequence, the following:

(Step K)

N-(2(R)-hydroxy-1(S)-indanyl)-5(S)-[1,1-dimethylethoxycarbonyl)amino]-4(S)-hydroxy-6-(4-benzyloxyphenyl)-2(R)-(phenylmethyl) hexanamide, 343 mg (96% yield), as a white solid which was essentially homogeneous by TLC (3% methanol/$CH_2Cl_2$).

(Step L)

N-(2(R)-hydroxy-1(S)-indanyl)-5(S)-(1,1-dimethylethoxycarbonylamino)-4(S)-hydroxy-6-(4-hydroxyphenyl)-2(R)-(phenylmethyl) hexanamide, 280 mg (92% yield) of pure product: mp 210-211 °C (effervescent)

| elemental analysis, Calc'd for $C_{33}H_{40}N_2O_6$ (560.696): | | | | | | |
|---|---|---|---|---|---|---|
| | C, | 70.69; | H, | 7.19; | N, | 5.00. |
| Found: | C, | 70.62; | H, | 7.39; | N, | 4.79. |

## EXAMPLE 13

Preparation of N-(2(R)-hydroxy-1(S)-indanyl)-5(S)-(1,1-dimethylethoxycarbonylamino)-4(S)-hydroxy-6-(4-hydroxyphenyl)-2(R)-(4-hydroxyphenylmethyl)hexanamide

Employing the procedure substantially as described in Example 12, Steps H through L, but substituting for the (5S,1S′)-3-carboethoxy-5[1-((1′,1′-dimethylethoxycarbonyl)amino)-2-phenylethyldihydrofuran-2-(3H)-one the product of Example 12, Step G, 1.80 g, there was obtained in sequence the following (Step A through E).

(Step A)

(5S,1S′)-3-carboethoxy-3-(4-benzyloxyphenylmethyl)-5-[1-((1,1-dimethylethoxycarbonyl)-amino)-2-(4-benzyloxyphenyl)ethyl]-dihydrofuran-2-(3H)-one, 1.17 g (67% yield) as a clear resin essentially homogeneous by TLC (50% ether/hexanes).

(Step B)

(3R,5S,1′S)-3-(4-benzyloxyphenylmethyl)-5-[1-((1,1-dimethylethoxycarbonyl)amino)-2-(4-benzyloxyphenyl)ethyl]-dihydrofuran-2-(3H)-one, 0.65 g (65% yield) as a clear resin which was essentially homogeneous by TLC (25% ethyl acetate/hexanes). The latter fractions of the chromatography gave a 30% yield of the 3S lactone.

(Step C)

N′-(1,1-dimethylethoxycarbonyl)-5(S)-amino-4(S)-(1′,1′-dimethylethyl-1,1-dimethylsilyloxy)-6-(4-benzyloxyphenyl)-2(R)-(4-benzyloxyphenylmethyl)-hexanoic acid, 0.80 g (quantitative yield) as a colorless foam, essentially homogeneous by TLC (5% methanol/$CHCl_3$).

(Step D)

N-(2(R)-hydroxy-1(S)-indanyl-5(S)-[1,1-dimethylethoxycarbonyl)amino]-4(S)-hydroxy-6-(4-benzyloxyphenyl)-2(R)-(4-benzyloxyphenylmethyl) hexanamide, 256 mg (95% yield) as a white solid, essentially homogeneous by TLC (2% methanol/$CH_2Cl_2$).

(Step E)

N-(2(R)-hydroxy-1(S)-indanyl)-5(S)-(1,1-dimethylethoxycarbonylamino)-4(S)-hydroxy-6-(4-hydroxyphenyl)-2(R)-(4-hydroxyphenylmethyl) hexanamide hydrate, 196 mg (quantitative yield) as a white solid: mp 203-4° C (effervescents sinters at 185° C),

| elemental analysis, calc'd for $C_{33}H_{40}N_2O_7 \cdot 0.75\ H_2O$ (590.207): | | | | | | |
|---|---|---|---|---|---|---|
| | C, | 67.15; | H, | 6.91; | N, | 4.74. |
| Found: | C, | 66.96; | H, | 6.68; | N, | 4.64. |

## EXAMPLE 14

Preparation of N′-(1,1-dimethylethoxycarbonyl)-5(S)-amino-3(S),4(R)-dihydroxy-2(R)-(phenylmethyl)-6-phenyl-hexanoylleucyl-phenylalanyl amide

Step A: Preparation of 4(S)-[(1,1-dimethylethoxycarbonyl)amino]-3(S)-hydroxy-5-phenyl-1-pentene

To a stirred solution of 80 mL of commercial 1 M zinc chloride in ether and 200 mL of dry tetrahydrofuran cooled to 0° C was added dropwise 120 mL of a commercial 1 M solution of vinylmagnesium bromide in tetrahydrofuran. The mixture was cooled to -30° C and a solution of 10.0 g, 40.1 mmol) of N-2(S)-[1,1-dimethylethoxycarbonyl)amino]-3-phenyl propionaldehyde in 200 mL of dry tetrahydrofuran was added slowly over 15 minutes, keeping the internal temperature at -30 ± 5° C. The reaction was allowed to warm to room temperature and stir for 90 minutes, then quenched by addition at 500 mL of 10% aqueous citric acid and extracted into 1 L of diethyl ether. The ether extracts were dried over anhydrous magnesium sulfate, filtered and concentrated to give a white solid. Crude product was purified by low pressure chromatography (silica gel 230-400 mesh, 8 X 18 cm column; ethyl acetate:hexanes; 15%:85%) to yield Step A product, 4(S)-[(1,1-dimethylethoxycarbonyl)amino]-3(S)-hydroxy-5-phenyl-1-pentene (6.7 g, 60% yield), as a white crystalline solid. Further elution gave 1.0 g of the 4(S)-hydroxy isomer.

Step B: Preparation of 4(S)-[(1,1-dimethylethoxycarbonyl)amino]-3(S)-(benzyloxymethyl)oxy-5-phenyl-1-pentene

The product from Step A, 1.62 g (5.8 mmol) was dissolved in 50 mL of methylene chloride and to it was added 5.1 mL of N,N-diisopropylethylamine and 5.1 mL of technical grade benzylchloromethyl ether. After stirring for 10 days at room temperature, the mixture was diluted with 150 mL of methylene chloride and washed with 100 mL of 10% citric acid, dried over $MgSO_4$ and concentrated to an oil. Crude product was purified by low pressure chromatography (silica gel 230-400 mesh, 8 X 18 cm column; diethyl ether:hexanes; 15%:85%) to yield of Step B product, 4(S)-[(1,1-dimethylethoxycarbonyl)amino]-3(S)-(benzyloxymethyl)-oxy-5-phenyl-1-pentene (2.0 g, 87%), as a white crystalline solid. mp 56-8 °C;

| elemental analysis, Calc'd for $C_{22}H_{31}NO_4$ (397.518): | | | | | | | |
|---|---|---|---|---|---|---|---|
| | C, | 72.52; | H, | 7.86; | N, | 3.52. |
| Found: | C, | 72.36; | H, | 7.87; | N, | 3.41. $[a]_D^{25}$ = ± 16.25 (C = 1.7, methanol). |

Step C: Preparation of 3(S)-[(1,1-dimethylethoxycarbonyl)amino]-2(S)-(benzyloxymethyl)oxy-4-phenylpropionaldehyde

The product from Step B, 0.60 g, was dissolved in 12 mL of methylene chloride and 6 mL of methanol containing 0.125 g of sodium bicarbonate as a suspension. The mixture was cooled to -78 °C and a stream of ozone was bubbled in until the blue color persisted (ca. 15 min). The mixture was purged with nitrogen and 1 mL of dimethyl sulfide was added. After warming to room temperature 100 mg of zinc dust and 0.18 mL of glacial acetic acid were added. After 1 hour stirring, the mixture was filtered, diluted with 50 mL of methylene chloride, washed with 1 X 20 mL sat'd $NaHCO_3$, dried over $MgSO_4$ and concentrated to yield crude Step C product, 3(S)-[(1,1-dimethylethoxycarbonyl)amino]2(S)-(benzyloxymethyl)oxy-4-phenyl propionaldehyde (0.60 g), as a clear resin. The crude product was homogeneous by thin-layer chromatography (silica gel; diethyl ether:hexanes; 25%;75%).

Step D: Preparation of ethyl (2R,3R,4R,5S), (2S,3R,4R,5S),(2S,3S,4R,5S) and (2R,3S,4R,5S)-5-[(1,1-dimethyl-ethoxycarbonyl)amino]-3-hydroxy-4-(benzyloxymethyl)-oxy-6-phenyl-2-(phenylmethyl) hexanoate

To a stirred solution of 0.60 mL of diisopropylamine in 5 mL of dry tetrahydrofuran cooled to -78 °C was added 2.0 mL of 1.6 M n-butyllithum in hexanes. After 5 minutes, a solution of ethyl dihydrocinnamate (0.48 g, 2.7 mmole) in 2 mL tetrahydrofuran was added dropwise over 5 minutes. After an additional 5 minutes at -78 °C, a solution of the crude product of Step C, 0.60 g (1.5 mmol), in 5 mL of dry tetrahydrofuran was added. The reaction mixture was allowed to warm to -40 °C for 15 minutes, then quenched with 20 mL of 10% citric acid and extracted into 3 X 50 mL of diethyl ether. The combined organic extracts were washed 1 X 50 mL of $H_2O$, 1 X 50 mL sat'd $NaHCO_3$, dried over $MgSO_4$ and concentrated to give a colorless oil. The crude product was taken up in 5 mL of ethanol and 20 mg of $NaBH_4$ was added. After 5 minutes the mixture was concentrated to dryness, diluted with 10 mL of 10% citric acid and extracted with 3 X 50 mL ethyl acetate. The combined organic extracts were washed 1 X 50 mL sat'd $NaHCO_3$, dried over $MgSO_4$, and concentrated to give an oil. Purification by medium pressure chromatography (silica gel E. Merck Lobar™ size C column), eluting with 15% ethyl acetate/hexanes gave first the (2R,3R,4R,5S) and (2S,3R,4R,5S) isomers, 0.14 g, as an inseparable mixture, then secondly 0.13 g of the (2S,3S,4R,5S) isomer. The latter fractions contained 0.19 g of ethyl (2R,3S,4R,5S)-5-[(1,1-dimethylethoxycarbonyl)-amino]-3-hydroxy-4-(benzyloxymethyl)oxy-6-phenyl-2-(phenylmethyl)hexanoate as a clear resin.

Step E: Preparation of 5(S)-[(1,1-dimethylethoxycarbonyl)amino]-3(S)-hydroxy-4(R)-(benzyloxymethyl)oxy-6-phenyl-2-(phenylmethyl) hexanoic acid

The (2R,3S,4R,5S) product of Step D, 0.19 g, was dissolved in 4 mL of 1,2-dimethoxyethane, and to it was added 4 mL of 1 M lithium hydroxide. The mixture was heated to 40 °C for 30 minutes, allowed to cool to room temperature, then acidified with 10 mL 10% citric acid and extracted with 3 X 50 mL of diethyl ether. The combined organic extracts were dried over $MgSO_4$ and concentrated to give 0.18 g of crude carboxylic acid which was homogeneous by thin layer chromatography (silica gel plate; methanol:chloroform; 5%:95%).

Step F: Preparation of N′-(1,1-dimethylethoxycarbonyl)-5(S)-amino-3(S)-hydroxy-4(R)-(benzyloxymethyl)oxy-6-phenyl-2-(phenylmethyl) hexanoyl leucyl-phenylalanyl amide

The product of Step E, 0.18 g, was dissolved in 4 mL of dry DMF, and to it was added 0.13 g of

leucinyl-phenylalanyl amide hydrochloride hemihydrate, 0.046 g of 1-hydroxybenzotriazole hydrate and 0.095 g of dimethyl-3-(3-dimethylaminopropyl)carbodiimide hydrochloride. Triethylamine, 92 mL, was added and the mixture allowed to stir at room temperature for 24 hours, then diluted with 50 mL of 10% citric acid and extracted with 3 X 50 mL of ethyl acetate. The combined organic extracts were washed with 70 mL of water, 50 mL of sat'd $NaHCO_3$, dried over $MgSO_4$ and concentrated to give 0.2 g of crude product purification by low pressure chromatography on silica gel, eluting with 5% methanol/chloroform gave 0.12 g of a clear resin.

Step G: Preparation of N′-(1,1-dimethylethoxycarbonyl)-5(S)-amino-3(S),4(R)-dihydroxy-6-phenyl-2(R)-(phenylmethyl) hexanoyl leucyl phenylalanyl amide

The product from Step H, 0.12 g, was dissolved in 20 mL of absolute ethanol, and to it was added 100 mg of 10% palladium on carbon and 5 mL of glacial acetic acid. The mixture was stirred under an atmosphere of hydrogen for 5 days at room temperature, then filtered and concentrated to dryness. The residue was dissolved in 1 mL of tetrahydrofuran and 1 mL of water was added. A white solid was collected and dried under vacuum over $P_2O_5$. The yield was 0.040 g of product. mp 231-232° (C,H,N).

## EXAMPLE 15

Preparation of N-(3(S)-hydroxy-4(S)-benzopyranyl)-N′-(1,1-dimethylethoxycarbonyl)-5(S)-amino-4(S)-hydroxy-6-phenyl-2(R)-(phenylmethyl) hexanamide

Step A: Preparation of tetrahydro-2H-benzopyrano-(4,3)oxazole

A mixture of 0.138 g of silver cyanate and 0.228 g of iodine in 5 mL of ether were stirred at room temperature for 1 hour. The reaction mixture was then cooled (ice-water bath) and a solution of 0.116 g of 3-chromene in 1 mL of ether was added dropwise. The suspension was stirred vigorously at room temperature for about 4 hours and the precipitated silver salts filtered off. Methanol (5 mL) and a few drops of a solution of lithium methoxide in methanol, were added to the filtrate and the mixture stirred at room temperature for 12 hours. Solvents were then evaporated, and the residue redissolved in a small volume of ether, washed with water, dried over anhydrous $Na_2SO_4$, and evaporated to furnish iodo-carbamate. The crude iodo-carbamate was dissolved in 2.5 mL of diglyme and heated to 160°C for 12 hours. After evaporation of solvent, the residue was chromatographed over silica gel (50% ethyl acetate-hexane as eluent) to give the Step A product.

Step B: Preparation of Cis-4-aminochroman-3-ol, also known as (cis) 3-hydroxy-4-amino-benzopyran

The oxazole from Step A was dissolved in 1 mL of methanol and 2 mL of 1 N methanolic potassium hydroxide was added. The resulting mixture was heated to 40°C for 12 hours. After this period, methanol was evaporated, and the residue diluted with water, and extracted thoroughly with a mixture (3:1) of chloroform-ether (3 X 10). The combined extracts were dried over anhydrous $Na_2SO_4$ and evaporated to afford the Step B amino-chromanol product which was used directly without further purification.

Step C: Preparation of N-(3(S)-hydroxy-4(S)-benzopyranyl)-N′-(1,1-dimethylethoxycarbonyl)-5(S)-amino-4-(S)-(1′,1′-dimethylethyl-1,1-dimethylsilyloxy)-6-phenyl-2(R)-S(phenylmethyl) hexanamide

N′-(1,1-dimethylethoxycarbonyl)-5(S)-amino-4(S)-(1′,1′-dimethylethyl-1,1-dimethylsilyloxy)-6-phenyl-2-(R)-(phenylmethyl)hexanoic acid of Example 1, Step H, 0.054 g, was dissolved in 1.5 mL of dry DMF, and to it was added 0.020 g of 1-hydroxybenztriazole hydrate, 0.029 g of 1-ethyl-3-(3-dimethylaminopropyl) carbodiimide hydrochloride and 0.018 g of cis-3-hydroxy-4-amino-benzopyran (racemic). Triethylamine was added to the stirring solution until the pH is 8.5. After stirring for 12 hours at room temperature, the reaction

was poured into 10 mL of water and extracted with 3 X 15 mL of ethyl acetate. The combined organic extracts were washed with 10% citric acid, water, saturated aqueous $NaHCO_3$ solution and dried over anhydrous $Na_2SO_4$. Evaporation of the solvent gave a residue of a mixture of diastereomers which were separated by chromatography over silica gel (50% ethyl acetate/hexanes) to afford the pure isomers as colorless oils.

Step D: Preparation of n-(3(S)-hydroxy-4(S)-benzopyranyl)-N′-(1,1-dimethylethoxycarbonyl)-5(S)-amino-4(S)-hydroxy-6-phenyl-2(R)-(phenylmethyl) hexanamide

The bottom diastereomer (50% ethyl acetate-hexane) from Step C (0.022 g), was placed in a flask and to it was added 0.3 mL of a 1 M solution of tetrabutylammonium fluoride in THF. After stirring for 12 hours at room temperature, the solvent was removed in vacuo, and the residue was extracted with ethyl acetate (2 X 15 mL). The combined extracts were washed sequentially with brine and water and dried over anhydrous $Na_2SO_4$. Evaporation of the solvent gave a residue which was chromatographed over silica gel to afford the product as a white solid.

EXAMPLE 16

Preparation of N-(2,3-dihydroxypropyl)-N′-(1,1-dimethylethoxycarbonyl)-5(S)-amino-4(S)-hydroxy-6-phenyl-2-(R)-(phenylmethyl) hexanoyl valyl amide

Step A: Preparationof N′-(1,1-dimethylethoxycarbonyl)valyl-N-(2,3-dihydroxypropyl)amide

N′-(1,1-dimethylethoxycarbonyl)valine, 4.34 g, was dissolved in 100 mL of dry DMF, and to it was added 1.82 g of 1-amino-2,3-dihydroxypropane, 3.82 g of 1-ethyl-3-(3-dimethylaminopropyl) carbodiimide hydrochloride, and 2.7 g of 1-hydroxybenztriazole hydrate. Triethylamine was then added until the pH was 8.5. The reaction was stirred for 2 hours, after which time it was poured into 500 mL of water and extracted with 3 X 200 mL ethyl acetate. The organics were combined and extracted with 3 X 50 mL 10% citric acid, 1 X 50 mL water, 1 X 50 mL of a saturated aqueous solution of $Na_2CO_3$, 1 X 50 mL brine, dried ($Na_2SO_4$), and the solvent removed to give the Step A product without need for further purification.

Step B: Preparation of N′-(1,1-dimethylethoxycarbonyl)-5(S)-amino-4(S)-(1′,1′-dimethylethyl-1,1-dimethylsilyloxy)-6-phenyl-2(R)-(phenylmethyl)hexanoylvalyl-N-(2,3-dihydroxypropyl)amide

The Step A product, N′-(1,1-dimethylethoxycarbonyl)valyl-N-(2,3-dihydroxypropyl)amide, .029 g, was dissolved in 10 mL of ethyl acetate and cooled to 0° C with an ice bath. HCl gas was then bubbled through the solution for 10 minutes after which time the solvent was removed. The residue was dissolved in 10 mL dry DMF, and reacted as in Step H above using .05 g N′-(1,1-dimethylethoxycarbonyl)-5(S)-amino-4(S)-(1′,1′-dimethylethyl-1,1-dimethylsilyloxy)-6-phenyl-2(R)-(phenylmethyl)hexanoic acid from Step A in Example 1, .019 g 1-ethyl-3-(3-dimethylaminopropyl) carbodiimide hydrochloride, and .014 g 1-hydroxybenztriazole hydrate at a pH of 8.5 to give the Step B product which was purified by preparative thin layer chromatography (10% methanol/chloroform saturated with ammonia).

Step C: Preparation of N′-(1,1-dimethylethoxycarbonyl)-5(S)-amino-4(S)-hydroxy-6-phenyl-2(R)-(phenylmethyl)-hexanoylvalyl-N-(2,3-dihydroxypropyl)amide

The product from Step B, .047 g, was treated with 1 mL of a 1 M solution of tetrabutylammonium fluoride in THF in a similar manner as Step B. Example 2 to give .023 g of the product (M.P. = 170-171 0° C) after purification by preparative thin layer chromatography (10% methanol/methylene chloride).

EXAMPLE 17

Preparation of N-(Methyl-5-amino-5-deoxy-β-D-ribosyl)-N′-(1,1-dimethylethoxycarbonyl)-5(S)-amino-4(S)-hydroxy-6-phenyl-2(R)-(phenylmethyl)hexanoyl valyl amide

Step A: Preparation of Methyl-5-amino-5-deoxy-β-D-ribosylvalamide

To a stirred solution of 0.50 g og N′-(1,1-dimethylethoxycarbonyl)-valine-hydroxy succinimide ester in 10 mL of DMF was added a solution of 0.808 g of methyl-5-amino-5-deoxy-2,3,O-isopropylidene-β-D-ribose in 2 mL of DMF. After stirring the resulting mixture at room temperature for 12 hours, the solvent was removed in vacuo, and the residue diluted with 10 mL of brine and extracted with ethyl acetate (3 X 10). The combined extracts were dried over anhydrous $Na_2SO_4$ and evaporated. The residue was chromatographed over silica gel (50% ethyl acetate-hexane) to give methyl-5-amino-5-deoxy-2,3,O-isopropylidene-β-D-ribosyl-N′-(1,1-dimethylethoxycarbonyl) valamide. To a solution of 0.316 g of the above valamide in 5 mL of ethyl acetate-methanol (1:1) at -25° C, anhydrous hydrochloric gas was passed for 5 minutes. The resulting solution was stirred at that temperature for 2 hours and then nitrogen gas bubbled through. Evaporation of the solvent gave a residue which was washed with ether (2x) and dried under vacuum to give the Step A product amine hydrochloride salt as a white solid.

Step B: Preparation of N-(Methyl-5-amino-5-deoxy-β-D-ribosyl)-N′-(1,1-dimethylethoxycarbonyl)-5(S)-amino-4(S)-(1′,1′-dimethylethyl-1,1-dimethylsilyloxy)-6-phenyl-2(R)-(phenylmethyl)-hexanoyl-valyl amide

N′-(1,1-dimethylethoxycarbonyl)-5(S)-amino-4(S)-(1′,1′-dimethylethyl-1,1-dimethylsilyloxy)-6-phenyl-2-(R)-(phenylmethyl) hexanoic acid,0.150 g, was dissolved in 8 mL of dry DMF, and to it was added 0.115 g of 1-hydroxybenztriazole hydrate, 0.163 g of 1-ethyl-3-(3-dimethylaminopropyl) carbodiimide hydrochloride and 0.235 g of Step A product methyl-5-amino-5-deoxy-β-D-ribosyl-valamide. Triethylamine was added to the stirring solution until the pH was 8.5. After stirring for 12 hours at room temperature, the reaction was poured into 10 mL of water and extracted with 3 X 15 mL of ethyl acetate. The combined organic extracts were washed with 10% citric acid, water, saturated aqueous $NaHCO_3$ solution and dried over anhydrous $Na_2SO_4$. Evaporation of the solvent gave a residue which was flash chromatographed over silica gel (5% methanol-chloroform) to afford the Step B product compound as a colorless oil.

Step C: Preparation of N-(Methyl-5-amino-5-deoxy-β-D-ribosyl)-N′-(1,1-dimethylethoxycarbonyl)-5(S)-amino-4(S)-hydroxy-6-phenyl-2(R)-(phenylmethyl)hexanoyl-valyl amide

The product from the previous step 0.170 g, was placed in a flask and to it was added 2 mL of a 1 M solution of tetrabutylammonium fluoride in THF. After stirring for 12 hours at room temperature, the solvent was removed in vacuo, and the residue extracted with ethyl acetate (2 X 15 mL). The combined extracts were washed sequentially with brine and water and dried over anhydrous $Na_2SO_4$. Evaporation of the solvent gave a residue which was chromatographed over silica gel to afford the product as a white solid (mp 186-189° C).

EXAMPLE 18

Preparation of N-(2(R)-hydroxy-1(S)-indanyl)-N′-(1,1-dimethylethoxycarbonyl)-5(S)-amino-4(S)-hydroxy-6-(2-naphthyl)-2(R)-(phenylmethyl)hexanamide

Step A: Preparation of N-( -2(R)-hydroxy-1(S)-indanyl)N′-(1,1-1,1-dimethylethoxycarbonyl)-5(S)-amino-4(S)-(1′,1′-dimethylethyl-1,1-dimethylsilyloxy)-6-(2-naphthyl-2(R)-(phenylmethyl)hexanamide

N′(1,1-dimethylethoxycarbonyl)-5(S)-amino-4(S)-(1′,1′-dimethylethyl-1,1-dimethylsilyloxy)-6-(2-naphthyl)-2(R)-(phenylmethyl)hexanoic acid, 0.068 g (0.118 mmol), [prepared in a similar manner as described for Example 1, Steps A-H but starting in Step A with N-2 (S)-[(1,1-dimethylethoxycarbonyl)-amino]-3-(2-naphthyl)propionaldehyde], was dissolved in 2 mL of dry DMF. To this solution were added 0.025 g (0.13 mmol) of EDC, 0.018 g (0.13 mmol) of HOBT, and 0.023 g (0.15 mmol) of 1(S)-amino-2(R)-hydroxyindane. Triethylamine was added to the stirring solution until the pH was 8.5. After stirring at 25°C for 48 hours, the reaction mixture was poured into 20 mL of ice water and extracted with 3 X 20 mL of ethyl acetate. The combined organic extracts were washed with 1 X 50 mL of 10% citric acid, 1 X 50 mL of saturated aqueous sodium bicarbonate solution, and 1 X 50 mL of brine, dried ($Na_2SO_4$). Filtration and concentration gave 0.08 g of the product which was used without further purification.

Step B: Preparation of N-(2(R)-hydroxy-1(S)-indanyl)-N′-(1,1-dimethylethoxycarbonyl)-5(S)-amino-4(S)-hydroxy-6-(2-naphthyl)-2(R)-(phenylmethyl)-hexanoic carboxamide

The product from Step A, 0.08 g, was dissolved in 1 mL of THF and to it was added 1 mL of a 1 M solution of tetrabutylammonium fluoride in THF. After stirring for 7 hours, the solvent was removed in vacuo, and the residue was treated with 20 mL of 10% citric acid solution to precipitate Step B product, after chromatography on silica gel eluting with $CHCl_3$:$CH_3OH$, 97:3. An analytical sample was obtained by recrystallization from $CH_2Cl_2$:hexane, mp 198-200°C;

| elemental analysis, Calcd. for $C_{37}H_{42}N_2O_5$ (594.76): | | | | | | |
|---|---|---|---|---|---|---|
| | C = | 74.72; | H = | 7.12; | N = | 4.60. |
| Found: | C = | 74.36; | H = | 7.46; | N = | 4.60. |

EXAMPLE 19

Preparation of N-(2-benzimidazolylmethyl)-N′-(1,1-dimethylethoxycarbonyl)-5(S)-amino-4(S)-hydroxy-6-phenyl-2(R)-(phenylmethyl)-hexanoyl isoleucyl amide

Step A: Preparation of N-(1,1-dimethylethoxycarbonyl)isoleucyl(succinimide)

A 15 g (0.065 mole) quantity of Boc-isoleucine, 8.2 g of N-hydroxysuccinimide, and 13.7 g of EDC were dissolved in 80 mL of DMF. After stirring at 25°C for 18 hours, the solution was added to 500 mL of water and extracted with 3 x 200 mL of ethyl acetate. The organic layers were combined, washed with 4 X 200 mL portions of water, 200 mL of brine, and dried ($Na_2SO_4$). Filtration and concentration in vacuo gave 20.2 g (95%) of Step A product, mp 62-64°C.

Step B: Preparation of N-(2-benzimidazolylmethyl)-N′-(1,1-dimethylethoxycarbonyl)isoleucyl amide

The product of Step A, 4.6 g (14 mmol), was dissolved in 70 mL of 1,2-dimethoxyethane, and to it were added 5.9 g (28 mmol) of 2-aminomethyl-benzimidazole dihydrochloride and 7.8 mL (56 mmol) of triethylamine. After stirring at 25°C for 18 hours, the solvent was removed in vacuo and the residue was dissolved in 250 mL of ethyl acetate. This solution was washed with 3 x 100 mL portions of water, 100 mL of brine, and dried ($Na_2SO_4$). Filtration and concentration in vacuo gave 4.4 g (87%) of Step B product, after chromatography on silica gel ($CH_2Cl_2$:$CH_3OH$, 95:5).

Step C: Preparation of N-(2-benzimidazolylmethyl)-isoleucyl amide

The product of Step B, 2 g (5.6 mmol), was dissolved in 50 mL of ethyl acetate and cooled to -25°C.

Hydrogen chloride gas was bubbled into the solution for 0.75 hour or until TLC indicated complete reaction. Nitrogen was bubble into the reaction as it was left to warm to ambient temperature. Concentration in vacuo gave 1.6 g (86%) of Step C product.

Step D: Preparation of N-(2-benzimidazolylmethyl)-N′-(1,1-dimethylethoxycarbonyl)-5(S)-amino-4(S)-(1′,1′-dimethyl-1,1-dimethylsilyloxy)-6-phenyl-2(R)-(phenylmethyl)-hexanoyl isoleucyl amide

N′-(1,1-dimethylethoxycarbonyl)-5(S)-amino-4(S)-(1′,1′-dimethylethyl-1,1-dimethylsilyloxy)-6-phenyl-2-(R)-(phenylmethyl)-hexanoic acid, 0.50 g (0.95 mmol), was dissolved in20 mL of DMF. To this solution were added 0.199 g (1.04 mmol) of EDC, 0.141 g (1.04 mmol) of HOBT, and 0.38 g (1.14 mmol) of N-(2-benzimidazolylmethyl)isoleucyl amide dihydrochloride. Triethylamine was added to the stirring solution until the pH was 8.5. After stirring at 25°C for 72 hours, the reaction was poured into 100 mL of water and extracted with 2 X 100 mL of ethyl acetate. The combined organic extracts were washed with 3 X 50 mL portions of water, 50 mL of brine, and dried ($Na_2SO_4$). Concentration in vacuo gave 0.60 g (85%) of Step D product, after purification of column chromatography on silica gel ($CH_2Cl_2$:$CH_2OH$,95:5).

Step E: Preparation of N-(2-benzimidazolylmethyl)-N′-(1,1-dimethylethoxycarbonyl)-5(S)-amino-4(S)-hydroxy-6-phenyl-2(R)-(phenylmethyl)-hexanoyl isoleucyl amide

The product from Step D, 0.60 g (0.081 mol), was dissolved in 7 mL of THF and to it was added 7 mL of a 1 M solution of tetrabutylammonium fluoride in THF. After stirring for 18 hours, the solvent was removed in vacuo, and the residue was treated with 100 mL of water to precipitate 0.50 g (95%) of the title compound, after chromatography on silica gel ($CHCl_3$ saturated with ammonia:$CH_3OH$, 95:5). mp 232-233°C.

| Elemental analysis Calcd for $C_{38}H_{49}N_5O_5$: | | | | | | |
|---|---|---|---|---|---|---|
| | C = | 69.59; | H = | 7.53; | N = | 10.68. |
| Found: | C = | 69.66; | H = | 7.56; | N = | 10.54. |

## EXAMPLE 20

Preparation of N′-(1,1-dimethylethoxycarbonyl)-5(S)-amino-4(S)-hydroxy-6-phenyl-2(R)-(3′-phenylprop-2′-en-1′-yl)hexanoyl-(S)-phenylglycyl-(2-hydroxyethyl)-amide

Using the general procedure outlined in Example 1, Step F, (5S, 1′S) 3-carbethoxy-5-(1-((1,1-dimethylethoxycarbonyl)amino)-2-phenylethyl)dihydrofuran-2-(3H)-one (4.15 g, 11.0 mmol) was alkylated with cinnamyl bromide (2.17 g, 11.0 mmol) to give (5S,1′S) 3-carbethoxy-3-(3′-phenylprop-2′-en-1′-yl)-5-(1-((1,1-dimethylethoxycarbonyl)amino)-2-phenylethyl)-dihydrofuran-2-(3H)-one (5.43 g). This material (5.43 g, 11.0 mmol) was decarboxylated (Example 1, Step G) to give (3R,5S,1′S) 3-(3′phenylprop-2′-en-1′-yl)-5-(1-(-(1,1-dimethylethoxycarbonyl)amino)-2-phenylethyl)dihyrofuran-2-(3H)-one (2.50 g). Following Example 1, Step H, the aforementioned lactone (2.50 g, 5.9 mmol) was hydrolyzed, silylated, and rehydrolyzed to give N′-(1,1-dimethylethoxycarbonyl)-5(S)-amino-4(S)-(1′,1′-dimethylethyl-1,1-dimethylsilyloxy)-6-phenyl-2-(R)-(3′-phenylprop-2′-en-1′-yl)-hexanoic acid (2.14 g). This material (0.31 g, 0.6 mmol) was coupled to (S)-phenylglycine-2-hydroxyethyl amide (0.14 g. 0.6 mmol) according to Example 1, Step I to give N′-(1,1-dimethylethoxycarbonyl)-5(S)-amino-4(S)(1′,1′-dimethylethyl-1,1-dimethylsilyloxy)-6-phenyl-2(R)-(3′-phenylprop-2′-en-1′-yl-)hexanoyl (S)-phenylgycyl-2-hydroxyethyl amide (0.085 g). This silyl ether (0.085 g, 0.12 mmol) was deprotected according to Example 1, Step J to give the title compound (0.039 g), mp 198-200°C.

## EXAMPLE 21

Preparation of N-(1(R),2(S)-dihydroxy-3(S)-indanyl)-N′-(1,1-dimethylethoxycarbonyl)-5(S)-amino-4(S)-hydroxy-6-phenyl-2(R)-(3′-phenylprop-2′-en-1′-yl)-hexanamide

According to Example 1, Step I, N′-(1,1-dimethylethoxycarbonyl)-5(S)-amino-4(S)-(1′,1′-dimethylethyl-1,1-dimethylsilyloxy)-6-phenyl-2(R)-(3′-phenylprop-2′en-1′-yl)hexanoic acid (0.15 g, 0.27 mmol) was coupled to 1(R),2(S)-dihydroxy-3(S)-aminoindane (0.045 g, 0.27 mmol) to give N′-(1,1-dimethylethoxycarbonyl)-5(S)-amino-4(S)-(1′,1-dimethylethyl-1,1-dimethylsilyloxy)-6-phenyl-2(R)-(3′-phenylprop-2′-en-1′-yl)hexanoyl-1(R)-,2(S)-dihydroxy-3(S)-aminoindane (0.19 g). This material (0.19 g, 0.27 mmol) was deprotected according to Example 1, Step J to give the title compound, (0.11 g), mp 218-219° C.

## EXAMPLE 22

Preparation of N-(2-hydroxyethyl)-N′-(1,1-dimethylethoxycarbonyl)-5(S)-amino-4(S)-hydroxy-6-phenyl-2(R)-(phenylthiomethyl)hexanoyl-(S)-phenylglycyl amide

According to Example 1, Step F, (5S,1′S) 3-carbethoxy-5-(1-((1,1-dimethylethoxycarbonyl)amino)-2-phenylethyl)dihydrofuran-2-(3H)-one (5.8 g, 15.37 mmol) was alkylated with phenyl thiomethyl chloride (2.44 g, 15.37 mmol) to give (5S,1′S) 3-carbethoxy-3-(phenylthiomethyl)-5-(1-((1,1-dimethylethoxycarbonyl)amino)-2-phenylethyl)-dihydrofuran-2-(3H)-one (8.58 g). This material (8.58 g, 17.2 mmol) was decarboxylated according to Example 1, Step G, to give (3R,5S,1′S) 3-(phenylthiomethyl)-5-(1((1,1-dimethylethoxycarbonyl)amino)-2-phenylethyl)dihydrofuran-2-(3H)-one (1.3 g). Again, following Example 1, Step H, the decarboxylation product (1.3 g, 3.04 mmol) was hydrolyzed, silylated, and rehydrolyzed to give N′-(1,1-dimethylethoxycarbonyl)-5(S)-amino-4(S)-(1′,1′-dimethylethyl-1,1-dimethylsilyloxy)-6-phenyl-2(R)-(phenylthiomethyl)hexanoic acid (0.80 g). This material (0.35 g, 0.63 mmol) was coupled to (S)-phenylglycine-2-hydroxyethyl amide (0.43 g, 1.87 mmol) according to Example 1, Step I to give N′-(1,1-dimethylethyl-1,1-dimethylsilyloxy)-6-phenyl-2(R)- (phenylthiomethyl)hexanoyl-(S)-phenylglycyl-2-hydroxyethylamide (0.34 g). This silyl ether (0.33 g, 0.45 mmol) was deprotected according to Example 1, Step J to give the title compound (0.01 g), mp 158-159° C.

## EXAMPLE 23

Preparation of N-(2(R)-hydroxy-1(S)-indanyl)-N′-(1,1-dimethylethoxycarbonyl)-5(S)-amino-4(S)-hydroxy-6-phenyl-2(R)-((4-(1,1-dimethylethyl)phenyl)methyl)-hexanamide

According to Example 1, Step F, (5S,1′S) 3-carbethoxy-5-(1-((1,1-dimethylethoxycarbonyl)amino)-2-phenylethyl)dihydrofuran-2-(3H)-one (2.28 g, 6.05 mmol) was alkylated with 4-(1,1-dimethylethyl)-phenylmethyl bromide (1.37 g, 6.05 mmol) to give (5S,1′S) 3-carbethoxy-3-(4-(1,1-dimethylethyl)-phenylmethyl)-5-(1-((1,1-dimethylethoxycarbonyl)amino)-2-phenylethyl)-dihydrofuran-2-(3H)-one (3.17 g). This material (3.17 g. 6.05 mmol) was decarboxylated according to Example 1, Step G, to give (3R,5S,1′S) 3-(4-(1,1-dimethylethyl)phenylmethyl)-5-(1-((1,1-dimethylethoxycarbonyl)amino)-2-phenylethyl)dihydrofruan-2-(3H)-one (0.60 g). Following Example 1, Step H, the decarboxylation product (0.60 g, 1.33 mmol) was hydrolyzed, silylated, and rehydrolyzed to give N′-(1,1-dimethethoxycarbonyl)-5(S)-amino-4(S)-(1,1-dimethylethyl-1,1-dimethysilyloxy)-6-phenyl-2(R)-(4-(1,1-dimethylethyl)phenylmethyl)hexanoic acid (0.22 g). This material (0.14 g, 0.24 mmol) was coupled to 1(S)-amino-2(R)-hydroxyindane (0.043 g, 0.29 mmol) according to Example 1, Step I, to give N′-(1,1-dimethylethoxycarbonyl)-5(S)-amino-4(S)- (1′,1′-dimethylethyl-1,1-dimethylsilyloxy)-6-phenyl-2(R)-(4-(1,1-dimethylethyl)phenylmethyl)hexanoyl-1(S)-amino-2-(R)-hydroxyindane (0.172 g). This silyl ether (0.172 g, 0.24 mmol) was deprotected according to Example 1, Step J, to give the title compound (0.067 g), mp 187-189° C.

## EXAMPLE 24

Preparation of N-(2-benzimidazolylmethyl)-N′-(4-pyridylmethoxycarbonyl)-5(S)-amino-4(S)-hydroxy-6-phenyl-2(R)-(phenylmethyl)hexanoylisoleucyl amide

Step A: Preparation of N-(2-benzimidazolylmethyl)-5(S)-amino-4(S)-hydroxy-6-phenyl-2(R)-(phenylmethyl)-hexanoyl isoleucyl amide

N-(2-benzimidazolylmethyl)-N′-(1,1-dimethylethoxycarbonyl)-5(S)-amino-4(S)-hydroxy-6-phenyl-2(R)-(phenylmethyl)-hexanoyl isoleucyl amide, 0.08 g (0.12 mmol) was dissolved in 10 mL of $CH_2Cl_2$ with stirring and cooling in an ice water bath under argon. To this solution was added 2 mL of trifluoroacetic acid. After stirring at 0° C for 2 hours, the reaction was concentrated in vacuo, and the residue partitioned between 100 mL of $CH_2Cl_2$ and 50 mL of saturated aqueous sodium bicarbonate solution. The organic layer was separated, washed with 50 mL of brine and dried ($Na_2SO_4$), filtered and concentrated in vacuo to give 0.065 g (97%) of N-(2-benzimidazolymethyl)-5(S)-amino-4(S)-hydroxy-6-phenyl-2(R)-(phenylmethyl)-hexanoyl isoleucyl amide.

Step B: N-(2-benzimidazolylmethyl)-N′-(4-pyridylmethoxycarbonyl)-5(S)-amino-4(S)-hydroxy-6-phenyl-2(R)-(phenylmethyl)-hexanoyl isoleucyl amide

The product from Step A, 0.065 g (0.12 mmol), and 0.185 g (0.68 mmol) of isonicotinoyl p-nitrophenyl carbonate were dissolved in 6 mL of dry DMF. After stirring at 25° C for 18 hours, the reaction was dissolved in 50 mL of $CH_2Cl_2$, washed with 5 X 25 mL portions of 5% sodium hydroxide solution, 25 mL of brine, and dried ($Na_2SO_4$). Filtration and concentration in vacuo followed by chromatography on silica gel ($CH_2Cl_2$:$CH_3OH$, 95:5) gave 0.016 g (20%) of title compound, mp 210-212° C;

| elemental analysis Calcd. for $C_{40}H_{46}N_6O_5 \cdot 0.75\ H_2O$: | | | | | | |
|---|---|---|---|---|---|---|
| | C = | 60.20; | H = | 6.80; | N = | 11.93. |
| Found: | C = | 68.14; | H = | 6.62; | N = | 11.81. |

## EXAMPLE 25

Preparation of Dilithium N-(2-phosphoryloxyethyl)-N′-(1,1-dimethylethoxycarbonyl)-5(S)-amino-4(S)-hydroxy-6-phenyl-2(R)-(phenylmethyl)hexanoyl-leucine amide

Step A: Preparation of Methyl N′-(1,1-dimethylethoxycarbonyl)-5(S)-amino-4(S)-hydroxy-6-phenyl-2(R)-(phenylmethyl)hexanoyl-isoleucinate

A solution of 170 mg (0.32 mmol) of N′-(1,1-dimethylethoxycarbonyl)-5(S)-amino-4(S)-(t-butyldimethylsilyloxy)-6-phenyl-2(R)-(phenylmethyl)hexanoic acid, prepared as described in Example 1, Step H, was dissolved in 1 mL DMF. To this solution was added 47 mg (0.35 mmol) 1-hydroxybenzotriazole hydrate, 68 mg (0.35 mmol) dimethyl-3-(3-dimethylaminopropyl)-carbodiimide hydrochloride and 64 mg (0.35 mmol) isoleucine methyl ester hydrochloride. After 5 minutes, triethylamine (98 µL, 0.70 mmol) was added and the mixture was stirred overnight. The product was partitioned between EtOAc and 10% citric acid and the organic phase was washed with water and brine. After drying ($Na_2SO_4$), the solvent was evaporated to give 250 mg of an oil which was purified by chromatography on silica (10% EtOAc/hexane). This material (220 mg) was dissolved in 2 mL 1 M tetrabutylammonium fluoride in THF. After stirring overnight, the mixtire was diluted with ether and washed with 10% citric acid, water and brine. After drying, the solvent was

evaporated to give 171 mg of material which was chromatographed on silica (2:1 hexane/EtOAc). There was obtained 153 mg of the Step A product as a white solid.

Step B: Preparation of N-(2-hydroxyethyl)-N′-(1,1-dimethylethoxycarbonyl)-5(S)-amino-4(S)-hydroxy-6-phenyl-2(R)-(phenylmethyl)hexanoyl-leucine amide

The methyl ester obtained in Step A (0.28 mmol) was dissolved in 3 mL DME and 0.6 mL 1 N LiOH was added followed by 1 mL water. The mixture was stirred 1 hour at room temperature and overnight at 4° C. This mixture was allowed to warm to room temperature over 2 hours, diluted with 10% citric acid and the product was extracted into EtOAc. The extract was washed with water and brine. The extract was dried ($Na_2SO_4$) and evaporated to give 125 mg of a white solid. This solid was dissolved in 2 mL DMF with 1-hydroxybenzotriazole (28 mg, 0.28 mmol), ethanolamine (34 µL, 0.56 mmol) and dimethyl-3-(3-dimethylaminopropyl)carbodiimide hydrochloride (54 mg, 0.28 mmol). After stirring overnight, the mixture was diluted with water and extracted with EtOAc. The extract was washed with 10% citric acid, water and brine. After drying ($Na_2SO_4$), the solvent was evaporated to give a white solid. The solid was stirred with EtOAc and a white solid weighing 5.4 mg was isolated by filtration. Recrystallization from acetone gave 30 mg of the Step B product, mp 202-204° C.

Step C: Preparation of N-(2-dibenzylphosphoryloxyethyl)-N′-(1,1-dimethylethoxycarbonyl)-5(S)-amino-4(S)-hydroxy-6-phenyl-2(R)-(phenylmethyl)hexanoyl-leucine amide

The phosphorylating agent [bis(benzyloxy)-(N,N-diisopropylamino)phosphine] used in the following procedure is described in W. Bannwarth and A. Trzeciak, Helv. Chim. Acta. 70, 175 (1987).

The compound prepared in Step B (21.7 mg, 0.038 mmol) was dissolved in 0.5 mL DMF and 15 mg (0.043 mmol) of bis(benzyloxy)-(N,N-diisopropylamino)phosphine was added followed by 3.9 mg (0.056 mmol) tetrazole. After 2 hours, an additional 11 mg of the phosphine and 3 mg of tetrazole were added and stirring was continued overnight. A quantity of m-Chloro-peroxybenzoic acid (30 mg of 65% purity, 0.056 mmol) was added and the mixture was stirred for 1 hour. The mixture was diluted with EtOAc and washed sequentially with 5% $NaHSO_3$, 10% $NaHCO_3$ and brine. After drying, the solvent was evaporated and the residue was chromatographed on 5 g silica gel (2.5% MeOH/$CHCl_3$). There was obtained 22.2 mg of Step C product.

| Analysis Calcd. for $C_{32}H_{47}N_3O_6$: | | | | | | |
|---|---|---|---|---|---|---|
| | C = | 67.46; | H = | 8.31; | N = | 7.38. |
| Found: | C = | 67.20; | H = | 8.37; | N = | 7.32. |

Step D: Preparation of Dilithium N-(2-phosphoryloxyethyl)-N′-(1,1-dimethyl-ethoxycarbonyl)-5(S)-amino-4(S)-hydroxy-6-phenyl-2(R)-(phenylmethyl)hexanoyl-leucine amide

The product of Step C (19.6 mg, 0.024 mmol) was dissolved in3 mL 95% ethanol and 19 mg 10% Pd/C was added. This mixture was stirred 4 hours under an atmosphere of hydrogen. Lithium hydroxide (48 L of 1 N) was added and the mixture was filtered through Celite. The Celite pad was washed with ethanol and water. The solvents were removed on a rotary evaporator and the residue was redissolved in 2 mL water. The cloudy solution was filtered through Celite and lyophilized to afford 16.7 mg of the title compound as a fluffy white powder. $^1H$-NMR ($D_2O$) δ (Chemical shift data relative to water peak assigned 5.50 ppm.) 7.29-7.05 (10H, m); 3.9 (1H, d); 3.64 (3H, m); 3.42 (1H, m); 3.16 (2H, t); 2.86-2.5 (6H, m); 1.5-1.8 (3H, m); 1.19 (9H, s); 0.98 (1H, m); 0.7 (6H, m).

EXAMPLE 26

Preparation of N-(2-(2-(2-methoxyethoxy)ethoxy)ethyl)-N′-(1,1-dimethylethoxycarbonyl)-5(S)-amino-4(S)-hydroxy-6-phenyl-2(R)-(phenylmethyl)hexanoyl valyl amide

Step A: Preparation of 2-[2-(2-methoxyethoxy)ethoxy]-ethyl tosylate

Powdered KOH (10.6 g, 0.20 mol) was suspended in anhydrous diethyl ether (250 mL) and cooled to 0 °C under an argon atmosphere. Triethylene glycol monomethyl ether (23.9 mL, 0.15 mol) and p-toluene sulfonyl chloride (30.5 g, 0.16 mol) were added sequentially to the suspension. The progress of the reaction was monitored by thin layer chromatography. After stirring for 16 hours, additional powdered KOH (2.5 g, 0.04 mol) and triethylene glycol monomethyl ether (1.0 mL, 0.006 mol) were added to the reaction mixture. After another 2 hours no p-toluenesulfonyl chloride was present in the reaction mixture. The reaction mixture was filtered of solids and the filtrate was washed with water, and brine, dried ($MgSO_4$), filtered, and concentrated in vacuo to give 46.5 g of Step A product as a colorless oil. $^1$H-NMR ($CDCl_3$) δ 2.45 (s, 3H); 3.38 (s, 3H); 3.53 (m, 2H); 3.60 (m, 6H); 3.68 (t, 2H); 4.16 (t, 2H); 7.32 (d, 2H); 7.80 (d, 2H).

Step B: Preparationof 2-[2-(2-methoxyethoxy)ethoxy]-ethyl phthalimide

Potassium phthalimide (40.7 g, 0.22 mol) and the product of Step A (46.5 g, 0.146 mol) were mixed together in anhydrous dimethyl formamide (150 mL) and heated at 120 °C for 2 hours under an argon atmosphere. The reaction mixture was cooled to room temperature, diluted with diethyl ether (600 mL) and filtered through Celite. The filtrate was concentrated in vacuo to give an oil. This oil was dissolved in diethyl ether (225 mL), and the ether solution washed with water, 0.1 N NaOH, and brine, dried ($MgSO_4$), filtered and concentrated in vacuo to give another oil. This oil was triturated with water, causing some solid to crystallize out. The solid was filtered and the filtrate extracted with methylene chloride (2 X 75 mL). The combined extracts were dried ($MgSO_4$), filtered, and concentrated in vacuo to give Step B product (24.4 g)-as a colorless oil. $^1$H-NMR ($CDCl_3$) δ 3.33 (s, 3H); 3.48 (m, 2H); 3.60 (m, 4H); 3.66 (m, 2H); 3.75 (t, 2H); 3.91 (t, 2H); 7.70 (m, 2H); 7.84 (m, 2H).

Step C: Preparation of 2-[2-(2-methoxyethoxy)ethoxy]-ethyl amine

The product of Step B (24.3 g, 0.083 mol) was dissolved in ethanol (100 mL) and hydrazine (26.5 mL, 0.83 mol) was added. The reaction mixture was heated to reflux and a copius white solid precipitated out. The reaction mixture was cooled to room temperature, diluted with ethanol (500 mL) and filtered. The filtrate was concentrated in vacuo to give an oil. This oil was combined with the solid from above, mixed with water (250 mL) and the pH adjusted to 2.0 by addition of concentrated hydrochloric acid. An insoluble solid was filtered and the filtrate was made strongly basic by addition of 10 N NaOH. This solution was extracted with methylene chloride(4 x 100 mL). The methylene chloride extracts were combined, dried (NaOH) and concentrated in vacuo to give 12.1 g of oil. This oil was chromatographed on silica gel eluted with 5% methanol in chloroform, saturated with ammonia to give Step C product (9.57 g) as a colorless oil. $^1$H-NMR ($CDCl_3$) δ 1.54 (s, 2H); 2.87 (t, 2H); 3.38 (s, 3H); 3.52 (t, 2H); 3.56 (m, 2H); 3.66 (m, 6H).

Step D: Preparation of N-(tert-butoxycarbonyl)-L-valine succinimide ester

N-(tert-butoxy carbonyl)-L-valine (5.02 g, 0.023 mol), N-hydroxy succinimide (2.88 g, 0.24 mol), and 1-(3-dimethylaminopropyl)-3-ethyl carbodiimide hydrochloride (4.89 g, 0.025 mol) were dissolved together in anhydrous dimethylformamide (25 mL). After stirring for 16 hours at room temperature, the reaction mixture was concentrated in vacuo to remove solvent. The residue was partitioned between ethyl acetate and 10% aqueous citric acid. The organic phase was separated, washed with saturated aqueous $NaHCO_3$, brine, dried ($MgSO_4$), filtered and concentrated in vacuo to give 6.83 g of a white solid. $^1$H-NMR ($CDCl_3$) δ 1.05 (m, 6H); 1.46 (s, 9H); 2.30 (m, 1H); 2.85 (s, 4H); 4.60 (dd, 1H); 5.02 (d. 1H).

Step E: Preparationof N-[2-(2-[2-methoxyethoxy]-ethoxy)ethyl]valine amide hydrochloride

The product of Step D (130.5 mg, 0.41 mmol) and the product of Step C (73 mg, 0.45 mmol) were dissolved together in dimethoxyethane (1.5 mL) at room temperature under an atmosphere of argon. After 4 hours, the reaction mixture was concentrated in vacuo to remove solvent and the residue was chromatographed on silica gel using a gradient elution of 0-5% methanol in chloroform. This gave 108.5 mg of a colorless oil as product. $^1$H-NMR ($CDCl_3$) δ 0.90 (d, 3H); 0.95 (d, 3H); 1.45 (s, 9H); 2.10 (m, 1H); 3.40 (s, 3H); 3.48 (m, 2H); 3.58 (m, 4H); 3.62 (m, 6H); 3.92 (t,1H); 5.15 (d, 1H); 6.40 (br.s, 1H). This oil was dissolved in ethyl acetate (5 mL) and cooled to 0° C. Hydrogen chloride gas was bubbled into the solution vigorously for 20 seconds and then stopped. After another 15 minutes at 0° C, the reaction mixture was concentrated in vacuo to give 76.8 mg of a non-crystalline solid as the Step E product.

Step F: Preparation of N-(2-(2-(2-methoxyethoxy)-ethoxy)ethyl)-N′-(1,1-dimethylethoxycarbonyl)-5(S)-amino-4(S)-hydroxy-6-phenyl-2-(R)-(phenylmethyl)hexanoyl valyl amide

5(S)-tert-butoxycarbonylamino-2(R)-benzyl-4(S)-tert-butyldimethylsilyloxy-6-phenyl-hexanoic acid (98 mg, 0.186 mmol), 1-hydroxybenzotriazole (28.9 mg, 0.208 mmol), 1-(3-dimethylaminopropyl)-3-ethyl carbodiimide hydrochloride (40.2 mg, 0.21 mmol) and the product of Step E (69 mg, 0.231 mmol) were dissolved together in anhydrous dimethyl formamide (4.5 mL). Triethyl amine (70 μl, 0.502 mmol) was added to the solution and the reaction mixture was stirred at room temperature for 16 hours under an atmosphere of argon. The solvent was removed in vacuo and the oil residue was partitioned between ethyl acetate and 10% aqueous citric acid. The organic phase was separated and washed with brine, dried ($MgSO_4$), filtered and concentrated in vacuo to give an oil weighing 176.8 mg. This oil chromatographed on silica gel eluted with a gradient of 0-3% methanol in methylene chloride to give 135 mg of a colorless oil. This oil was dissolved in a 1 molar tetrahydrofuran solution of tetrabutyl ammonium fluoride (3 mL) and stirred at room temperature for 16 hours. The reaction mixture was concentrated in vacuo and the residue partitioned between ethyl acetate and 10% aqueous citric acid. The organic phase was separated, washed with saturated aqueous $NaHCO_3$ and brine, dried ($MgSO_4$) and concentrated in vacuo to give 108 mg of oil. This oil wash chromatographed on silica gel eluted with 10% ethanol in hexane to give 35 mg of the title compound as a white solid. $^1$H-NMR ($CDCl_3$) δ 0.79 (d, 3H); 0.84 (d, 3H); 1.36 (s, 9H); 1.73 (m, 2H); 2.00 (m, 1H); 2.66 (dd, 1H); 2.86 (m, 4H); 3.33 (m, 5H); 3.56 (m, 12H); 4.13 (t, 1H); 5.00 (d, 1H); 7.00 (d, 2H); 7.17 (m, 10H).

| Analysis Calcd. for $C_{36}H_{55}N_3O_8$•1/2 $C_2H_6O$: | | | | | | |
|---|---|---|---|---|---|---|
| | C = | 65.27; | H = | 8.59; | N = | 6.17. |
| Found: | C = | 65.19; | H = | 8.34; | N = | 6.25. |

## EXAMPLE 27

Preparation of N′-(1,1-dimethylethoxycarbonyl)-4(S)-amino-5-cyclohexyl-3(S)-hydroxypentanoyl-glutamyl-phenylalanine amide

Step A: Preparation of N′-(1,1-dimethylethoxycarbonyl)-4(S)-amino-5-cyclohexyl-3(S)-hydroxypentanoyl-(w-O-benzylglutamyl)-phenylalanine amide

To a solution of Glu (γ$OCH_2C_6H_5$)-Phe-$NH_2$•HCl (820 mg, 0.00195 mole), Boc ACHPA•OH (590 mg, 0.0019 mole), and 1-hydroxybenzatriazole (800 mg, 0.0059 mole) in N,N-dimethylformamide (10 mL) cooled at -20° C was added N-methylmorpholine (1.0 mL, 0.009 mole) followed by 1-ethyl-3-(3-dimethylaminopropyl) carbodiimide-hydrochloride (375 mg, 0.0019 mole). After addition of another 0.7 mL of N-methylmorpholine (pH-8), the reaction mixture was stirred under $N_2$ at about -20° C for 2 hours and at room temperature overnight (20 hours). The solution was concentrated in vacuo and the residue taken up in ethyl acetate and 10% aqueous citric acid. The ethyl acetate solution was washed with water, aqueous $NaHCO_3$, and brine, and dried over $Na_2SO_4$. Filtration and evaporation gave a pale yellow residue, which

was triturated with ether to give the Step A product as a white solide (1.15 g, 90% yield), mp 147-150 ° C.

| Analysis Calcd. for $C_{37}H_{52}N_4O_8$ Nat. Wt. 680.855 | | | | | | |
|---|---|---|---|---|---|---|
| | C = | 65.27; | H = | 7.70; | N = | 8.23. |
| Found: | C = | 65.41; | H = | 7.81; | N = | 8.34. |

Step B: Preparation of N′-(1,1-dimethylethoxycarbonyl)-4(S)-amino-5-cyclohexyl-3(S)-hydroxypentanoyl-glutamyl-phenylalanine amide

A mixture of Boc ACHPA-Glu ($\gamma CH_2C_6H_5$) $PheNH_2$ (1.1 g, 0.0016 mole) and 10% palladium on activated carbon (60 mg) in methanol (25 mL) was stirred under a hydrogen atmosphere for 4 hours and then filtered through a pad of Super-cel. The filtrate was concentrated in vacuo and the residue recrystallized from methanol to give the title product as a colorless crystalline solid (560 mg, 56% yield), mp >180 ° C eff.

| Analysis Calcd for $C_{30}H_{46}N_4O_8 \cdot CH_3OH$, | | | | | | |
|---|---|---|---|---|---|---|
| | C = | 59.79; | H = | 8.09; | N = | 9.00. |
| Found: | C = | 59.43; | H = | 8.18; | N = | 9.06. |

## EXAMPLE 28

Preparation of N-(3-dimethylaminopropyl)-N′-(1,1-dimethylethoxycarbonyl)-5(S)-amino-4(S)-hydroxy-6-phenyl-2(R)-(phenylmethyl)hexanoyl valyl amide

Step A: Preparation of N′-(1,1-dimethylethoxycarbonyl)-5(S)-amino-4(S)-(1′ ,1′-dimethylethyl-1,1-dimethylsilyloxy)-6-phenyl-2(R)-(phenylmethyl)hexanoyl-N-(4 ,4 -dimethylamino propyl)valyl amide

N′-(1,1-dimethylethoxycarbonyl)-5(S)-amino4(S)-(1′ ,1′-dimethylethyl-1,1-dimethylsilyloxy)-6-phenyl-2-(R)-(phenylmethyl)hexanoyl-valine, 0.2 g, was reacted with 0.04 g of 4,4-dimethylaminopropyl amine, 0.075 g 1-ethyl-3-(3-dimethylaminopropyl) carbodiimide hydrochloride, and 0.053 g 1-hydroxybenztriazole hydrate using triethylamine to adjust the pH to 8.5 as in Step A, Example 2, to give the Step A product after purification of preparative thin layer chromatography (10% methanol/chloroform saturated with ammonia).

Step B: Preparation of N′-(1,1-dimethylethoxycarbonyl)-5(S)-amino-4(S)-hydroxy-6-phenyl2(R)-(phenylmethyl)-hexanoyl-N-(4′,4′-dimethylaminopropyl)valyl amide

The product from Step A, .18 g, was treated with 2 mL of a 1 M solution of tetrabutylammonium fluoride as in Step A, Example 2, to give .078 g of the product (mp 175-177 0 ° C) after purification by preparative thin layer chromatography (10% methanol/methylene chloride). A sample of this product, 0.03 g, and maleic acid, 0.006 g, were dissolved in 10 mL of methanol and stirred for 10 minutes, after which time the solvent was removed and the resulting solid recrystallized from ethyl acetate/methanol to yield 0.027 g of the product as the maleate salt (mp 125-127 0 ° C).

## EXAMPLE 29

Preparation of N-Benzyl-2(S)-[N-2(S)-((1,1-dimethylethoxycarbonyl)amino)-2-(4-hydroxyphenyl)-2(S)-hydroxy-2-propyl]-1(R)-cyclopentane carboxamide ("cis-Tyr-Pro")

Step A: Preparation of N-6(S)-[(1,1-Dimethylethoxycarbonyl)amino]-7-(4-benzyloxyphenyl)-(Z)-4-heptenoic acid

To a stirred suspension of 4-carboxybutyltriphenyl phosphonium chloride (77.4 g, 201 mmol), (prepared as described by D.B. Denney and L.C. Smith, J. Org. Chem., 27, 3404 (1962)) in 400 mL dry tetrahydrofuran cooled to 0° C in an ice/water bath and under nitrogen was added dropwise a solution of potassium bis(trimethylsilyl)amide in toluene (505 ml, 0.692 M, 349 mmol) such that the temperature of the reaction remained below 0.8° C. After addition the reaction was stirred at 0° C for 5 hours, cooled to -75° C in a dry ice/isopropanol bath, and a solution of N-2(S)-[(1,1-dimethylethoxycarbonyl)-amino]-3-(4-benzyloxyphenyl)propionaldehyde (17.7 g, 49.5 mmol) in 200 mL dry tetrahydrofuran was added dropwise such that temperature remained below -73° C. After addition was complete, the reaction mixture was allowed to slowly warm to 25° C where it was stirred for 18 hours. Reaction was quenched by the addition of methanol (40 mL) and concentrated under reduced pressure. The residue was taken up in ethyl acetate (200 mL) and 10% citric acid (200 mL), the organic phase was collected, and the aqueous phase was extracted with ethyl acetate (3 x 100 mL). The combined organics were washed with saturated $NaHCO_3$ (3 x 100 mL) and brine (1 x 100 mL), dried over anhydrous magnesium sulfate, filtered and concentrated to give 35 g of a yellow oil. The crude product was purified by low pressure chromatography (silica gel 230-400 mesh; 9 x 15 cm column; chloroform (97%):methanol (3%)) to yield Step A product (19.0 g, 90% yield). An analytical sample was obtained by recrystallization from diethyl ether:hexane, mp 113-115° C;

| elemental analysis, Calcd. for $C_{25}H_{31}NO_5$ (425.526): | | | | | | |
|---|---|---|---|---|---|---|
| | C = | 70.56; | H = | 7.34; | N = | 3.29. |
| Found: | C = | 70.45; | H = | 7.54; | N = | 3.30. |

Step B: Preparation of N-6(S)-[(1,1-Dimethylethoxycarbonyl)amino]-7-(4-benzyloxyphenyl)-1-hydroxy-(Z)-4-heptene

To a stirred solution of the product of Step A (18.8 g, 44 mmol) in 50 mL dry tetrahydrofuran under nitrogen was added triethylamine (9.2 mL, 66 mmol). The solution was cooled in an ice/methanol bath to -18° C and ethyl chloroformate (6.3 mL, 66 mmol) was added such that the temperature remained below -10° C to give a light yellow suspension. After warming to -2° C over 45 minutes, the mixture was filtered through a medium sintered glass funnel with cooling of the filtrate in an ice bath. To the filtrate at 0° C under nitrogen was added portionwise sodium borohydride (3.1 g, 82 mmol) followed by dropwise addition of methanol. After stirring at 0° C for 1 hour following addition, the reaction was quenched by the additionof water (50 mL) and allowed to warm to 25° C. The mixture was poured into 10% citric acid (200 mL) and extracted with diethyl ether (3 X 150 mL). The combined organics were washed with 5% NaOH (3 X 100 mL), water (1 X 100 mL), and brine (1 X 100 mL), dried over anhydrous magnesium sulfate, filtered, and concentrated. The crude product was purified by low pressure chromatography (silica gel 230-400 mesh; 9 x 15 cm column; chloroform (98%):methanol (2%)) to yield Step B product (14.5 g, 80% yield). An analytical sample was obtained by recrystallization from ethyl acetate:hexane, mp 94-97° C;

| elemental analysis Calcd. for $C_{25}H_{33}NO_4$ (411.542): | | | | | | |
|---|---|---|---|---|---|---|
| | C = | 72.96; | H = | 8.80; | N = | 3.40. |
| Found: | C = | 72.89; | H = | 8.05; | N = | 3.26. |

Step C: Preparation of N-6(S)-[1,1-Dimethylethoxycarbonyl)amino]-7-(4-benzyloxyphenyl)-1-methanesulfonyl-(Z)-4-heptene

To a stirred solution of the product of Step B (5.55 g, 13.4 mmol) in 100 mL dry methylene chloride cooled to 0°C in an ice/water bath and under nitrogen was added triethylamine (7.7 mL, 55 mmol) followed by methanesulfonyl chloride (2.1 mL, 27 mmol) dropwise such that temperature of the reaction remained below 2°C. The reaction was stirred at 0°C for 30 minutes then quenched by the addition of ice water (100 mL). After warming to 25°C, the organic phase was collected and the aqueous phase was extracted with methylene chloride (2 X 25 mL). The combined organics were washed with water (2 X 50 mL), 10% citric acid (2 X 50 mL), saturated $NaHCO_3$ (2 X 50 mL), and brine (1 X 50 mL), dried over anhydrous magnesium sulfate, filtered, and concentrated to give Step C product (6.55 g, quant. yield) as a white solid, mp 73-75°C.

Step D: Preparation of N-6(S)-[(1,1-Dimethylethoxycarbonyl)amino]-7-(4-benzyloxyphenyl)-1-iodo-(Z)-4-heptene

To a stirred solution of sodium iodide (19.9 g, 13.3 mmol) in 100 mL acetone under nitrogen was added a solution of the product of Step C (6.5 g, 13.3 mmol) in 130 mL acetone. The clear yellow solution was stirred at 25°C for 1 hour, heated under reflux for 1 hours, and stirred at 25°C for 1 hour. Upon concentration under reduced pressure, the resultant residue was taken up in water (50 mL) and extracted into ethyl acetate (3 X 50 mL). The combined organics were dried over anhydrous magnesium sulfate, filtered, and concentrated to give Step D product (6.7 g, 97% yield) as a light yellow solid. An analytical sample was obtained by recrystallization from diethyl ether, mp 83-84.5°C;

| elemental analysis Calcd. for $C_{25}H_{32}NO_3I$ (521.44): | | | | | | |
|---|---|---|---|---|---|---|
| | C = | 57.58; | H = | 6.18; | N = | 2.69. |
| Found: | C = | 57.61; | H = | 6.10; | N = | 2.67. |

Step E: Preparation of Ethyl N-8(S)-[(1,1-dimethylethoxycarbonyl)amino]-9-(4-benzyloxyphenyl)-2-ethoxycarbonyl-(Z)-6-nonenoate

To a stirred suspensionof sodium hydride (53.5 mmol, from 2.14 g, 60% in oil dispersion; rinsed with 3 X 30 mL dry hexanes) in 20 mL dry dimethylformamide cooled to 0°C in an ice/water bath and under nitrogen was added dropwise diethyl malonate (12.1 mL, 80 mmol). The mixture was stirred at 25°C for 30 minutes, then a solution of the product of Step D (6.57 g, 12.6 mmol) in 35 mL dry dimethylformamide was added rapidly via cannula. The reaction was heated at 80°C for 40 minutes, cooled to 25°C, and concentrated under high vacuum. The resultant residue was partitioned between ethyl acetate (200 mL) and 10% citric acid (100 mL), the organic phase was collected and the aqueous phase was extracted with ethyl acetate (3 X 50 mL). The combined organics were washed with water (2 X 100 mL), saturated $NaHCO_3$ (2 X 100 mL), and brine (1 X 100 mL), dried over anhydrous magnesium sulfate, filtered, and concentrated to give 16 g yellow oil. Crude product was purified by low pressure chromatography (silica gel 230-400 mesh; 9 X 15 cm column; hexanes (85%):ethyl acetate (15%)) to yield Step E product (6.95 g, 99% yield as an oily white solid. An analytical sample was obtained by recrystallization from diethyl ether:hexanes, mp 45-46°C;

| elemental analysis Calcd. for $C_{32}H_{43}NO_7$ (553.697): | | | | | | |
|---|---|---|---|---|---|---|
| | C = | 69.42; | H = | 7.83; | N = | 2.53. |
| Found: | C = | 69.40; | H = | 7.77; | N = | 2.46. |

Step F: Preparation of Ethyl N-8(S)-[(1,1-dimethylethoxycarbonyl)amino]-9-(4-benzyloxyphenyl)-2-ethoxycarbonyl-6(R),7(R)-epoxy-nonenoate

To a stirred solution of the product of Step E (6.60 g, 12 mmol) in 100 mL dry methylene chloride

cooled in an ice methanol bath to -16°C and under nitrogen was added in one portion 3-chloroperoxybenzoic acid (97%, 8.2 g, 47 mmol). Reaction mixture was slowly allowed to warm to 15°C over 6 hours then was concentrated to dryness. The residue was taken up in diethyl ether (400 mL) and washed with diluted $NaHCO_3$ (2 X 100 mL), diluted $Na_2SO_3$ (2 X 100 mL), diluted $NaHCO_3$ (2 X 100 mL), and brine (1 X 100 mL), dried over anhydrous magnesium sulfate, filtered, and concentrated. The crude product (as a mixture of isomers (threo:erythro = 5:2 by $^1$H-NMR)) was purified by low pressure chromatography (silica gel 230-400 mesh; 10 X 16 cm column; hexanes (70%):ethyl acetate (30%)) to yield the product of Step F (4.46 g, 65%), mp 49-51°C.

Step G: Preparation of 3(R)-Carboxyethyl-8(S)-[N-1(S)-((1,1-dimethylethoxycarbonyl)amino)-2-(4-benzyloxyphenyl)ethyl]-1-oxa-7(S)-bicyclo-[3.3.0]octane-2-one

To a stirred solution of Step F product, N-7(S)-[(1,1-dimethylethoxycarbonyl)amino]-8-(4-benzyloxyphenyl)-1,1-dicarbonxyethyl-5(R),6(R)-epoxy-octane (2.1 g, 3.7 mmol), in 100 mL dry tetrahydrofuran cooled to -20°C in an ice/methanol bath and under nitrogen was added dropwise a solution of lithium bis-(trimethylsilyl)amide in hexanes (7.4 mL, 1.0M, 7.4 mmol). The reaction was allowed to warm to -5°C over 5 minutes then was cooled to -10°C and a solutionof zinc chloride (7.4 mL, 1.0 M, 7.4 mL) was added. The clear solution was allowed to warm to 25°C where it was stirred for 64 hours. The reaction was poured into 10% citric acid (150 mL) and the mixture was extracted with diethyl ether (3 X 100 mL). The combined organics were washed with saturated $NaHCO_3$ (2 X 50 mL) and brine (1 X 50 mL), dried over anhydrous magnesium sulfate, filtered, and concentrated to give a clear oil. The crude product was purified by low pressure chromatography (silica gel 230-400 mesh; 3 X 16 cm column; methylene chloride (95%):ethyl acetate (5%)) to yield Step G product (1.4 g, 72% yield) as a clear foam, mp 41-43°C;

| elemental analysis Calcd. for $C_{30}H_{37}NO_7$ (523.627): | | | | | | |
|---|---|---|---|---|---|---|
| | C = | 68.81; | H = | 7.12; | N = | 2.67. |
| Found: | C = | 68.77; | H = | 7.13; | N = | 2.61. |

Step H: Preparation of 8(S)-[N-1(S)-((1,1-Dimethylethoxycarbonyl)amino)-2-(4-benzyloxyphenyl)-ethyl]-1-oxa-3(R),7(S)-bicyclo[3.3.0]octane-2-one

To a stirred solution of the product of Step G, (47 mg, 0.09 mmol) in 1:0 mL 1,2-dimethoxyethane under nitrogen was added 1.0 mL 1N LiOH (aq.). The clear solution was stirred for 4 hours then 13 mL 10% citric acid was added. The mixture was extracted with diethyl ether (3 X 20 mL) and the combined organics were washed with water (1 X 20 mL) and brine (1 X 20 mL), dried over anhydrous magnesium sulfate, filtered, diluted with 20 mL dry toluene, and concentrated to dryness. The crude acid was dissolved in 10 ml xylenes and heated at 160°C for 10 hours then concentrated under high vacuum. The crude product was purified by low pressure chromatography (silica gel 230-400 mesh; 3 X 16 cm column; hexanes (75%):ethyl acetate (25%)) to yield the title product of Step H, (20 mg, 49% yield), as a white solid, mp 134-137°C, (C;H;N).

Step I: Preparation of N-Benzyl-2(S)-[N-2(S)-((1,1-dimethylethoxycarbonyl)amino)-3-(4-benzyloxyphenyl)-2-(S)-hydroxy-1-propyl]-1(R)-cyclopentane carboxamide

To a stirred solution of benzylamine (0.05 mL, 0.45 mmol) in 3 mL dry tetrahydrofuran cooled to -78°C in a dry ice/isopropanol bath was added a solution of n-butyllithium in hexanes (0.20 mL, 1.6 M, 0.32 mmol) to give a red solution. After 15 minutes at -70°C a solution of the product of Step H (20 mg, 0.04 mmol) in 2 mL dry tetrahydrofuran was added. After 90 minutes at -70°C, the reaction was quenched by the additionof 10% citric acid (10 mL). The mixture was extracted with ethyl acetate (3 X 15 mL); the combined organics were washed with water (1 X 15 mL), saturated $NaHCO_3$ (1 X 15 mL), and brine (1 X 15 mL), dried over anhydrous magnesium sulfate, filtered, and concentrated. The crude product was purified by low pressure chromatography (silica gel 230-400 mesh, 5 X 16 cm column; acetonitrile (10%):methylene chloride (90%) to acetonitrile (20%):methylene chloride (80%)) followed by preparative thin layer

chromatography (E. Merck plate, silica gel F254, 0.5 mm; acetonitrile (20%):methylene chloride (80%)) to yield the Step I product (18 mg, 73% yield), (C;H;N).

Step J: Preparation of N-Benzyl-2(S)-[N-2(S)-((1,1-dimethylethoxycarbonyl)amino)-3-(4-hydroxyphenyl)-2(S)-hydroxy-1-propyl]-1(R)-cyclopentane carboxamide

The product of Step I, 15 mg, was dissolved in 3 mL ethanol and 3 mL tetrahydrofuran and to it was added 5% palladium on carbon, 20 mg. The mixture was stirred under hydrogen at atmospheric pressure for 20 hours, then filtered and concentrated to dryness. The oily residue was dissolved in 2 mL of ethanol and 10 mL of water was added. A white solid precipitated which was collected and dried. The yield was 12 mg of pure product (95% yield), (C;H;N).

EXAMPLE 30

Preparation of N-Benzyl-2(R)-[N-2(S)-((1,1-dimethylethoxycarbonyl)amino)-3-(4-hydroxyphenyl)-2(S)-hydroxy-1-propyl]-1(R)-cyclopentane carboxamide ("trans-Tyr-Pro")

Step A: Preparation of 4(S)-[(1,1-dimethyl-ethoxycarbonyl)amino]-3(S)-hydroxy-5-(4-benzyloxyphenyl)-1-pentene:

To a stirred solution of 80 mL of commercial 1 M zinc chloride in ether and 200 mL of dry THF cooled to 0° C was added dropwise 120 mL of commercial 1 M vinylmagnesium bromide in THF. The mixture was cooled to -30° C and a solution of 10.8 g (30 mmol) of N-2(S)-[(1,1-dimethylethoxycarbonyl)amino]-3-(4-benzyloxyphenyl)-propionaldehyde in 200 mL of dry THF was added slowly keeping the temperature at -30 ± 5° C. The reaction was allowed to warm to room temperature and stir for 90 minutes, then quenched by addition of 500 mL of 10% citric acid and extracted into 1 L of diethyl ether. The ether extracts were dried over $MgSO_4$, filtered and concentrated to give a white solid. Crude product was purified by low pressure chromatography (silica gel; 10%-50% ethyl acetate/$CH_2Cl_2$) to yield the 3(S) alcohol, 6.0 g (56% yield) as a white solid which was essentially homogenous by TLC (10% ethyl acetate/hexanes). Further elution with 50% ethyl acetate/hexanes afforded 0.77 g (7% yield) of the 3(R) alcohol.

Step B: Preparation of 4(S)-[1,1-dimethylethoxycarbonyl)amino]-3(S)-acetoxy-5-(4-benzyloxyphenyl)-1-pentene

The product of Step A, 6 g, was dissolved in 200 mL of $CH_2Cl_2$ and to it was added 3 mL of acetic anhydride, 4.4 mL of triethylamine and 25 mg of 4-dimethylaminopyridine. The mixture was allowed to stir at room temperature overnight, then diluted with 100 mL $CH_2Cl_2$ and washed with 1 X 20 mL 10% citric acid 1 X 200 mL $H_2O$, 1 X 200 mL sat'd $NaHCO_3$ and dried over $MgSO_4$. After filtration and removal of solvents under reduced pressure, the crude product was purified by low pressure chromatography (silica gel; 25% ethyl acetate/hexanes) to yield the pure acetate, 6.65 g (quant.), as an oil which was homogenous by TLC (25% ethylacetate/hexanes).

Step C: Preparation of N-6(S)-[(1,1-dimethylethoxycarbonyl)amino]-7-(4-benzyloxyphenyl)-(E)-4-heptenoic acid

The product of Step B, 7.43 g, was dissolved in 40 mL of dry THF and added dropwise to a solution of 62 mL of commercial 0.692 M potassium hexamethyldisilazide in toluene and 100 mL of dry THF cooled to -78° (±5)° C under nitrogen atmosphere. After 10 minutes at -78(±5)° C, a solution of 7.3 g of t-butyldimethylsilyl chloride in 30 mL of dry hexamethylphosphoramide was added. The mixture was allowed to warm to room temperature and stir for 15 hours, then diluted with 400 mL of ethyl acetate and washed with 2 X 100 mL 10% citric acid and 2 X 200 mL of $H_2O$. The aqueous layers were extracted with 1 X 100 mL of

ethyl acetate and the combined organic extracts were washed 2 X 200 mL of sat'd brine and dried over $MgSO_4$. After filtration and removal of solvents under reduced pressure the residue was dissolved in 20 mL of 1 M tetra-n-butylammonium fluoride in THF and allowed to stir under nitrogen for 24 hours at room temperature. The mixture was diluted with 50 mL of 10% citric acid and extracted with 3 X 100 mL of chloroform. The combined organic extracts were washed with 1 X 200 mL of water and dried over $MgSO_4$ then concentrated to dryness. The crude product was purified by low pressure chromatography (silica gel, 5% methanol/$CHCl_3$) and recrystallization from ethanol/water to yield 6.33 g (85% yield) of pure product: mp 121-2°C

| elemental analysis, Calc'd for $C_{25}H_{31}NO_5$ (425.52): | | | | | | |
|---|---|---|---|---|---|---|
| | C = | 70.57, | H = | 7.34, | N = | 3.29; |
| Found: | C = | 70.38, | H = | 7.22, | N = | 3.16. |

Step D thru Step G: Preparation of 8(S)-[N-1(S)-((1,1-dimethylethoxycarbonyl)amino)-2-(4-benzyloxyphenyl)-ethyl]-1-oxa-3(S),7(R)-bicyclo[3.3.0]-octane-2-one

Employing the procedure substantially as described in Example V, Steps C through F, but substituting for the N-6(S)-[(1,1-dimethylethoxy carbonyl)amino]-7-(4-benzyloxyphenyl)-(E)-heptenoic acid the product of Step C, 4.8 g, there was obtained in sequence the following:

(Step D)

N-6(S)-[(1,1-dimethylethoxycarbonyl)amino]-7-(4-benzyloxyphenyl)-1-hydroxy-(E)-heptene as a white solid. 4.4 g (quant. yield) which was essentially homogeneous by TLC (5% methanol/$CHCl_3$).

(Step E)

N-6(S)-[(1,1-dimethylethoxycarbonyl)amino]-7-(4-benzyloxyphenyl)-1-methanesulfonyl-(E)-4-heptene as a clear resin, 4.2 g (78% yield) which was essentially homogeneous by TLC (5% methanol/$CHCl_3$.

(Step F)

N-6(S)-[(1,1-dimethylethoxycarbonyl)amino]-7-(4-benzyloxyphenyl)-1-iodo-(E)-4-heptene as a colorless resin, 4.3 g (96% yield) which was essentially homogeneous by TLC (2% acetonitrile/$CH_2Cl_2$).

(Step G)

Ethyl N-8(S)-[(1,1-dimethylethoxycarbonyl)-amino]-9-(4-benzyloxyphenyl)-2-ethoxy-carbonyl-(E)-6-non-enoate as a waxy solid, 4.4 g (98% yield) which was essentially homogeneous by TLC (25% acelate/hexanes).

(Step H)

Ethyl N-8(S)-[(1,1-dimethylethoxycarbonyl)-amino]-9-(4-benzyloxyphenyl)-2-ethoxy-carbonyl-6(R), 7(S)-epoxynonenoate as an oil which crystallizes in the freezer (melts below 0°C), 3.48 g (77% yield). The product was essentially homogeneous by TLC (30% ethyl acetate/hexanes).

(Step I)

3-Ethoxycarbonyl-8(S)-[N-(1(S)-((1,1-dimethyl ethoxycarbonyl)amino)-2-(4-benzyloxyphenyl)ethyl]-1-oxa-7(S)-bicyclo[3.3.0]octane-2-one as a colorless foam, 3.2 g (quantitative yield) which was essentially homogeneous by TLC (5% ethyl acelate/$CH_2Cl_2$).

(Step J)

8(S)-[N-(1(S)-((1,1-dimethylethoxycarbonyl)amino)-2-(4-benzyloxyphenyl)ethyl]-1-oxa-3(S), 7(R)-bicyclo-[3.3.0]octane-2-one as a white solid (1.98 g, 72% yield): mp 157-159 °C,

| elemental analysis, Calc'd for $C_{27}H_{33}NO_5$ (451.567): | | | | | | |
|---|---|---|---|---|---|---|
| | C = | 71.71; | H = | 7.37; | N = | 3.10. |
| Found: | C = | 71.82; | H = | 7.31; | N = | 3.0. |

Step K: Preparation of 3-(1,1-dimethylethoxycarbonyl)-2,2-dimethyl-4(S)-(4-benzyloxyphenylmethyl)-5(S)-[2-(S)-hydroxymethyl-1(R)-cyclopentyl]oxazolidine:

The product of Step J, 1.0 g (2.2 mmol) was dissolved in 50 mL of 1,2-dimethoxyethane and to it was added 5.5 mL (11 mmol) of 2 M lithium borohydride in THF. The solution was allowed to stir for 8 hours at room temperature then quenched by dropwise addition of 10 mL of water, followed by 25 mL of 10% citric acid. The mixture was extracted with ethyl acetate (2 X 50 mL) and the organic extracts washed 50 mL saturated $NaHCO_3$ dried over $MgSO_4$ and concentrated to dryness. The oily residue (1.0 g) was dissolved in 5 mL of 2,2-dimethoxypropane and 15 mL of acetone and to it was added 16 mg of p-toluenesulfonic acid, monohydrate. The mixture was stirred under nitrogen atmosphere for 24 hours, then poured into 50 mL of saturated $NaHCO_3$ and extracted with 3 X 100 mL of ethyl acetate. The combined organic extract were dried over $MgSO_4$ and concentrated to dryness. The residue was purified by low pressure chromatography (silica gel, 3% ethyl acetate/hexanes) to give 1.05 g (95% yield) of a colorless foam which was essentially homogeneous by TLC.

Step L: Preparation of 3-(1,1-dimethylethoxycarbonyl)-2,2-dimethyl-4(S)-(4-benzyloxyphenylmethyl)-5(S)-[2-(R)-formyl-1(R)-cyclopentyl]oxazolidine:

The product of Step K, 1.05 g was dissolved in 50 mL of $CH_2Cl_2$ and added dropwise to a stirred solution of 0.20 mL at anhydrous DMSO and 0.20 mL of oxalyl chloride at -78 °C. After 20 minutes stirring at -78 °C, dry triethylamine, 0.70 mL was added slowly. After stirring for 40 minutes at -78 °C, the mixture was allowed to warm to room temperature and stir for 30 minutes. The mixture was diluted with 200 mL of $CH_2Cl_2$ and washed with 1 X 100 mL 10% citric acid, 1 X 100 mL $H_2O$, 1 X 50 mL saturated $NaHCO_3$ and dried over $MgSO_4$. After removal of solvents under reduced pressure, the residue, 1.1 g, was dissolved in 16 mL of absolute methanol and to it was added 0.276 g (2 mmol) powdered anhydrous $K_2CO_3$. After 2 hours at room temperature, the reaction was cooled to 0 °C and treated with 230 mL (4 mmol) of glacial acetic acid. After 5 minutes, the mixture was warmed to room temperature and 10 mL 1 M pH 7 phosphate buffer was added. After 30 minutes stirring, the mixture was concentrated and the aqueous phase extracted with 5 X 50 mL of ether. The combined ether extracts were dried over $MgSO_4$ and concentrated to dryness. The crude product was purified by low pressure chromatography (silica gel, 15% ethyl acetate/hexanes) to yield 0.92 g of the product (>10:1 of the 2(R):2(S) aldehydes) as a foam. The product obtained in this manner was essentially homogeneous by TLC (15% ethyl acetate/hexanes).

Step M: Preparation of 2(R)-[N-2(S)-((1,1-dimethylethoxycarbonyl)amino)-1(S)-hydroxy-3-(4-benzyloxyphenyl)-1-propyl]-1(R)-cyclopentanecarboxylic acid

The product of Step L, 0.25 g (0.5 mmol) was dissolved in 3 mL of tert-butanol and 2 mL of 1.25 M aqueous potassium phosphate buffer (pH = 6.8). To it was added 3.0 mL of aqueous 1 M $KMnO_4$ solution with stirring. After 5 minutes, the reaction was quenched by addition of solid $Na_2SO_3$ (3mL) and the pH

adjusted to 3 with ice cold 10% citric acid. The mixture was extracted into 5 X 50 mL of ethyl acetate and the combined organic extracts dried over $MgSO_4$ then concentrated to dryness. The residue was dissolved in methanol and 5 mg of p-toluenesulfonic acid was added. After stirring for 30 minutes at room temperature, 1 mL of aqueous 1 M sodium acetate was added and the mixture concentrated to dryness. The residue was dissolved in chloroform (100 mL), filtered and again concentrated to dryness. The product formed a solid on drying under vacuum (0.20 g, 87% yield) and was essentially homogeneous by TLC (1:3:26, AcOH: MeOH: $CHCl_3$).

Step N: Preparation of N-Benzyl-2(R)-[N-2(S)-((1,1-dimethylethoxycarbonyl)amino)-3-(4-benzyloxyphenyl)-1-(S)-hydroxy-1-propyl]-1(R)-cyclopentene carboxamide

The product from Step M, 0.20 g (0.43 mmol, was dissolved in 5 mL of dry DMF and to it was added 50 mL (0.45 mmol) of benzylamine, 58 mg (0.43 mmol) of 1-hydroxybenzotriazole hydrate and 86 mg (0.45 mmol) of dimethyl-3-(3-dimethylaminopropyl)carbodiimide hydrochloride. Triethylamine was added dropwise until the pH was 8.5. After stirring for 15 hours at room temperature, the reaction was concentrated to dryness under reduced pressure. The residue was dissolved in 100 mL of chloroform and washed with 1 X 50 mL of 10% citric acid, 1 X 50 mL $H_2O$, 1 X 50 mL saturated $NaHCO_3$ dried over $MgSO_4$, and concentrated to dryness. Crude product was purified by low pressure chromatography on silica gel eluting with 20% acetonitrile in methylene chloride to yield 150 mg of product as a foam which was essentially homogeneous by TLC (20% $CH_3CN/CH_2Cl_2$). The product formed a solid precipitate from ethanol/water.

Step O: Preparation of N-Benzyl-2(R)-[N-2(S)-((1,1-dimethylethoxycarbonyl)amino)-3-(4-hydroxyphenyl)-2-(S)-hydroxy-1-propyl]-1(R)-cyclopentane carboxamide

The product from Step N, 150 mg was dissolved in 25 mL of ethanol and 25% of THF containing 50 mg of 10% palladium on carbon. The mixture was stirred under hydrogen atmosphere for 24 hours, then filtered and concentrated to dryness. The residue was dissolved in 6 mL of ethanol and 30 mL of water was added. A white solid precipitated which was collected and dried. The yield was 120 mg of pure product (95%): (C,H,N).

EXAMPLE 31

Assay for Inhibition of Microbial Expressed Viral Protease

Additional compounds were tested by the assay of Example 9. The results show substantial inhibition of HIV protease for many of the compounds tested. The products of synthesis in Example 10-28 inclusive shown $IC_{50}$ values in the range of 0.1 - 10 nM.

While the foregoing specification teaches the principles of the present invention, with examples provided for the purpose of illustration, it will be understood that the practice of the invention encompasses all of the usual variations, adaptations, modifications, deletions, or additions of procedures and protocols described herein, as come within the scope of the following claims and its equivalents.

## Claims

1. Compounds of the formula:

A-G-B-B-J I,

wherein A is:

1) trityl,

2) hydrogen;

3)

$$R^1-\overset{O}{\overset{\|}{C}}-$$

wherein $R^1$ is

a) hydrogen,

b) $C_{1-4}$ alkyl, substituted with one or more halogens adjacent to the carbonyl carbon where halogen is F, Cl, Br, and I;

4) phthaloyl wherein the aromatic ring is unsubstituted or substituted with one or more of

a) $C_{1-4}$ alkyl,

b) halo,

c) hydroxy,

d) nitro,

e) $C_{1-3}$ alkoxy,

f) $C_{1-3}$ alkoxycarbonyl,

g) cyano,

h) $-\overset{O}{\overset{\|}{C}}-NR_2$ wherein R is H or $C_{1-4}$ alkyl;

5)

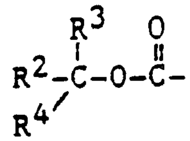

wherein $R^2$,$R^3$, and $R^4$ are independently

a) H,

b) $C_{1-6}$ alkyl unsubstituted or substituted with one or more of

i) halo,

ii) alkyl $SO_2$-,

iii) aryl $SO_2$-,

c) Aryl unsubstituted or substituted with one or more of

i) $C_{1-4}$ alkyl,

ii) $C_{1-3}$ alkoxy,

iii) halo,

iv) nitro,

v) acetoxy,

vi) dimethylaminocarbonyl,

vii) phenyl,

viii) $C_{1-3}$ alkoxycarbonyl

d) fluorenyl,

e) $R^2$, $R^3$, and $R^4$ may be independently joined to form a monocyclic, bicyclic, or tricyclic ring system which is $C_{3-10}$ cycloalkyl and may be substituted with $C_{1-4}$ alkyl, or

f) a 5-7 membered heterocycle;

6)

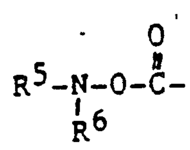

wherein $R^5$ and $R^6$ are

a) $C_{1-4}$ alkyl,

b) aryl,

c) $R^5$ and $R^6$ are joined to form a 5-7 membered heterocycle;

7)

$R^7-SO_2NH-\overset{O}{\overset{\|}{C}}-$ wherein $R^7$ is aryl unsubstituted or substituted with one or more of

a) $C_{1-4}$ alkyl,

b) halo,
c) nitro,
d) $C_{1-3}$ alkoxy;
8)

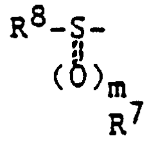

wherein m is 0-2 and $R^8$ is
a) $R^7$ as defined above,
b) trityl;
9)
$(R^7)_2\overset{X}{\overset{\|}{P}}$ - wherein X is O or S, or NH, and $R^7$ is defined above;
G is

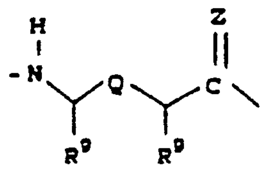 or 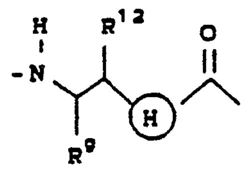 ,

wherein Z is O, S, or HH and
$R^9$ is independently
1) hydrogen;

2) 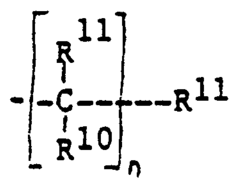

3) -OR, wherein R is H, or $C_{1-4}$ alkyl
4) $-NR_2$,
5) $C_{1-4}$ alkylene-$R^{11}$;
wherein n is 0-5 and $R^{10}$ is independently
a) hydrogen,
b) hydroxy, or
c) $C_{1-4}$-alkyl;
$R^{11}$ is
a) hydrogen,
b) aryl, unsubstituted or substituted with one or more of
i) halo,
ii) hydroxy,
iii) $-NH_2$, $-NO_2$, -NHR, or $-NR_2$, wherein R is H, or $C_{1-4}$ alkyl,
iv) $C_{1-4}$ alkyl,
v) $C_{1-3}$ Alkoxy,
vi) -COOR
vii) $-\underset{\underset{O}{\|}}{C}NR_2$,
viii) $-CH_2NR_2$,
ix)

$-CH_2NH\overset{O}{\overset{\|}{C}}R$,
x) -CN,
xi) $-CF_3$,
xii)
$-NH\overset{O}{\overset{\|}{C}}R$,
xiii) aryl $C_{1-3}$ alkoxy or aryl $C_{1-4}$ alkyl,
xiv) aryl,
xv) $-NRSO_2R$,
xvi) $-OP(O)(OR_x)_2$ wherein $R_x$ is H or aryl, or
xvii)
$-O-\overset{O}{\overset{\|}{C}}-C_{1-4}$alkyl substituted with one or more of amine or quaternary amine;

c) 5 or 6 membered heterocycle including up to 3 heteroatoms selected from N, O, and S, any of which heterocycle may be unsubstituted or substituted with one or more of
i) halo,
ii) hydroxy,
iii) $-NH_2$, -NHR, $-NR_2$;
iv) $C_{1-4}$ alkyl,
v) $C_{1-3}$ alkoxy,
vi) -COOR,
vii)
$-\overset{O}{\overset{\|}{C}}NR_2$,
viii) $-CH_2NR_2$,
ix)
$-NH\overset{O}{\overset{\|}{C}}R$,
x) -CN,
xi) $CF_3$,
xii) $-NHSO_2R$,
xiii) $-OP(O)(OR_x)_2$ wherein $R_x$ is H or aryl, or
xiv)
$-O-\overset{O}{\overset{\|}{C}}-C_{1-4}$alkyl substituted with one or more of amine or quaternary amine;

d) $C_{1-6}$ alkyl or $C_{1-6}$ alkenyl, unsubstituted or substituted with one or more of
i) hydroxy,
ii) $C_{1-4}$ alkyl,
iii) $-NH_2$, -NHR, $-NR_2$,
iv)
$-NH\overset{NH}{\overset{\|}{C}}H$ ,
v)
$-NH-\overset{NH}{\overset{\|}{C}}-NH_2$,
vi) -COOH,
vii)
$-\overset{O}{\overset{\|}{C}}OR$,
viii) -SR, or arylthio,
ix) $-SO_2NHR$,
x) $C_{1-4}$ alkyl sulfonylamino or aryl sulfonyl amino,
xi) -CONHR,
xii)
$-NH\overset{O}{\overset{\|}{C}}R$,
xiii) -OR,
xiv) aryl $C_{1-3}$alkoxy, or,

xv) aryl;
e) $C_{3-7}$ cycloalkyl unsubstituted or substituted with one or more of
i) hydroxy,
ii) $C_{1-4}$ alkyl,
iii) $-NH_2$, -NHR, $-NHR_2$,
iv)
$$-NH-\overset{NH}{\overset{\|}{C}}H ,$$
v)
$$-NH-\overset{NH}{\overset{\|}{C}}-NH_2,$$
vi) -COOH,
vii)
$$-\overset{O}{\overset{\|}{C}}-OR,$$
viii) -SR,
ix) $-SO_2NH_2$,
x) alkyl sulfonylamino or aryl sulfonylamino,
xi) -CONHR, or
xii)
$$-NH\overset{O}{\overset{\|}{C}}R;$$
f) a 5- to 7-membered carbocyclic or 7- to 10-membered bicyclic carbocyclic ring which is either saturated or unsaturated, said carbocyclic ring being unsubstituted or substituted with one or more of
i) halo,
ii) -OR, wherein R is H or $C_{1-4}$ alkyl,
iii)
$$-\overset{O}{\overset{\|}{C}}OR,$$
iv)
$$-\overset{O}{\overset{\|}{C}}NR_2,$$
v) $-CH_2NR_2$,
vi) $-SO_2NR_2$: $-S(O)_yR$ and $y$ = 0. 1 or 2;
vii) $-NR_2$.
viii)
$$-NH\overset{O}{\overset{\|}{C}}R,$$
ix) $C_{1-4}$ alkyl,
x) phenyl,
xi) $-CF_3$, or
xii)
$$-\overset{R}{\overset{|}{N}}-SO_2R;$$
g) benzofuryl, indolyl; azabicyclo $C_{7-11}$ cycloalkyl; or benzopiperidinyl;
$R^{12}$ is -OH or $-NHR^{13}$ wherein $R^{13}$ is -H,
$$-\overset{O}{\overset{\|}{C}}H,$$ $C_{1-4}$ alkyl, or -COOR; and
Ⓗ is
1) $C_{3-7}$ cycloalkyl either unsubstituted or substituted with one or more of
a) $C_{1-4}$ alkyl,
b) hydroxy,
c) $-NR_2$,
d) -COOR,
e) -CONHR,
f) $-NHSO_2R$,
g)

$-NH\overset{O}{\overset{\|}{C}}R$,
h) aryl,
i) aryl substituted with $C_{1-4}$alkyl,
j) heterocycle, or
k) heterocycle substituted with $C_{1-4}$alkyl;
2) phenyl either unsubstituted or substituted with one or more of
a) hydroxy,
b) -OR,
c) $-NHR^{13}$,
d) -COOR,
e)
$-\overset{O}{\overset{\|}{C}}NR_2$, or,
f)
$-NH\overset{O}{\overset{\|}{C}}R$;
3) 5 to 7-membered heterocycle, any of which heterocycle may be unsubstituted or substituted with one or more of
i) halo,
ii) hydroxy,
iii) $NR_2$, or,
iv) $C_{1-4}$ alkyl;
Q is

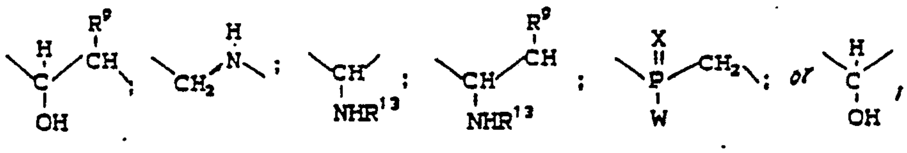
; ; ; ; ; or ,

wherein $R^9$ and $R^{13}$ are defined above;
X is O, S, or NH; and
W is
1) OH,
2) $NH_2$,
3) OR, or
4) NHR;
B is, independently, absent, or

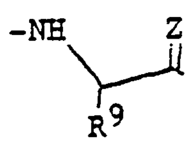
;

J is
1) $YR^{14}$ wherein:
Y is 0 or NH, and
$R^{14}$ is
a) H;
b) $C_{1-6}$ alkyl, unsubstituted or substituted with one or more of
i) $-NR_2$,
ii) -OR,
iii) $-NHSO_2C_{1-4}$ alkyl,
iv) $-NHSO_2$ aryl, or $-NHSO_2$-(dialkylaminoaryl),
v) $-CH_2OR$,
vi) $-C_{1-4}$ alkyl,
vii)

$-\overset{O}{\overset{\|}{C}}OR$,

viii) $-\overset{O}{\overset{\|}{C}}NR_2$,

ix) 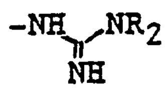, or 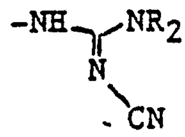 )

x) $-NH\overset{O}{\overset{\|}{C}}R$,

xi) 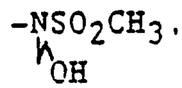,

xii) 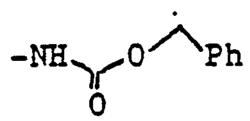,

xiii) $-NR_3^{\oplus}A^{\ominus}$ wherein $A^{\ominus}$ is a counterion,
xiv) $-NR^{15}R^{16}$ wherein $R^{15}$ and $R^{16}$ are the same or different and are $C_{1-5}$ alkyl joined together directly to form a 5-7 membered heterocycle,
xv) aryl,
xvi) -CHO,
xvii) $-OP(O)(OR_x)_2$ wherein $R_x$ is H or aryl, or
xviii) $-O-\overset{O}{\overset{\|}{C}}-C_{1-4}$alkyl substituted with one or more of amine or quaternary amine;
c) $-(CH_2CH_2O)_nCH_3$ or $-(CH_2CH_2O)_nH$;
2) $N(R^{14})_2$; or
3) $-NR^{15}R^{16}$ wherein $R^{15}$ and $R^{16}$ are defined above;

4) 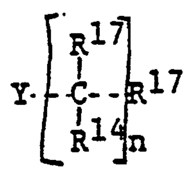

wherein:
Y, $R^{14}$ and n are defined above, and
$R^{17}$ is a) hydrogen;
b) aryl unsubstituted or substituted with one or more of
i) halo,
ii) -OR, wherein R is H or $C_{1-4}$ alkyl
iii) $-\overset{O}{\overset{\|}{C}}OR$,
iv) $-\overset{O}{\overset{\|}{C}}NR_2$,
v) $-CH_2NR_2$,
vi) $-SO_2NR_2$,
vii) $-NR_2^2$,
viii) $-NH\overset{O}{\overset{\|}{C}}R$,

xi) $C_{1-4}$ alkyl,
x) phenyl
xi) $-CF_3$,
xii)
R
|
- N $-SO_2R$,
xiii) $-C_{1-4}$ alkyl $-NR_2$,
xiv) $-OP(O)(OR_x)_2$ wherein $R_x$ is H or aryl, or
xv)
O
||
-O- C $-C_{1-4}$alkyl substituted with one or more of amine or quaternary amine;
c) Heterocycle as defined below,
unsubstituted or substituted with one or more of
i) halo,
ii) -OR, wherein R is H, $C_{1-4}$ alkyl, or $C_{1-4}$alkenyl,
iii)
O
||
- C OR,
iv)
O
||
- C $NR_2$,
v) $-CH_2NR_2$,
vi) $-SO_2NR_2$,
vii) $-NR_2$,
viii)
O
||
-NH C R,
xi) $C_{1-4}$ alkyl,
x) phenyl
xi) $-CF_3$,

R
\
xii) $-NSO_2R$,

xiii) phenyl $C_{1-4}$ alkyl,
xiv) $-OP(O)(OR_x)_2$ wherein $R_x$ is H or aryl, or
xv)
O
||
-O- C $-C_{1-4}$alkyl substituted with one or more of amine or quaternary amine;
d) A 5 to 7 membered carbocyclic or 7-10 membered bicyclic ring which is either saturated or unsaturated, said carbocyclic ring unsubstituted or substituted with one or more of
i) halo,
ii) -OR, wherein R is H or $C_{1-4}$ alkyl,
iii)
O
||
- C OR,
iv)
O
||
- $CNR_2$,
v) $-CH_2NR_2$,
vi) $-SO_2NR_2$,
vii) $-NR_2$,
viii)
O
||
-NH C R,
xi) $C_{1-4}$ alkyl,

x) phenyl
xi) $-CF_3$,
xii)
R
|
$- N SO_2R$,
xiii)$-OP(O)(OR_x)_2$ wherein $R_x$ is H or aryl, or
xiv)
O
||
$-O-C-C_{1-4}$alkyl substituted with one or more of amine or quaternary amine; or pharmaceutically acceptable salts thereof.

2. The compounds of Claim 1 wherein B is independently present twice and Z is O.

3. The compounds of Claim 2 wherein J is $NH_2$ and Q is

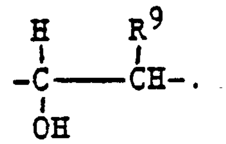

4. The compounds of Claim 2 wherein J is $NH_2$ and Q is

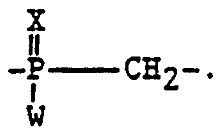

5. The compounds of Claim 2 wherein J is $NH_2$ and Q is

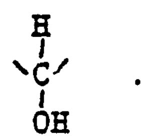

6. The compounds of Claim 1 wherein B is present once and Z is 0.

7. The compounds of Claim 6 wherein Q is

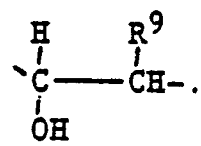

8. The compounds of Claim 6 wherein Q is

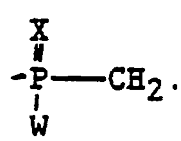

9. The compounds of Claim 6 wherein Q is

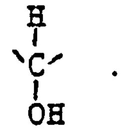

10. The compounds of Claim 1 wherein B is always absent.

11. The compounds of Claim 10 wherein Q is

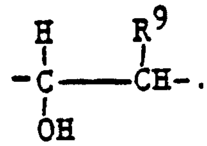

12. The compounds of Claim 10 wherein Q is

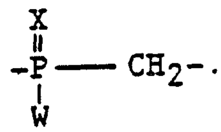

13. The compounds of Claim 10 wherein Q is 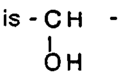

14. The compounds of claim 1 wherein G is

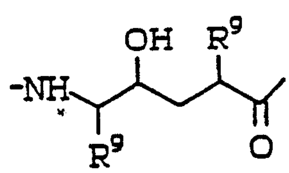 or 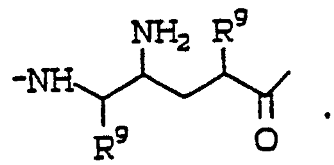

15. The compounds of Claim 14 wherein B is absent or present once.

16. The compounds of claim 15 wherein J is

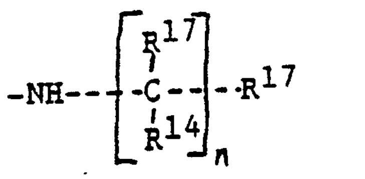

17. The compounds of claim 16 wherein A is

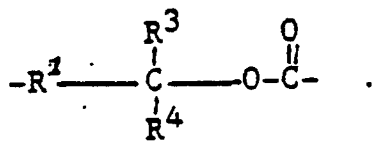

18. N′-(1,1-dimethylethoxycarbonyl)-5(S)-amino-4(S)-hydroxy-6-phenyl-2(R)-(phenylmethyl)hexanoyl-leucyl-phenylalanylamide, or pharmaceutically acceptable salts thereof.

19. N-Benzyl-N′-(1,1-dimethylethoxy carbonyl)-5(S)-amino-4(S)-hydroxy-6-phenyl-2(R)-(phenylmethyl)-hexanoyl-leucyl-amide, or pharmaceutically acceptable salts thereof.

20. N-benzyl-N′-(1,1-dimethylethoxycarbonyl)-5(S)-amino-4(S)-hydroxy-6-phenyl-2(R)-(phenylmethyl)-hexanoic carboxamide, or pharmaceutically acceptable salts thereof.

21. N′-(1,1-Dimethylethoxycarbonyl)-5(S)-amino-4(S)-hydroxy-6-cyclohexyl-2(R)-(phenylmethyl)-hexanoyl-leucyl-phenylalanylamide, or pharmaceutically acceptable salts thereof.

22. N′-(1,1-Dimethylethoxycarbonyl)-5(S)-amino-4(S)-hydroxy-6-phenyl-2(R)-(phenylmethyl)- hexanoyl-leucyl-phenylalanine methyl ester, or pharmaceutically acceptable salts thereof.

23. N′-(1,1-Dimethylethoxycarbonyl)-5(S)-amino-4(S)-hydroxy-6-phenyl-2(R)-(phenylmethyl)hexanoyl-isoleucylamide, or pharmaceutically acceptable salts thereof.

24. N-(2-(Methanesulfonylamino)ethyl)-N′-(1,1-dimethylethoxycarbonyl)-5(S)-amino-4(S)-hydroxy-6-phenyl-2(R)-(phenylmethyl)hexanoyl-leucylamide, or pharmaceutically acceptable salts thereof.

25. N′-(1,1-Dimethylethoxycarbonyl)-5(S)-amino-4(S)-hydroxy-6-phenyl-2(R)-(phenylmethyl)hexanoyl-leucyl-phenylalanylamide,
N-Benzyl-N′(1,1-Dimethylethoxycarbonyl)-5(S)-amino-4(S)-hydroxy-6-phenyl-2(R)-(phenylmethyl)hexanoyl-leucyl-amide,
5(S)-[(1,1-Dimethylethoxycarbonyl)-amino]-4(S)-hydroxy-6-phenyl-2(R)-(phenylmethyl)hexanoyl-Leu-amide,
N′-(1,1-Dimethylethoxycarbonyl)-5(S)-amino-4(S)-hydroxy-6-cyclohexyl-2(R)-(phenylmethyl)hexanoyl-leucyl-phenylalanylamide,
5(S)-[(1,1-Dimethylethoxycarbonyl)amino]-4(S)-hydroxy-6-phenyl-2(R)(phenylmethyl)hexanoyl Leu-(4-I-Phe)-amide,
5(S)-[(Phenylmethyloxycarbonyl)amino]-4(S)-hydroxy-6-phenyl-2(R)(phenylmethyl)hexanoyl Leu-Phe amide,
5(S)-[(1,1-dimethylethoxycarbonyl)amino]-4(S)-hydroxy-2(R)-n-butyl-6-phenylhexanoyl Leu-Phe-amide,
3-(1(S)-Benzyloxycarbonylamino-2-phenylethyl phosphinyl)-2(S,R)-(phenylmethyl)propanoyl-L-Leu-Phe-amide,
N-[4-[(Benzyloxycarbonyl)amino]butyl]-5(S)-[(1,1-dimethylethoxy)carbonylamino]-4(S)-hydroxy-6-phenyl-2-(R)-(phenylmethyl)hexanoyl-Leu-amide,
N′-(1,1-Dimethylethoxycarbonyl)-5(S)-amino-4(S)-hydroxy-6-phenyl-2(R)-(phenylmethyl)-hexanoyl-leucyl-phenylalanine methyl ester,
5(S)-[(1,1-Dimethylethoxycarbonyl)-amino]-4(S)-hydroxy-6-phenyl-2(R)-(2-naphthylmethyl)hexanoyl Leucine-Phenylalanine amide,
5(S)-[(1,1-Dimethylethoxycarbonyl)-amino]-4(S)-hydroxy-2(R)-(phenylmethyl)nonanoyl-Leucyl-Phenylalanyl amide,
5(S)-[(1,1-Dimethylethoxycarbonyl)-amino]-4(S)-hydroxy-6-phenyl-2(R)-(phenylmethyl)-hexanoyl-N-(2-hydroxy-1(S)-(phenylmethyl)ethyl)-Leu amide,
5(S)-[(1,1-dimethylethoxycarbonyl)-amino]-4(S)-hydroxy-6-phenyl-2(R)-(phenylmethyl)-hexanoyl-Ala amide,
N-(2-(Methanesulfonylamino)ethyl)-N′-(1,1-dimethylethoxycarbonyl)-5(S)-amino-4(S)-hydroxy-6-phenyl-2(R)-(phenylmethyl)hexanoyl-leucylamide,
5(S)-[(1,1-Dimethylethoxycarbonyl)-amino]-4(S)-hydroxy-6-phenyl-2(R)-(phenylmethyl)hexanoyl-Ile amide,
5(S)-[(1,1-Dimethylethoxycarbonyl)-amino]-4(S)-hydroxy-6-phenyl-2(R)-[(4-benzyloxyphenyl)methyl]-hexanoyl-Leu-Phe amide,
5(S)-[(1,1-Dimethylethoxycarbonyl)-amino]-4(S)-hydroxy-6-phenyl-2(R)-(phenylmethyl)hexanoyl-Ile-Val-amide,
5(S)-[(1,1-Dimethylethoxycarbonyl)-amino]-4(S)-hydroxy-6-phenyl-2(R)-[(4-hydroxyphenyl)methyl]hexanoyl-Leu-Phe amide,
5(S)-[(1,1-Dimethylethoxycarbonyl)-amino]-4(S)-hydroxy-6-phenyl-2(R)-(methyl)hexanoyl-Leu-Phe-amide
5(S)-[(1,1-Dimethylethoxycarbonyl)-amino]-4(S)-hydroxy-6-phenyl-2(R)-(phenylmethyl)-hexanoyl-N-(2-hydroxy-1(S)-phenylethyl)Leu amide,
5(S)-[(1,1-Dimethylethoxycarbonyl)-amino]-4(S)-hydroxy-6-phenyl-2(R)-(phenylmethyl)-hexanoyl-N-(2-acetamidoethyl)Leu amide,
5(S)-[(1,1-Dimethylethoxycarbonyl)-amino]-4(S)-hydroxy-6-phenyl-2(R)-(3′phenyl-prop-2′-en-1′-yl)-leucine-phenylalanine-amide,
5(S)-[(1,1-Dimethylethoxycarbonyl)-amino]-4(S)-hydroxy-6-phenyl-2(R)-(phenylmethyl)-hexanoyl-N-(2-hydroxyethyl)Leu amide,
5(S)-[(1,1-Dimethylethoxycarbonyl)-amino]-4(S)-hydroxy-6-phenyl-2(R)-(phenylmethyl)hexanoyl-Ile-benzylamide,
5(S)-[(1,1-Dimethylethoxycarbonyl)amino]-4(S)-hydroxy-6-phenyl-2(R)-(phenylmethyl)hexanoyl-phenylglycine amide,
4(S)-[(1,1-Dimethylethoxycarbonyl)amino]-3(S)-hydroxy-6-methylheptanoyl-glutamyl-phenylalanine amide,
5(S)-[(1,1-Dimethylethoxycarbonyl)amino]-4(S)-hydroxy-6-phenyl-2(R)-(phenylmethyl)hexanoyl-N-(4-dimethylaminopropyl)-Ile-amide,
5(S)-[(1,1-Dimethylethoxycarbonyl)amino]-4(S)-hydroxy-6-phenyl-2(R)-(4′-phenyl-phenylmethyl)leucine-phenylalanine amide,
5(S)-[(1,1-Dimethylethoxycarbonyl)amino]-4(S)-hydroxy-6-phenyl-2(R)-(phenylmethyl)hexanoyl valine amide,
5(S)-[(1,1-Dimethylethoxycarbonyl)amino]-4(S)-hydroxy-6-phenyl-2(R)-(phenylmethyl)hexanoyl-Leu-Phe-benzyl-ester,
5(S)-[(1,1-Dimethylethoxycarbonyl)amino]-4(S)-hydroxy-6-phenyl-2(R)-(4′-phenyl-phenylmethyl)-isoleucine-N-benzyl amide,
5(S)-[(1,1-Dimethylethoxycarbonyl)amino]-4(S)-hydroxy-6-(2-naphthyl)-2(R)-(phenylmethyl)hexanoyl-Leu-Phe amide,
5(S)-[(1,1-Dimethylethoxycarbonyl)amino]-4(S)-hydroxy-2(R),6-diphenylhexanoyl-Leu-Phe-amide,
5(S)-[(1,1-Dimethylethoxycarbonyl)amino]-4(S)-hydroxy-6-phenyl-2(R)-(phenylmethyl)hexanoyl-N-(2-dimethylamino ethyl)-Ile amide,
5(S)-[(1,1-Dimethylethoxycarbonyl)amino]-4(S)-hydroxy-6-phenyl-2(R)-(phenylmethyl)hexanoyl-N-(2-pyridylmethyl)Ile amide,
5(S)-[(1,1-Dimethylethoxycarbonyl)amino]-4(S)-hydroxy-6-(2-naphthyl)-2(R)-(phenylmethyl)hexanoyl-Ile-benzylamide,
5(S)-[(1,1-Dimethylethoxycarbonyl)amino]-4(S)-hydroxy-6-phenyl-2(R)-(phenylmethyl)-hexanoyl-Ile-Phe amide,
N-(Methyl-5-amino-5-deoxy-2,3,0-isopropylidene-β-D-ribosyl)-5(S)-(1,1-dimethylethoxycarbonylamino)-4(S)-hydroxy-6-phenyl-2(R)(phenylmethyl)-hexanoyl Ile amide,
5(S)-[(1,1-Dimethylethoxycarbonyl)amino]-4(S)-hydroxy-6-phenyl-2(R)-(phenylmethyl)hexanoyl-S-neopentylglycine amide,
N-(2(S,R)-Hydroxy-1(R,S)-indanyl)-5(S)-(1,1-dimethylethoxycarbonylamino)-4(S)-hydroxy-6-phenyl-2(R)-phenylmethylhexanamide,
5(S)-(1,1-Dimethylethoxycarbonylamino)-4(S)-hydroxy-6-phenyl-2(R)-(3′-phenylpropyl)hexanoyl-leucine-phenylalanine amide,
5(S)-(1,1-Dimethylethoxycarbonylamino)-4(S)-hydroxy-6-phenyl-2(R)-(phenylmethyl)hexanoyl-N-(4-dimethylamino propyl)-Val amide,
N-(Methyl-5-amino-5-deoxy-β-D-ribosyl)-5(S)-(1,1-dimethylethoxycarbonylamino)-4(S)-hydroxy-6-phenyl-2-(R)-(phenylmethyl)hexanoyl-Ile amide,
5(S)-[(1,1-Dimethylethoxycarbonyl)amino]-4(S)-hydroxy-6-phenyl-2(R)-(phenylmethyl)hexanoyl-S-tert-butylglycine-benzylamide,
5(S)-[(1,1-Dimethylethoxycarbonyl)amino]-4(S)-hydroxy-6-phenyl-2(R)-(phenylmethyl)hexanoyl-N-(2,3-dihydroxy-propyl)-Ile amide,
5(S)-[(1,1-Dimethylethoxycarbonyl)amino]-4(S)-hydroxy-6-phenyl-2(R)-(phenylmethyl)hexanoyl-cyclohexylglycine-amide,
5(S)-[(1,1-Dimethylethoxycarbonyl)amino]-4(S)-hydroxy-6-phenyl-2(R)-(3′phenylprop-2′-en-l′yl)hexanoyl-N-(3-dimethyl amino propyl) valine amide,
N′-(1,1-Dimethylethoxycarbonyl)-5(S)-amino-4(S)-hydroxy-6-phenyl-2(R)-(3′-phenylprop-2′-en-1′-yl)-hexanoyl-(S)-phenylglycyl-(2-hydroxyethyl)amide,
5(S)-[1,1-Dimethylethoxycarbonylamino]-4(S)-hydroxy-6-phenyl-2(R)-(phenylmethyl)hexanoyl-N-(phenylmethyl)-valine amide,
N-(cis-2(S,R)Hydroxy-1(R,S)-indanyl)-5(S)-(1,1-dimethylethoxycarbonylamino)-4(S)-hydroxy-6-(4-benzyloxyphenylmethyl)-2(R)-(phenylmethyl)hexanamide,
5(S)-(1,1-Dimethylethoxycarbonylamino)-4(S)-hydroxy-6-phenyl-2(R)-(phenylmethyl)-hexanoyl-N-[2-(imidazol-4-yl)-1-(hydroxymethyl)-propyl]-Ile-amide,
4(S)-[(1,1-Dimethylethoxycarbonyl)amino]-3(S)-hydroxy-5-cyclohexylpentanoyl-glutamyl-phenylalanine amide,
5(S)-[(1,1-Dimethylethoxycarbonyl)amino]-4(S)-hydroxy-6-phenyl-2(R)-(3′phenylprop-2′-en-1′-yl)-hexanoyl --(S)-phenylglycine amide,
5(S)-[(1,1-Dimethylethoxycarbonyl)amino]-4(S)-hydroxy-6-phenyl-2(R)-(4-nitrophenylmethyl)-phenylglycine amide,
5(S)-[1,1-Dimethylethoxycarbonylamino]-4(S)-hydroxy-6-phenyl-2(R)-(phenylmethyl)hexanoyl-phenylglycine amide,
N-(2(S or R)-hydroxy-1,2,3,4-tetrahydro-1(R or S)-naphthyl)-5(S)-(1,1-dimethylethoxycarbonylamino)-4(S)-hydroxy-6-phenyl-2(R)-phenylmethyl hexanamide,
N-(2(R or S)-hydroxy-1,2,3,4-tetrahydro-1(S or R)-naphthyl)-5(S)-(1,1-dimethylethoxycarbonylamino)-4(S)-hydroxy-6-phenyl-2(R)-phenylmethyl hexanamide,
N-(cis-2(S,R)-Hydroxy-1(R,S)-indanyl)-5(S)-(1,1-dimethylethoxycarbonylamino)-4(S)-hydroxy-6-(4-hydroxyphenyl)-2(R)-phenylmethylhexanamide,
5(S)-(1,1-Dimethylethoxycarbonyl)amino-4(S)-hydroxy-6-phenyl-2(R)-(phenylmethyl)hexanoyl-N-(2,3-dihydroxypropyl)-Val-amide,
N-(cis-2(R)-Hydroxy-1(S)-indanyl)-5(S)-(1,1-dimethylethoxycarbonylamino)-4(S)-hydroxy-6-phenyl-2(R)-phenylmethyl-hexanamide,
5(S)-(1,1-Dimethylethoxycarbonylamino)-4(S)-hydroxy-3(S or R)-hydroxy-6-phenyl-2(R)-phenylmethyl-hexanoyl-Leu-Phe-amide,
5(S)-(1,1-Dimethylethoxycarbonylamino)-4(S)-hydroxy-6-phenyl-2(R)-(3′-phenylprop-2-en-1′-yl)-hexanoyl-N-(2(R,S),3-dihydroxypropyl)-phenylglycine amide,
5(S)-[(1,1-Dimethylethoxycarbonyl)amino]-4(S)-hydroxy-6-phenyl-2(R)-(phenylmethyl)hexanoyl-N-(2-benzimidazolyl methyl)Ile amide
5(S)-(1,1-Dimethylethoxycarbonylamino)-4(S)-hydroxy-6-phenyl-2(R)-(phenylmethyl)hexanoyl-N-(benzyl)-allo-Ile amide,
N-(Methyl-5-amino-5-deoxy-β-D-ribosyl)-5(S)-(1,1-dimethylethoxycarbonylamino)-4(S)-hydroxy-6-phenyl-2-(R)-phenylmethylhexanoyl-Val amide,
N-(Methyl-5-amino-5-deoxy-2,3,0-isoproplidene-β-D-ribosyl)-5(S)-(1,1-dimethylethoxycarbonylamino)-4(S)-hydroxy-6-phenyl-2(R)-(phenylmethyl)hexanoyl-Val amide,
5(S)-(1,1-Dimethylethoxycarbonylamino)-4(S)-hydroxy-6-phenyl-2(R)(phenylmethyl)hexanoyl-N-[2-(2-methoxyethoxy)ethyl]isoleucine amide,
5(S)-(1,1-Dimethylethoxycarbonylamino)-4(S)-hydroxy-6-phenyl-2(R)-(3′phenylpropyl)hexanoyl-N-(2″(R,S),3″-dihydroxypropyl)-phenylglycine amide,
[4(S)-(1,1-Dimethylethoxycarbonylamino)-3(S)-hydroxy-5-phenylpentanoyl]-S-glutamyl-S-phenylalanine amide,
5(S)-[(1,1-Dimethylethoxycarbonyl)amino]-4(S)-hydroxy-6-phenyl-2(R)-(phenylmethyl)hexanoyl-N-[2-(4-imidazolyl)ethyl]valine amide,
N-(cis-2(R)-Hydroxy-1(S)-indanyl)-5-(S)-(1,1-dimethylethoxycarbonylamino)-4(S)-hydroxy-6-phenyl-2(R)-(3′-phenylprop-2′-en-1′-yl)hexanamide,
N-(1(R or S), 2(S or R)-Dihydroxy-3(S or R)-indanyl)-5(S)-(1,1-dimethylethoxycarbonylamino)-4(S)-hydroxy-6-phenyl-2(R)-(phenylmethyl)hexanamide,
N-[2(R)-Hydroxy-1(S)-indanyl]-5(S)-[(1,1-dimethylethoxycarbonyl)amino]-4(S)-hydroxy-6-(4-hydroxyphenyl)-2(R)-(phenylmethyl) hexanamide,
5(S)-[(1,1-Dimethylethoxycarbonyl)amino]-4(S)-hydroxy-6-phenyl-2(R)-((4-iodophenyl)methyl)-hexanoyl valine amide,
N-(cis-2(R)-Hydroxy-1(S)-indanyl)-5(S)-((1,1-dimethylethoxycarbonyl)amino)-4(S)-hydroxy-6-phenyl-2(R)-((4-iodophenyl)methyl)hexanamide,
5(S)-[(1,1-Dimethylethoxycarbonyl)amino]-4(S)-hydroxy-6-phenyl-2(R)-(phenylmethyl)hexanoyl-N-(2-(1-piperidinyl)ethyl)valine amide,
5(S)-[(1,1-Dimethylethoxycarbonyl)amino]-4(S)-hydroxy-6-phenyl-2(R)-(phenylmethyl)hexanoyl-N-(2-hydroxyethyl)valine amide,
5(S)-[(1,1-Dimethylethoxycarbonyl)amino]-2(R)-phenylmethyl-4(S)-hydroxy-6-phenyl-hexanoyl-N-(2-[(1-methoxyethoxy)-ethoxy]ethyl)isoleucine amide,
5(S)-(1,1-Dimethylethoxycarbonylamino)-2(R)-phenylmethyl-4(S)-hydroxy-6-phenylhexanoyl-N-[2-(5-dimethylaminonaphthylsulfonamido)ethyl]isoleucine amide,
5(S)-(1,1-Dimethylethoxycarbonylamino)-4(S)-hydroxy-6-phenyl-2(R)-(phenylmethyl)hexanoyl-N-(3-diethylamino-2(R,S)-hydroxypropyl)valine amide,
N-(1(R or S), 2(S or R)-dihydroxy-3-(S or R)-indanyl)-5(S)-(1,1-dimethylethoxycarbonylamino)-4(S)-hydroxy-6-(4′-hydroxyphenyl)-2(R)-(phenylmethyl)hexanamide,
N-(cis-2(R)-Hydroxy-1(S)-indanyl)-5(S)-(1,1-dimethylethoxycarbonylamino)-4(S)-hydroxy-6-(3-dimethylaminomethyl-4-hydroxyphenyl)-2(R)-(phenylmethyl)hexanamide,
5(S)-[(1,1-Dimethylethoxycarbonyl)amino]-4(S)-hydroxy-6-phenyl-2(R)-(phenylmethyl)-hexanoyl-N-(2-benzimidazoylmethyl)valine amide,
5(S)-[(1,1-Dimethylethoxycarbonyl)amino]-4(S)-hydroxy-6-phenyl-2(R)-(phenylmethyl)-hexanoyl-N-[2-(3-(1H-imidazol-4-yl))-1-hydroxymethylethyl]valine amide,
N-(cis-2(R)-Hydroxy-1(S)-indanyl)-5(S)-[(1,1-dimethylethoxycarbonyl)amino]-4(S)-hydroxy-6-(2-naphthyl)-2-(R)-(phenylmethyl)hexanamide,
5(S)-[(1,1-Dimethylethoxycarbonyl)amino]-4(S)-hydroxy-6-phenyl-2(R)-(phenylmethyl)-hexanoyl-N-(4-dimethylaminobenzyl)valine amide,
5(S)-[(1,1-Dimethylethoxycarbonyl)amino]-4(S)-hydroxy-6-phenyl-2(R)-(phenylmethyl)-hexanoyl-N-(2-benzimidazolylmethyl)phenylglycineamide,
3[1-(1R)-(1,1-Dimethylethoxycarbonylamino)-2-(phenylethyl)phosphinyl]-2(S or R)-phenylmethyl-N-(2(R)-hydroxy-1-indanyl)propanamide,
5(S)-[(1,1-Dimethylethoxycarbonyl)amino]-4(S)-hydroxy-6-phenyl-2(R)-(phenylmethyl)hexanoyl-N-(2-benzimidazolyl)valine amide,
N-(2(R)-hydroxy-1(S)-indanyl)-5(S)-[(4-pyridinylmethoxycarbonyl)amino]-4(S)-hydroxy-6-phenyl-2(R)-(phenylmethyl)hexanamide,
N-(2(R)-hydroxy-1(S)-indanyl)-N′-[(1-methylethoxycarbonyl)amino]-4(S)-hydroxy-6-phenyl-2(R)-(phenylmethyl)hexanamide,
5(S)-(1,1-Dimethylethoxycarbonylamino)-4(S)-hydroxy-6-phenyl-2(R)-(4′-iodophenylmethyl)hexanoyl-N-3′-dimethylaminopropyl)-valine amide,
N-(benzyl)-5(S)-(1,1-dimethylethoxycarbonylamino)-4(S)-hydroxy-6-phenyl-2(R)-(phenylmethyl)hexanoyl valine thiomide,
N-(1(R),2(S)-dihydroxy-3(S)-indanyl)-N′-(1,1-dimethylethoxycarbonylamino)-4(S)-hydroxy-6-phenyl-2(R)-(3′phenylprop-2′en-yl)hexanamide,
N-[4(S)-benzopyranyl]-5(S)-(1,1-dimethylethoxycarbonylamino)-4(S)-hydroxy-6-phenyl-2(R)-(phenylmethyl)-hexanamide,
N-[4(R,S)-benzopyranyl]-5(S)-(1,1-dimethylethoxycarbonylamino)-4(S)-hydroxy-6-phenyl-2(R)-(phenylmethyl)hexanoyl-valine amide,
N-(allyl-5-amino-5-deoxy-2,3-0-isopropylidene-β-D-ribosyl)-5(S)-(1,1-dimethylethoxycarbonylamino)-4(S)-hydroxy-6-phenyl-2(R)-(phenylmethyl)hexanoyl-valine amide,
5(S)-[(1,1-dimethylethoxycarbonyl)amino]-4(S)-hydroxy-6-phenyl-2(R)-(phenylmethyl)hexanoyl-N-(2-methylaminoethyl)-valine amide,
N-(cis-1-hydroxycyclopent-2-en-3-yl)-5(S)-(1,1-dimethylethox6ycarbonylamino)-4(S)-hydroxy-6-phenyl-2(R)-(phenylmethyl)hexanamide,
5(S)-[(1,1-dimethylethoxycarbonyl)amino]-4(S)-hydroxy-6-phenyl-2(R)-(phenylmethyl)hexanoyl-N-(3-hydroxy-5-isoxazoloyl-methyl)valine amide,
N-(cis-2(R)-hydroxy-1(S)-indanyl)-5(S)-benzyloxycarbonylamino -4(S)-hydroxy-6-phenyl-2(R)-(phenylmethyl)-hexanamide,
N-(2-hydroxyethyl)-5(S)-(1,1-dimethylethoxycarbonylamino)-4(S)-hydroxy-2(R)-(phenylmethyl)hexanoyl-isoleucyl-amide,
5(S)-(1,1-dimethylethoxycarbonylamino)-4(S)-hydroxy-6-phenyl-2(R)-(phenylthiomethyl)-N-(2-hydroxyethyl)-phenyl glycine amide,
N-(cis-2(R or S)-aminocarbonyl-1(S or R)-indanyl)-5(S)-(1,1-dimethylethoxycarbonylamino)-4(S)-hydroxy-6-phenyl-2(R)-(phenylmethyl)hexanamide,
N-(2(R)-hydroxy-1(S)-indanyl)-5(S)-[(1,1-dimethylethoxycarbonyl)amino]-4(S)-hydroxy-6-(4-hydroxyphenyl)-2-(R)-(4-hydroxyphenylmethyl)hexanamide,
N-(cis-2(R or S)-carboxy-1(S or R)-indanyl)-5(S)-(1,1-dimethylethoxycarbonylamino)-4(S)-hydroxy-6-phenyl-2(R)-(phenylmethyl)hexanamide,
N-(cis-2(R)-hydroxy-1(S)-indanyl)-5-(1,1-dimethylethoxycarbonylamino)-4(S)-hydroxy-6-phenyl-2(R)-(4-nitrophenylmethyl)hexanamide,
5(S)-(1,1-dimethylethoxycarbonylamino)-4(S)-hydroxy-6-phenyl-2(R)-(4-nitrophenylmethyl)-hexanoyl-N-(2-hydroxyethyl)-phenylglycine amide,
N-(cis-2(R)-hydroxy-1(S)-indanyl)-5(S)-(1,1-dimethylethoxycarbonylamino)-4(S)-hydroxy-6-phenyl-2(R)-(4-aminophenylmethyl)-hexanamide,
N′-(1,1-dimethylethoxycarbonyl)-5(S)-amino-4(S)-hydroxy-6-phenyl-2(R)-(phenylmethyl)-hexanoyl-N-[3-((4′-morpholino)propyl)]-valine amide,
N-[5-amino-5-deoxy-D-ribosyl]-5(S)-(1,1-dimethylethoxycarbonylamino)-4(S)-hydroxy-6-phenyl-2(R)-phenylmethyl-hexanoyl-valine amide,
N-(5-amino-5-deoxy-2,3,0-isopropylidene-D-ribosyl)-5(S)-(1,1-dimethylethoxycarbonylamino)-4(S)-hydroxy-6-phenyl-2(R)-(phenylmethyl)hexanoyl-valine amide,
5(S)-[(1,1-dimethylethoxycarbonyl)amino]-4(S)-hydroxy-6-phenyl-2(R)-(phenylmethyl)hexanoyl-N-(4-pyridylmethyl)valine amide,
5(S)-[(1,1-dimethylethoxycarbonyl)amino]-4(S)-hydroxy-6-phenyl-2(R)-(phenylmethyl)hexanoyl-N-(2-pyridylmethyl)valine amide,
5(S)-[(1,1-dimethylethoxycarbonyl)amino]-4(R)-benzyl-4(S)-hydroxy-6-phenylhexanoyl-N-(2-[2-(2-methoxyethoxy)ethoxy]ethyl)valine amide,
5(S)-[(1,1-dimethylethoxycarbonyl)amino]-2(S)-hydroxy-6-phenyl-2(R)-(phenylmethyl)hexanoyl-N-(3-pyridylmethyl)valine amide,
N-[cis-3(S)-hydroxy-4(S)-benzopyranyl]5(S)-(1,1-dimethylethoxycarbonylamino)-4(S)-hydroxy-6-phenyl-2(R)-phenylmethyl-hexanamide,
N-(1R,2S-dihydroxy-3S-indanyl)-5(S)-(1,1-dimethylethoxycarbonylamino)-4(S)-hydroxy-6-(4′-hydroxyphenyl)-2(R)-(4′-hydroxyphenyl)methylhexanamide,
N-(cis-2(R)-hydroxy-1(S)-indanyl)-5(S)-(1,1-dimethylethoxycarbonylamino)-4(S)-hydroxy-6-phenyl-2(R)-(phenylthiomethyl)hexanamide,
Dilithium N-(2-phosphoryloxyethyl)-5(S)-(1,1-dimethylethoxycarbonylamino)-4(S)-hydroxy-2(R)-(phenylmethyl)hexanoyl-isoleucylamide,
N-(Methyl-5-amino-5-deoxy-(αβ)-D-xylosyl)-5(S)-(1,1-dimethylethoxycarbonylamino)-4(S)-hydroxy-6-phenyl-2(R)-(phenylmethyl)hexanoyl-Ile amide,
5(S)-[(4-pyridylmethoxycarbonyl)amino]-4(S)-hydroxy-6-phenyl-2(R)-(phenylmethyl)hexanoyl-N-(2-benzimidazolylmethyl)-Ile amide,
N-(cis-2(R)-hydroxy-1(S)-indanyl)-5(S)-(1,1-dimethylethoxycarbonylamino)-4(S)-hydroxy-6-phenyl-2(R)-(4-(1,1-dimethylethyl)phenylmethyl)hexanamide,
or pharmaceutically acceptable salt thereof.

26. The compounds of Claims 1-25 in combination with any of the antivirals, immunomodulators, antibiotics or vaccines of Table IV.

27. A pharmaceutical composition comprising the compounds of Claims 1-25 and a pharmaceutically acceptable carrier.

28. A pharmaceutical composition comprising the compound in combination according to Claim 26 and a pharmaceutically acceptable carrier.

29. The pharmaceutical composition of Claim 27, for use in the treatment of AIDS, in the prevention of infection by HIV, in the treatment of infection by HIV, or in the inhibition of HIV protease.

30. The pharmaceutical composition of Claim 28 for use in the treatment of AIDS, in the prevention of infection by HIV, in the treatment of infection by HIV, or in the inhibition of HIV protease.

31. The use of a compound of any one of claims 1 - 25 for the manufacture of a medicament useful for treating AIDS.

32. The use of a compound in the combination according to claim 26, for the manufacture of a medicament useful for treating AIDS.

33. The use of a compound of any one of claims 1 - 25 for the manufacture of a medicament useful for preventing infection by HIV.

34. The use of a compound in combination according to Claim 26, for the manufacture of a medicament useful for preventing infection by HIV.

35. The use of a compound as claimed in any one of Claims 1 - 25, for the manufacture of a medicament useful for treating infection by HIV.

36. The use of a compound in combination according to Claim 26, for the manufacture of a medicament useful for treating infection by HIV.

37. The use of a compound as claimed in any one of Claims 1 - 25, for the manufacture of a medicament useful for inhibiting HIV protease.

38. The use of a compound in combination according to Claim 26, for the manufacture of a medicament useful for inhibiting HIV protease.